(19) Europäisches Patentamt / European Patent Office / Office européen des brevets

(11) EP 4 198 124 A1

(12) EUROPEAN PATENT APPLICATION

(43) Date of publication:
21.06.2023 Bulletin 2023/25

(21) Application number: 22214015.4

(22) Date of filing: 15.12.2022

(51) International Patent Classification (IPC):
*C12N 9/22* (2006.01)

(52) Cooperative Patent Classification (CPC):
**C12N 9/22; C12N 15/90;** C12N 15/113; C12N 2310/20; C12R 2001/445

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 15.12.2021 US 202163289914 P

(71) Applicants:
- **Versitech Limited**
  **Pokfulam, Hong Kong (HK)**
- **Centre for Oncology and Immunology Limited**
  **New Territories, Hong Kong (HK)**

(72) Inventors:
- **WONG, Siu Lun**
  **Hong Kong (HK)**
- **YUEN, Tsz Lo**
  **Hong Kong (HK)**

(74) Representative: **HGF**
**HGF Limited**
**1 City Walk**
**Leeds LS11 9DX (GB)**

Remarks:
The complete document including Reference Table(s) and the Sequence Listing(s) can be downloaded from the EPO website

# (54) ENGINEERED CAS9-NUCLEASES AND METHOD OF USE THEREOF

(57) Disclosed are SaCas9 protein variants, constructs encoding such variants, compositions comprising such variants and constructs, and methods of using the variants, constructs, and compositions. In some forms, the disclosed variants comprise the mutation Y239H and do not comprise the mutation R245A. Also disclosed are constructs encoding any of the disclosed variants for expression of the variant in a host of interest. Also disclosed are methods of editing a sequence of interest.

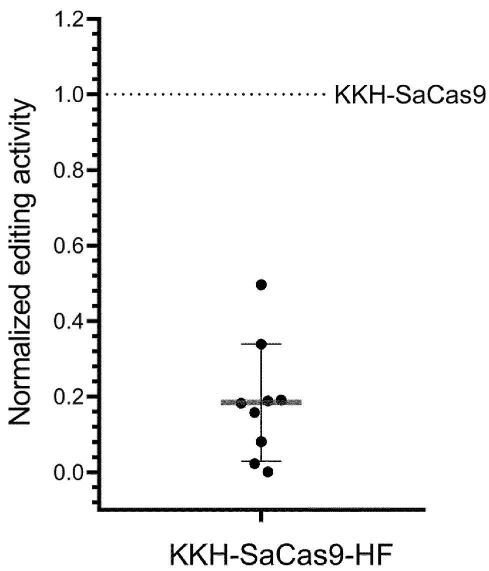


FIG. 1A

EP 4 198 124 A1

## Description

### CROSS-REFERENCE TO RELATED APPLICATIONS

This application claims priority to and benefit of U.S. Provisional No. 63/289,914, filed December 15, 2021. Application No. 63/289,914, filed December 15, 2021, is hereby incorporated by reference in its entirety.

### REFERENCE TO SEQUENCE LISTING

The Sequence Listing submitted 15 December 2022 as a text file named "UHK_01097_US_ST26.xml", created December 8, 2022, and having a size of 126,651 bytes is hereby incorporated by reference pursuant to 37 C.F.R. 1.834(c)(1).

### FIELD OF THE INVENTION

The invention generally relates to targeted genome modification. In particular, the disclosure relates to characterizing RNA-guided endonucleases comprising CRISPR/Cas9 proteins and methods of using said proteins for targeted genome modification.

### BACKGROUND OF THE INVENTION

Clustered Regularly Interspaced Short Palindromic Repeats (CRISPR)-Cas9 (CRISPR-associated protein 9) is a programmable gene-targeting system capable of genome editing in human cells. The CRISPR system has two components: (i) the Cas9 nuclease, and (ii) a single guide RNA (sgRNA) that directs the Cas9 nuclease to targeted DNA locations. The targeting by Cas9-sgRNA complex is carried out by the ~20-nucleotide recognition sequence encoded in the sgRNA, which is complementary to the target DNA sequence. The Cas9 nuclease also recognize a short protospacer adjacent motif (PAM) located downstream to the target DNA sequence for target location identification. Cas9The targeted gene can be knocked out after cleavage by Cas9 nuclease and the error-prone DNA repairing mechanism carried out by the cells.Cas9 The development of CRISPR-Cas9 systems as programmable nucleases for genome engineering has great potential for gene therapy approaches to disease treatment (Cong, et al. Science, 339, 819-823, doi: 10.1126/science.1231143 (2013); Mali, et al. Science, 339, 823-826, doi: 10.1126/science.1232033 (2013)).

A prerequisite for the safe application of CRISPR-Cas9 for high efficiency genomic manipulations in therapeutic and basic research applications is the ability to specifically cleave the intended target site with minimal off target activity. For this reason, variants of Cas9 nucleases have been developed for RNA-guided genome binding, enabling further applications in gene expression control. For example, the more commonly used CRISPR enzyme for genome editing, SpCas9, is derived from the bacteria strain *Streptococcus pyogenes,* and recognizes DNA targets carrying an "NGG" PAM site. SpCas9 variants with a specific combination of mutations were engineered to minimize its off-target editing (Slaymaker, et al. Science, 351, 84-88 doi: 10.1126/science.aad5227 (2016); Kleinstiver, et al. Nature, 529, 490-495, doi: 10.1038/nature16526 (2016); Chen, et al. Nature, 550, 407-410, doi: 10.1038/nature24268 (2017); Casini, et al. NatBiotechnol, 6, 265-271, doi: 10.1038/nbt.4066 (2018); Lee, etal. Nat Commun,9, 3048, doi: 10.1038/s41467-018-05477-x (2018); Vakulskas, et al. Nat Med, 24, 1216-1224, doi: 1.1038/s41591-018-0137-0 (2018); Choi, et al. Nat Methods, 16, 722-730, doi: 10.1038/s41592-019-0473-0 (2019)). However, fewer studies have been conducted on the *Staphylococcus aureus* derived CRISPR enzyme, SaCas9, although SaCas9 holds an important advantage of being smaller than SpCas9, a feature that enables its efficient packaging using adeno-associated virus vectors for *in vivo* gene editing and gene therapy applications.

SaCas9 was reported to edit the human genome with similar efficiency as with SpCas9 (Ran, et al. Nature, 520, 186-191, doi: 10.1038/nature14299 (2015)). Using SaCas9 for genome editing requires its target site to contain a longer PAM site (i.e., "NNGRRT"). To overcome this limitation, several mutational studies on SaCas9 were carried out to broaden its PAM recognition (Kleinstiver, et al. Nat Biotechnol, 33, 1293-1298, doi: 10.1038/nbt.3404 (2015); Ma, et al. Nat Commun, 10, 560, doi: 10.1038/s41467-019-08395-8 (2019); Luan, et al. JAm Chem Soc, 141, 6545-6552, doi: 10.1021/jacs.8b13144 (2019)), and KKH-SaCas9 is one of the identified variants that recognizes a "NNNRRT" PAM site. This variant is useful for therapeutic genome editing because it can edit sites with PAM that other small-sized Cas orthologs such as Cas9 from *Campylobacter jejuni* (Kim, et al. Nat Commun, 8, 14500, doi: 10.1038/ncomms14500 (2017)) and *Neisseria meningitidis* (Edraki, et al. Mol Cell, 73, 714-726, doi: (2019)), as well as Cas12a (Zetsche, et al. Cell, 163, P759-771, doi: 10.1016/j.cell.2015.09.038 (2015))and CasΦ (Pausch, et al. Science, 369, 333-337, doi: 10.1126/science.abb1400 (2020)) cannot recognize. In terms of editing fidelity, SaCas9 variants (including SaCas9-HF (Tan, et al. Proc Natl Acad Sci U.S.A, 116, 20969-20976, doi: 10.1073/pnas.1906843116 (2019) and eSaCas9 (Slaymaker, etal. Science, 351, 84-88 doi: 10.1126/science.aad5227 (2016)) carrying a specific combination of mutations at its amino acid residues that interact with the targeting or non-targeting DNA strand and the sgRNA were shown to exhibit reduced off-target activity. Comparison between SaCas9-HF with eSaCas9 revealed that they have comparable on-target activity and genome-wide targeting specificity (Tan, et al. Proc Natl Acad Sci U.S.A, 116, 20969-20976, doi: 10.1073/pnas. 1906843116 (2019)). However, grafting the mutations (i.e., R245A/N413A/N419A/R654A) from SaCas9-HF onto KKH-SaCas9 greatly reduced its on-target activity in targeting many of the tested gene targets (Tan, et al. Proc Natl Acad Sci U.S.A, 116, 20969-20976, doi: 10.1073/pnas. 1906843116 (2019)). There is no existing SaCas9 variant with a broad targeting range (such as KKH-SaCas9) with both high efficiency and proven genome-wide accuracy, which is needed for therapeutic applications. Thus, there is currently a need in the art for highly specific and efficient variants of Cas9 nucleases that can make edits across a broad range of genomic targets to improve the genome editing capability for the CRISPR-Cas9 system. Technology for engineering smaller site-specific endonucleases with improved specificity is also needed.

Therefore, it is an object of the invention to provide smaller Cas9 nuclease variants with more precise on-target editing and lower off-target editing that can be efficiently packaged using adeno-associated virus vectors for *in vivo* gene editing and gene therapy applications.

It is another object of the invention to provide a method for multi-domain combinatorial mutagenesis which employs a structure guided approach to selecting mutations for engineering and testing Cas9 variants.

## SUMMARY OF THE INVENTION

Disclosed are Cas9 protein variants for genome editing. These nucleases exploit the discovery that smaller Cas9 proteins can be engineered for high specificity with high on-target activity under a longer PAM site (i.e., "NNGRRT"). The disclosed endonucleases can be efficiently packaged using adeno-associated virus vectors for *in vivo* gene editing and gene therapy applications.

Disclosed are SaCas9 protein variants, constructs encoding such variants, compositions comprising such variants and constructs, and methods of using the variants, constructs, and compositions. In some forms, the disclosed variants comprise the mutation Y239H, and not comprising the mutation R245A. Such variants can be referred to as Y/H variants. Y/H variants are a preferred form of the disclosed variants. All of the most preferred Cas9 protein variants retain the key amino acid substitution mutations E782K, N968K and R1015H.

In some forms, the disclosed variant does not include any mutation or combination of mutations such that the variant has greater off-target activity than a control SaCas9 variant or a control Cas9 variant. In some forms, the disclosed variant does not include any mutation or combination of mutations that result in the variant having greater off-target activity than SaCas9 variant v3.2. In some forms, the off-target activity is measured in a GFP disruption assay. In some forms, the measurement is taken at 15 days. In some forms, the assay is performed in cells harboring a reporter construct expressing an off-target sgRNA. In some forms, the cells harboring the reporter construct expressing the off-target sgRNA are OVCAR8-ADR. In some forms, the cells harboring the reporter construct expressing the off-target sgRNA are MHCC97L cells. In some forms, the cells harboring the reporter construct expressing the off-target sgRNA are SK-N-MC cells. In some forms, the off-target sgRNA has the sequence CACCTACGGCAATCTGACCCTGAAGT (SEQ ID NO:1). In some forms, the control SaCas9 variant comprises the mutations N419D, R654A, G655A, E782K, N968K, and R1015H. In some forms, the control SaCas9 variant has only the mutations N419D, R654A, G655A, E782K, N968K, and R1015H. In some forms, the SaCas9 variant is variant (v) 3.2.

In some forms, the disclosed variant does not include any other mutation or combination of other mutations such that the variant has greater off-target activity than SaCas9 variant v3.2 in a GFP disruption assay at 15 days in OVCAR8-ADR cells harboring a reporter construct expressing an off-target sgRNA having the sequence CACCTACGGCAATCTGACCCTGAAGT (SEQ ID NO:1), wherein the SaCas9 variant v3.2 has only the mutations N419D, R654A, G655A, E782K, N968K, and R1015H.

In some forms, the disclosed variant does not include any mutation or combination of mutations such that the variant has greater on-target activity than a control SaCas9 variant or a control Cas9 variant. In some forms, the disclosed variant does not include any mutation or combination of mutations that result in the variant having greater on-target activity than SaCas9 variant KKH-SaCas9. In some forms, the on-target activity is measured in a GFP disruption assay. In some forms, the measurement is taken at 15 days. In some forms, the assay is performed in cells harboring a reporter construct expressing an on-target sgRNA. In some forms, the cells harboring the reporter construct expressing the on-target sgRNA are OVCAR8-ADR. In some forms, the cells harboring the reporter construct expressing the off-target sgRNA are MHCC97L cells. In some forms, the cells harboring the reporter construct expressing the off-target sgRNA are SK-N-MC cells. In some forms, the on-target sgRNA has the sequence CACCTACGGCAAGCTGACCCTGAAGT (SEQ ID NO:2). In some forms, the control SaCas9 variant comprises the mutations E782K, N968K, and R1015H. In some forms, the control SaCas9 variant has only the mutations E782K, N968K, and R1015H. In some forms, the SaCas9 variant is KKH-SaCas9.

In some forms, the disclosed variant does not include any other mutation or combination of other mutations such that the variant has on-target activity less than 0.5 of the on-target activity of SaCas9 variant KKH-SaCas9 in a GFP disruption assay at 15 days in OVCAR8-ADR cells harboring a reporter construct expressing an on-target sgRNA having the sequence CACCTACGGCAAGCTGACCCTGAAGT (SEQ ID NO:2), wherein the SaCas9 variant KKH-SaCas9 has only the mutations E782K, N968K, and R1015H. In some forms, the disclosed variant can further comprise one or more mutations selected from the group consisting of T238A, T392A, N394T, N394A, N413A, Q414R, N419A, N419D, N419S, N419G, R499A, Q500A, Y651H, R654A, and G655A. In some forms, the disclosed variant can include the mutation N419D. In some forms, the disclosed variant can include the mutation N419S. In some forms, the disclosed variant can include the mutation N419G. In some forms, the disclosed variant can include the mutation R499A. the mutation Q500A. In some forms, the disclosed variant can include the mutation Y651H. In some forms, the disclosed variant can include the mutation R654A. In some forms, the disclosed variant can include the mutation G655A. In some forms, the disclosed variant can include the mutation Q414R.In some forms, the disclosed variant can include the mutation N394T. In some forms, the disclosed variant can include the mutation N394A. In some forms, the disclosed variant can include the mutation T392A. In some forms, the disclosed variant can include the mutation T238A.

In some forms, the disclosed variant can include one or more mutations selected from the group consisting of R499A, Q500A, Y651H, R654A, and G655A. In some forms, the disclosed variant is v3.18, v3.8, v3.22, v3.16, v3.10, v3.24, or v3.19.

In some forms, the disclosed SaCas9 variant is an isolated *Staphylococcus aureus* Cas9 (SaCas9) protein comprising an amino acid sequence that (1) has at least 80% - 95% sequence identity to the amino acid sequence of KKH-SaCas9 and (2) has an amino acid substitution at Y239H.

In some forms, the disclosed SaCas9 variant is an isolated *Staphylococcus aureus* Cas9 (SaCas9) protein comprising an amino acid sequence that (1) has at least 80% - 95% sequence identity to the amino acid sequence of KKH-SaCas9 and (2) has amino acid substitutions at Y239H, N419D, R499A, Q500A and Y651H.

Also disclosed are constructs encoding any of the disclosed variants for expression of the variant in a host of interest. In some forms, the construct can comprise sequences for expression of the variant in the host of interest. In some forms, the construct can further encode an sgRNA targeting a sequence of interest and sequences for expression of the sgRNA in the host of interest. In some forms, the construct can be comprised in a virus vector. In some forms, the virus vector can be an adeno-associated virus vector.

Also disclosed are methods of editing a sequence of interest. In some forms, the method comprises contacting a disclosed construct with the host of interest, where the host of interest harbors the sequence of interest and where the cell expresses the construct to produce variant and the sgRNA. In some forms, the method comprises contacting a disclosed construct with the host of interest, where the host of interest harbors a sequence of interest and where the cell expresses the construct to produce the variant. In some forms, the method comprises contacting the sequence of interest with a disclosed mixture, whereby the variant edits the sequence of interest targeted by the sgRNA.

In some forms, the method can further comprises causing an sgRNA targeting the sequence of interest to be present in the host of interest with the produced variant, whereby the produced variant edits the sequence of interest targeted by the sgRNA.

Also disclosed are mixtures comprising any one or more of the disclosed variants and an sgRNA targeting a sequence of interest. In some forms, the mixture can be comprised in a delivery particle. In some forms, the mixture can be comprised in a cell containing the sequence of interest.

Additional advantages of the disclosed method and compositions will be set forth in part in the description which follows, and in part will be understood from the description, or can be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

## BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

**Figure 1** is a graph showing that KKH-SaCas9-HF exhibits low on-target activity. Assessment of KKH-SaCas9-HF's on-target editing with sgRNAs targeting endogenous loci. The percentage of sites with indels was measured using a T7 endonuclease I (T7E1) assay. The ratio of the on-target activity of KKH-SaCas9-HF to the activity of KKH-SaCas9 was determined, and the mean and standard deviation for the normalized percentage of indel formation are shown for the 9 loci tested. Each locus was measured three times. The full dataset is presented in **Figure 16.**

**Figure 2** shows protein expression of KKH-SaCas9 variants in human cells. OVCAR8-ADR cells were infected with lentiviruses encoding KKH-SaCas9 variants. Protein lysates were extracted for Western blot analysis and immunoblotted with anti-SaCas9 antibodies. GAPDH was used as loading control.

**Figure 3** is a dot plot showing the editing efficiency of the KKH-SaCas9 variants carrying different mutation combinations using GFP disruption assays. OVCAR8-ADR cells harboring reporter constructs with on-target (ON1 and ON2) and off-target (OFF1-3) sgRNAs were infected with lentiviruses encoding the individual KKH-SaCas9 mutants. ON1 is SEQ ID NO:2 and ON2 is SEQ ID NO:3. Editing efficiency of KKH-SaCas9 variants was measured as percentage of cells with depleted GFP fluorescence and compared to efficiency for KKH-SaCas9. The percentages of off-target GFP disruption are presented in **Figure 17.**

**Figures 4A-4C** are graphical representations of the construction of KKH-SaCas9 variants carrying different mutation combinations and their characterization using GFP disruption assays. **Figures 4A-4C** shows assessment of KKH-SaCas9 variants' on-target editing with sgRNAs targeting endogenous loci. OVCAR8-ADR cells harboring reporter constructs with on-target (ON1 and ON2) and off-target (OFF1-3) sgRNAs were infected with lentiviruses encoding the individual KKH-SaCas9 mutants. ON1 is SEQ ID NO:2 and ON2 is SEQ ID NO:3. OFF1 is SEQ ID NO:1, OFF2 is SEQ ID NO:4, and OFF3 is SEQ ID NO:5. KKH-SaCas9-SAV2 is SEQ ID NO:81. The percentage of sites with indels was measured using a T7 endonuclease I (T7E1) assay. The ratio of the on-target activity of KKH-SaCas9-SAV1, SAV2, and KKH-eSaCas9 to the activity of KKH-SaCas9 was determined, and the median and interquartile range for the normalized percentage of indel formation are shown for the 5-6 loci tested in three cell lines. Each locus was measured twice or three times; the full dataset is presented in **Figures 21A-21C.**

**Figure 5** is a bar graph of the off-target activity of the KKH-SaCas9 variants carrying different mutation combinations using GFP disruption assays. OVCAR8-ADR cells harboring reporter constructs with on-target (ON1 and ON2) and off-target (OFF1-3) sgRNAs were infected with lentiviruses encoding the individual KKH-SaCas9 mutants. OFF1 is SEQ ID NO:1, OFF2 is SEQ ID NO:4, and OFF3 is SEQ ID NO:5. KKH-SaCas9-SAV1 is SEQ ID NO:80. KKH-SaCas9-SAV2 is SEQ ID NO:81.

**Figure 6** is a molecular model of the predicted interaction between R245 mutations with the DNA backbone in SaCas9.

**Figures 7A and 7B** are bar graphs showing the editing efficiency of the KKH-SaCas9-SAV1 and KKH-SaCas9-SAV2. In **Figures 7A and 7B,** OVCAR8-ADR cells expressing KKH-SaCas9, SAV1, and SAV2 were infected with lentiviruses encoding sgRNAs carrying no or one- to two- base mismatch(es) against the target. Editing efficiency of KKH-SaCas9 variants was measured as percentage of cells with depleted GFP fluorescence and compared to efficiency for KKH-SaCas9. The percentages of off-target GFP disruption are presented in **Figure 17.** In **Figures 7A and 7B,** the sequences are, from top to bottom, SEQ ID NOs:6-29. KKH-SaCas9-SAV1 is SEQ ID NO:80. KKH-SaCas9-SAV2 is SEQ ID NO:81.

**Figures 8A-8C** are graphical representations of the off-target editing activity of the KKH-SaCas9 variants carrying different mutation combinations using GFP disruption assays. KKH-SaCas9-SAV2 carrying a Y239R substitution and/or additional mutations were individually constructed and characterized using GFP disruption assays. In **Figures 8A-8C,** OVCAR8-ADR cells harboring reporter constructs with on-target (ON1 and ON2) and off-target (OFF1-3) sgRNAs were infected with lentiviruses encoding the individual KKH-SaCas9 mutants. OFF1 is SEQ ID NO:1, OFF2 is SEQ ID NO:4, and OFF3 is SEQ ID NO:5. KKH-SaCas9-SAV1 is SEQ ID NO:80. KKH-SaCas9-SAV2 is SEQ ID NO:81. After 7- and 15- day post-infection, the editing efficiency of the KKH-SaCas9 variants was measured as the percentage of cells with depleted GFP fluorescence using flow cytometry. Mean and standard deviation obtained from at least two biological replicates are shown.

**Figure 9** is a graphical representation of the specificity and activity scores for tested KKH-SaCas9 variants. KKH-SaCas9-SAV1 is SEQ ID NO:80. KKH-SaCas9-SAV2 is SEQ ID NO:81.

**Figures 10A-10C** shows molecular modelling of Y239H/R mutations in SaCas9 depicts their differential interactions with F418 of SaCas9 and the sgRNA backbone.

**Figure 11** is a graphical representation comparing the on- and off-target activities of SAV1 and SAV2 with existing/candidate high-fidelity variants of KKH-SaCas9. In **Figure 11,** OVCAR8-ADR cells harboring reporter constructs with on-target (ON1 and ON2) and off-target (OFF1-3) sgRNAs were infected with lentiviruses encoding the individual KKH-SaCas9 mutants. KKH-SaCas9 variants carrying different mutation combinations were constructed and characterized using GFP disruption assays. The editing efficiency of the KKH-SaCas9 variants was measured as the percentage of cells with depleted GFP fluorescence and compared to the efficiency for KKH-SaCas9. ON1 is SEQ ID NO:2 and ON2 is SEQ ID NO:3. OFF1 is SEQ ID NO:1, OFF2 is SEQ ID NO:4, and OFF3 is SEQ ID NO:5. KKH-SaCas9-SAV1 is SEQ ID NO:80. KKH-SaCas9-SAV2 is SEQ ID NO:81.

**Figure 12** is a graphical representation of the on- and off-target activity of the KKH-SaCas9 variants. In **Figure 12,** OVCAR8-ADR cells stably expressing individual KKH-SaCas9 mutants were first generated and infected with lentiviruses encoding the reporter constructs with on- and off-target sgRNAs. The editing efficiency of the KKH-SaCas9 variants was measured as the percentage of cells with depleted GFP fluorescence and compared to the efficiency for KKH-SaCas9. ON1 is SEQ ID NO:2 and ON2 is SEQ ID NO:3. OFF1 is SEQ ID NO: 1, OFF2 is SEQ ID NO:4, and OFF3 is SEQ ID NO:5. KKH-SaCas9-SAV1 is SEQ ID NO:80. KKH-SaCas9-SAV2 is SEQ ID NO:81.

**Figure 13** shows how KKH-SaCas9- SAV1 and SAV2 exhibit low genome-wide off-target activity, depicting an assessment of KKH-SaCas9 variants' on-target editing with sgRNAs targeting endogenous loci. The percentage of sites with indels was measured using a T7 endonuclease I (T7E1) assay. The ratio of the on-target activity of KKH-SaCas9-SAV1 and SAV2 to the activity of KKH-SaCas9 was determined, and the mean and standard deviation for the normalized percentage of indel formation are shown for the 11 loci tested. Each locus was measured twice or three times.

**Figures 14A-14E** show how KKH-SaCas9- SAV1 and SAV2 exhibit low genome-wide off-target activity, showing GUIDE-seq genome-wide specificity profiles for the KKH-SaCas9 variants paired with the indicated sgRNAs. Mismatched positions in off-target sites are shaded, and GUIDE-seq read counts were used as a measure of the cleavage efficiency at a given site. Sequence EMX1-sg2 is SEQ ID NO:30. Sequence AAVS1-sg4 is SEQ ID NO:31. Sequence EMX1-sg7 is SEQ ID NO:32. Sequence VEGFA-sg3 is SEQ ID NO:33. Sequence CCR5-sg2 is SEQ ID NO:34.

**Figures 15A and 15B** show the editing percentage for different positions in target sequences of vascular endothelial growth factor (VEGF) (**Figure 15A**) and Fanconi anemia group F protein (FANCF) (**Figure 15B**). The VEGF target sequence is SEQ ID NO:35. **Figure 15A** depicts SEQ ID NO:35 without a substitution and with an A substituted at each position in turn, which are, from top to bottom, SEQ ID NO:35-56. The FANCF target sequence is SEQ ID NO:57. **Figure 15B** depicts SEQ ID NO:57 without a substitution and with an A substituted at each position in turn, which are, from top to bottom, SEQ ID NO:57-78. The data in **Figures 15A and 15B** show that SAV1 and SAV2 is that they have an enhanced ability to distinguish targets with single-nucleotide differences including those located distantly from the PAM.

**Figure 16** is a bar graph showing results from assessment of KKH-SaCas9-HF's on-target editing with sgRNAs targeting endogenous loci. The percentage of sites with indels was measured using a T7 endonuclease I (T7E1) assay. The ratio of the on-target activity of KKH-SaCas9-HF to the activity of KKH-SaCas9 (indicated by the symbol #) was determined, and the mean and standard deviation for the normalized percentage of indel formation are shown for the nine loci tested. Each locus was measured in three replicates.

**Figure 17** is a graph showing the off-target editing activity of KKH-SaCas9 variants. OVCAR8-ADR cells expressing KKH-SaCas9 variants (SEQ ID NOs:84-100) were infected with lentiviruses encoding sgRNAs carrying one-base mismatch against the target GFP sequence. After 7- and 13- day post-infection, the editing efficiency was measured as the percentage of cells with depleted GFP fluorescence using flow cytometry. Mean and standard deviation obtained from at least three biological replicates are shown for the variants tested. OFF1 is SEQ ID NO: 1, OFF2 is SEQ ID NO:4, and OFF3 is SEQ ID NO:5.

**Figures 18A-18D** are graphs showing that KKH-SaCas9-SAV1 and SAV2 reduce off-target edits at sites harboring sequences with single and double mismatch(es) to sgRNA spacer. OVCAR8-ADR cells expressing KKH-SaCas9 (**Figures 18A and 18C**)**,** SAV1 (**Figures 18B and 18D**), and SAV2 (**Figures 18B and 18D**) were infected with lentiviruses encoding sgRNAs carrying no or one- to two- base mismatch(es) against the target. After 4-, 7-, and 13-day post-infection, the editing efficiency was measured as the percentage of cells with depleted GFP fluorescence using flow cytometry. Day 7 data is presented in **Figures 7A and 7B.** Mean and standard deviation obtained from three biological replicates are shown for the variants tested. In **Figures 18A and 18C,** the sequences are, from top to bottom, SEQ ID NOs:6-29. KKH-SaCas9-SAV1 is SEQ ID NO:80. KKH-SaCas9-SAV2 is SEQ ID NO:81.

**Figure 19** shows the molecular modelling of N394T mutation in SaCas9. N394T mutation is modelled to reduce interaction with the sgRNA backbone at the side opposite to where Y239 interacts.

**Figures 20A and 20B** are bar graphs showing that adding N260D to SAV1 and SAV2 reduced their editing activities. KKH-SaCas9-SAV1 and SAV2 variants were added with a N260D mutation, and their editing efficiencies were characterized using GFP disruption assays. OVCAR8-ADR cells harboring reporter constructs with two on-target (**Figure 20A**) and three off-target (**Figure 20B**) sgRNAs were infected with lentiviruses encoding the individual KKH-SaCas9 variants. After 13-day post-infection, the editing efficiency of the KKH-SaCas9 variants was measured as the percentage of cells with depleted GFP fluorescence using flow cytometry. Mean and standard deviation obtained from three biological replicates are shown.

**Figures 21A-21C** are bar graphs summarizing the T7 endonuclease I (T7E1) assay results. OVCAR8-ADR (**Figure 21A**), SK-N-MC (**Figure 21B**), and MHCC97L (**Figure 21C**) cells were infected with KKH-SaCas9, SAV1, SAV2, or KKH-eSaCas9 and the indicated gRNAs. Genomic DNA was collected for T7E1 assay. Indel quantification for the infected samples is displayed as a bar graph. Mean and standard deviation obtained from three biological replicates are shown.

**Figures 22A-22D** are bar graphs showing gene knockdown efficiency of nuclease-dead KKH-SaCas9-KRAB variants grafted with KKH-eSaCas9, SAV1, and SAV2 mutations. OVCAR8-ADR cells were co-infected with lentiviruses encoding KKH-dSaCas9-KRAB variants (KKH-dSaCas9-KRAB, KKH-deSaCas9-KRAB, KKH-dSaCas9-SAV1-KRAB, or KKH-dSaCas9-SAV2-KRAB) and sgRNAs carrying no (i.e., DNMT1sg1 (**Figure 22A**) and EGFRsg1 (**Figure 22C**)) or one-base mismatch (i.e., DNMT1sg1-M12 (**Figure 22B**), DNMT1sg1-M18 (**Figure 22D**), and EGFRsg1-M12 (**Figure 22D**)) against the target. mRNA knockdown efficiency was measured using RT-qPCR. Mean and standard deviation obtained from three biological replicates are shown. *, $p<0.05$; **, $p<0.01$; ***, $p<0.001$ determined using one-way ANOVA with Dunnett's test. KKH-dSaCas9-KRAB refers to the fusion of the Kruppel associated box (KRAB) domain to the nuclease-dead version of KKH-SaCas9. UNT is the control with no KKH-dSaCas9-KRAB.

## DETAILED DESCRIPTION OF THE INVENTION

The disclosed compositions and methods may be understood more readily by reference to the following detailed description of particular embodiments, the Examples included herein and to the Figures and their previous and following description.

It is to be understood that the disclosed compositions and methods are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

It has been discovered that mutating multiple DNA- and sgRNA-interacting residues spanning the different parts of the protein encoding the KKH-SaCas9 enzyme produces variants that confer KKH-SaCas9 with genome-wide editing accuracy and efficiency superior to other previously identified variants. The present disclosure describes highly specific and efficient variants of KHH-SaCas9 that can make edits across a broad range of genomic targets (i.e., with "NNNRRT" PAM), including sites harboring "NHHRRT" PAM that could not be targeted by other high-fidelity SpCas9 variants that recognize "NGG" PAM. For example, two disclosed variants KKH-SaCas9-SAV1 (SAV1) and KKH-SaCas9-SAV2 (SAV2), were identified using combinatorial mutagenesis and have an enhanced ability to distinguish targets with single-nucleotide differences including those located distantly from the PAM. Current strategies to target mutant alleles using SaCas9 requires the pathogenic single-nucleotide polymorphism (SNP) or mutation to be located within the seed region of the sgRNA or using an SNP-derived PAM to achieve SNP-specific targeting without cleaving the wild-type allele. However, these do not apply to SNPs that are located outside of the seed region or those that do not generate a new PAM for SaCas9 targeting. The unique ability of SAV1 and SAV2 in distinguishing a broader range of single-nucleotide mismatches could expand the scope and capabilities of genome editing at loci with SNPs and mutations located further away from the PAM, which has not been previously achieved.

### A. Definitions

The terms "editing fidelity" or "editing efficiency" or "targeting accuracy" are understood to mean the percentage of desired mutation achieved and are measured by the precision of the Cas9 variant in altering the DNA construct of the targeted gene with minimal off-target editing. A DNA editing efficiency of 1 (or 100%) indicates that the number of edited cells obtained when the Cas9 variant is used is approximately equal or equal to the number of edited cells obtained when the wild type or parent Cas9 variant is used. Conversely, a DNA editing efficiency greater than 1 indicates that the number of edited cells obtained when the Cas9 variant used is greater than the number of edited cells obtained when the parent Cas9 variant is used. In this case, the Cas9 variant has improved properties, for example improved editing efficiency when compared to the parent Cas9 endonuclease.

The term "variant" or "mutant," as used herein refer to an artificial outcome that has a pattern that deviates from what occurs in nature. In the context of the disclosed SaCas9 variants, "variant" refers to a SaCas9 that has one or more amino acid changes relative to wildtype SaCas9 or relative to a starting, base, or reference SaCas9, such as KKH-SaCas9 or SaCas9-HF. Note that the disclosed SaCas9 variants have one or more amino acid changes relative to a reference, base, or starting SaCas9 (such as, e.g., wildtype SaCas9, KKH-SaCas9, or SaCas9-HF). While some such reference, base, or starting SaCas9 proteins (such as, e.g., KKH-SaCas9 or SaCas9-HF) are themselves a "variant" of another or other SaCas9 proteins, these reference, base, or starting SaCas9 proteins are not a disclosed variant as described herein, and reference herein to such reference, base, or starting SaCas9 proteins as a "variant" SaCas9 is not intended to, and does not, indicate that such reference, base, or starting SaCas9 proteins are a disclosed variant as described herein.

The terms "single guide RNA" or "sgRNA" refer to the polynucleotide sequence comprising the guide sequence, tracr sequence and the tracr mate sequence. "Guide sequence" refers to the around 20 base pair (bp) sequence within the guide RNA that specifies the target site and may be used interchangeably with the terms "guide" or "spacer."

The terms "genome editing," "genome engineering" or "genome mutagenesis" refer to selective and specific changes to one or more targeted genes or DNA sequences within a recipient cell through programming of the CRISPR-Cas system within the cell. The editing or changing of a targeted gene or genome can include one or more of a deletion, knock-in, point mutation, substitution mutation or any combination thereof in one or more genes of the recipient cell.

The terms "vector" or "expression vector" refer to a system suitable for delivering and expressing a desired nucleotide or protein sequence. Some vectors may be expression vectors, cloning vectors, transfer vectors etc.

The term "grafting" refers to the addition or fusion of a fragment of one gene (such as that encoding a protein residue) onto the DNA backbone of another gene.

The terms "Protospacer adjacent motif' or "PAM sequence" or "PAM interaction region" refer to short pieces of genetic code that flag editable sections of DNA and serve as a binding signal for specific CRISPR-Cas nucleases. The PAM interaction region in the wild-type SaCas9 or its variants contains amino acid residues 910-1053 (Nishimasu, et al. Cell, 162, 1113-1126, doi: 10.1016/j.cell.2015.08.007 (2016)) and includes a . conserved 13-amino acid region spanning positions 982 to 994 which plays a role in binding to the $4^{th}$ and $5^{th}$ bases of the PAM (Ma, et al. Nature Communications, 10, 560, doi: 10.1038/s41467-019-08395-8 (2019)).

The terms "Cas9," "Cas9 protein," or "Cas9 nuclease" refer to a RNA-guided endonuclease that is a Cas9 protein that catalyzes the site-specific cleavage of double stranded DNA. Also, referred to as "Cas nuclease" or "CRISPR-associated nuclease." In nature, the CRISPR system is an adaptive immune system found in bacteria that provides protection against mobile elements such as phage viruses and transposable elements. DNA binding and cleavage requires the Cas9 protein and two RNAs, a trans-encoded RNA (tracrRNA) and a CRISPR RNA (crRNA) in nature. Artificially, single-guided RNA or sgRNA can be engineered to incorporate aspects of both RNAs into a single species (Jinek, et al. Science, 337, 816-821, doi: 10.1126/science. 1225829 (2012)). The CRISPR system has two components: the Cas9 nuclease and a single guide RNA (sgRNA) that provides DNA sequence-targeting accuracy. The targeting of the Cas9-sgRNA complex is mediated by the protospacer adjacent motif (PAM) located at the DNA for Cas9 recognition and the homology between the ~20-nucleotide recognition sequence encoded in the sgRNA and the genomic DNA target. The targeted gene can be knocked out after the Cas9-sgRNA complex finds and cleaves the exonic region of the gene to generate frameshift mutations. Cas9 recognizes short motifs in CRISPR repeat sequences to help distinguish self from non-self. Cas9 nuclease sequences and structures are known to those of skill in the art (Ferretti, et al. Proc Natl Acad Sci U.S.A, 98, 4658-4863, doi: 10.1073/pnas.071559398 (2001); Deltcheva, et al. Nature, 471, 602-607, doi: 10.1038/nature09886 (2011)). Cas9 orthologs have been described in several species of bacteria, including but not limited to *Streptococcus pyogenes* and *Streptococcus thermophilus, Campylobacter jejuni* and *Neisseria meningitidis.* (Slaymaker, et al. Science, 351, 84-88 doi: 10.1126/science.aad5227 (2016); Kleinstiver, et al. Nature, 529, 490-495, doi: 10. 10 38/nature 16526 (2016); Chen, et al. Nature, 550, 407-410, doi: 10.1038/nature24268 (2017); Casini, et al. Nat Biotechnol, 6, 265-271, doi: 10.1038/nbt.4066 (2018); Lee, et al. Nat Commun,9, 3048, doi: 10.1038/s41467-018-05477-x (2018); Vakulskas, et al. Nat Med, 24, 1216-1224, doi: 1.1038/s41591-018-0137-0 (2018); Choi, et al. Nat Methods, 16, 722-730, doi: 10.1038/s41592-019-0473-0 (2019); Kim, et al. Nat Commun, 8, 14500, doi: 10.1038/ncomms14500 (2017); (Edraki, et al. Mol Cell, 73, 714-726, doi: (2019)).

The term "mutation" refers to a substitution of a residue within a sequence, e.g., a nucleic acid or amino acid sequence, with another residue, or a deletion or insertion of one or more residues within a sequence. Mutations are described by identifying the original residue followed by the position of the residue within the sequence and by the identity of the change in residue. For the purposes of this disclosure, amino acid positions are identified using the amino acid positions shown in SaCas9 sequence UniProtKB/Swiss-Prot No. J7RUA5.1 (SEQ ID NO:79), with the numbering beginning at the initial methionine residue. Various methods for making the mutations in the amino acids provided herein are well known in the art, and are provided by, for example, Green and Sambrook, Molecular Cloning: A Laboratory Manual 4th Edition, Cold Spring Harbor Laboratory Press, (2012).

## B. Compositions

As described herein, Cas9 proteins are engineered to have increased specificity with high on-target and low off-target editing, by altering the binding affinity of Cas9 for DNA. Several variants of the *Staphylococcus aureus* (SaCas9) were engineered by introducing substitution mutations into various residues in the SaCas9 that alters its bonding with the sgRNA backbone. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

Disclosed are SaCas9 protein variants, constructs encoding such variants, compositions comprising such variants and constructs, and methods of using the variants, constructs, and compositions. In some forms, the disclosed variants comprise the mutation Y239H, and do not comprise the mutation R245A. Such variants can be referred to as Y/H variants. Y/H variants are a preferred form of the disclosed variants. All of the preferred Cas9 protein variants retain the key amino acid substitution mutations E782K, N968K and R1015H.

In some forms, the disclosed variant does not include any mutation or combination of mutations such that the variant has greater off-target activity than a control SaCas9 variant or a control Cas9 variant. In some forms, the disclosed variant does not include any mutation or combination of mutations that result in the variant having greater off-target activity than SaCas9 variant v3.2. In some forms, the off-target activity is measured in a GFP disruption assay. In some forms, the measurement is taken at 15 days. In some forms, the assay is performed in cells harboring a reporter construct expressing an off-target sgRNA. In some forms, the cells harboring the reporter construct expressing the off-target sgRNA are OVCAR8-ADR. In some forms, the off-target sgRNA has the sequence CACCTACGGCAATCTGACCCTGAAGT (SEQ ID NO:1). In some forms, the control SaCas9 variant comprises the mutations N419D, R654A, G655A, E782K, N968K, and R1015H. In some forms, the control SaCas9 variant has only the mutations N419D, R654A, G655A, E782K, N968K, and R1015H. In some forms, the SaCas9 variant is variant 3.2.

In some forms, the disclosed variant does not include any other mutation or combination of other mutations such that the variant has greater off-target activity than SaCas9 variant v3.2 in a GFP disruption assay at 15 days in OVCAR8-ADR cells harboring a reporter construct expressing an off-target sgRNA having the sequence CACCTACGGCAATCTGACCCTGAAGT (SEQ ID NO:1), wherein the SaCas9 variant v3.2 has only the mutations N419D, R654A, G655A, E782K, N968K, and R1015H.

In some forms, the disclosed variant does not include any mutation or combination of mutations such that the variant has greater on-target activity than a control SaCas9 variant or a control Cas9 variant. In some forms, the disclosed variant does not include any mutation or combination of mutations that result in the variant having greater on-target activity than SaCas9 variant KKH-SaCas9. In some forms, the on-target activity is measured in a GFP disruption assay. In some forms, the measurement is taken at 15 days. In some forms, the assay is performed in cells harboring a reporter construct expressing an on-target sgRNA. In some forms, the cells harboring the reporter construct expressing the on-target sgRNA are OVCAR8-ADR. In some forms, the on-target sgRNA has the sequence CACCTACGGCAAGCTGACCCTGAAGT (SEQ ID NO.2). In some forms, the control SaCas9 variant comprises the mutations E782K, N968K, and R1015H. In some forms, the control SaCas9 variant has only the mutations E782K, N968K, and R1015H. In some forms, the SaCas9 variant is KKH-SaCas9.

In some forms, the disclosed variant does not include any other mutation or combination of other mutations such that the variant has on-target activity less than 0.5 of the on-target activity of SaCas9 variant KKH-SaCas9 in a GFP disruption assay at 15 days in OVCAR8-ADR cells harboring a reporter construct expressing an on-target sgRNA having the sequence CACCTACGGCAAGCTGACCCTGAAGT (SEQ ID NO:2), wherein the SaCas9 variant KKH-SaCas9 has only the mutations E782K, N968K, and R1015H.

In some forms, the disclosed variant can further comprise one or more mutations selected from the group consisting of T238A, T392A, N394T, N394A, N413A, Q414R, N419A, N419D, N419S, N419G, R499A, Q500A, Y651H, R654A, and G655A. In some forms, the disclosed variant can include the mutation N419D. In some forms, the disclosed variant can include the mutation N419S. In some forms, the disclosed variant can include the mutation N419G. In some forms, the disclosed variant can include the mutation R499A. the mutation Q500A. In some forms, the disclosed variant can include the mutation Y651H. In some forms, the disclosed variant can include the mutation R654A. In some forms, the disclosed variant can include the mutation G655A. In some forms, the disclosed variant can include the mutation Q414R. In some forms, the disclosed variant can include the mutation N394T. In some forms, the disclosed variant can include the mutation N394A. In some forms, the disclosed variant can include the mutation T392A. In some forms, the disclosed variant can include the mutation T238A.

In some forms, the disclosed variant can include one or more mutations selected from the group consisting of R499A, Q500A, Y651H, R654A, and G655A. In some forms, the disclosed variant is v3.18, v3.8, v3.22, v3.10 (SAV1; SEQ ID NO:80), v3.16 (SAV2; SEQ. ID NO. 81), v3.24, or v3.19.

The SaCas9 wild-type amino acid sequence is as follows (corresponding to UniProtKB/Swiss-Prot No. J7RUA5.1) (SEQ ID NO:79):

```
 1   Met  Lys  Arg  Asn  Tyr  Ile  Leu  Gly  Leu  Asp   10
11   Ile  Gly  Ile  Thr  Ser  Val  Gly  Tyr  Gly  Ile   20
21   Ile  Asp  Tyr  Glu  Thr  Arg  Asp  Val  Ile  Asp   30
31   Ala  Gly  Val  Arg  Leu  Phe  Lys  Glu  Ala  Asn   40
```

| | | | | | | | | | | | |
|---|---|---|---|---|---|---|---|---|---|---|---|
| 41 | Val | Glu | Asn | Asn | Glu | Gly | Arg | Arg | Ser | Lys | 50 |
| 51 | Arg | Gly | Ala | Arg | Arg | Leu | Lys | Arg | Arg | Arg | 60 |
| 61 | Arg | His | Arg | Ile | Gln | Arg | Val | Lys | Lys | Leu | 70 |
| 71 | Leu | Phe | Asp | Tyr | Asn | Leu | Leu | Thr | Asp | His | 80 |
| 81 | Ser | Glu | Leu | Ser | Gly | Ile | Asn | Pro | Tyr | Glu | 90 |
| 91 | Ala | Arg | Val | Lys | Gly | Leu | Ser | Gln | Lys | Leu | 100 |
| 101 | Ser | Glu | Glu | Glu | Phe | Ser | Ala | Ala | Leu | Leu | 110 |
| 111 | His | Leu | Ala | Lys | Arg | Arg | Gly | Val | His | Asn | 120 |
| 121 | Val | Asn | Glu | Val | Glu | Glu | Asp | Thr | Gly | Asn | 130 |
| 131 | Glu | Leu | Ser | Thr | Lys | Glu | Gln | Ile | Ser | Arg | 140 |
| 141 | Asn | Ser | Lys | Ala | Leu | Glu | Glu | Lys | Tyr | Val | 150 |
| 151 | Ala | Glu | Leu | Gln | Leu | Glu | Arg | Leu | Lys | Lys | 160 |
| 161 | Asp | Gly | Glu | Val | Arg | Gly | Ser | Ile | Asn | Arg | 170 |
| 171 | Phe | Lys | Thr | Ser | Asp | Tyr | Val | Lys | Glu | Ala | 180 |
| 181 | Lys | Gln | Leu | Leu | Lys | Val | Gln | Lys | Ala | Tyr | 190 |
| 191 | His | Gln | Leu | Asp | Gln | Ser | Phe | Ile | Asp | Thr | 200 |
| 201 | Tyr | Ile | Asp | Leu | Leu | Glu | Thr | Arg | Arg | Thr | 210 |
| 211 | Tyr | Tyr | Glu | Gly | Pro | Gly | Glu | Gly | Ser | Pro | 220 |
| 221 | Phe | Gly | Trp | Lys | Asp | Ile | Lys | Glu | Trp | Tyr | 230 |
| 231 | Glu | Met | Leu | Met | Gly | His | Cys | Thr | Tyr | Phe | 240 |
| 241 | Pro | Glu | Glu | Leu | Arg | Ser | Val | Lys | Tyr | Ala | 250 |
| 251 | Tyr | Asn | Ala | Asp | Leu | Tyr | Asn | Ala | Leu | Asn | 260 |
| 261 | Asp | Leu | Asn | Asn | Leu | Val | Ile | Thr | Arg | Asp | 270 |
| 271 | Glu | Asn | Glu | Lys | Leu | Glu | Tyr | Tyr | Glu | Lys | 280 |
| 281 | Phe | Gln | Ile | Ile | Glu | Asn | Val | Phe | Lys | Gln | 290 |
| 291 | Lys | Lys | Lys | Pro | Thr | Leu | Lys | Gln | Ile | Ala | 300 |
| 301 | Lys | Glu | Ile | Leu | Val | Asn | Glu | Glu | Asp | Ile | 310 |
| 311 | Lys | Gly | Tyr | Arg | Val | Thr | Ser | Thr | Gly | Lys | 320 |
| 321 | Pro | Glu | Phe | Thr | Asn | Leu | Lys | Val | Tyr | His | 330 |
| 331 | Asp | Ile | Lys | Asp | Ile | Thr | Ala | Arg | Lys | Glu | 340 |
| 341 | Ile | Ile | Glu | Asn | Ala | Glu | Leu | Leu | Asp | Gln | 350 |
| 351 | Ile | Ala | Lys | Ile | Leu | Thr | Ile | Tyr | Gln | Ser | 360 |
| 361 | Ser | Glu | Asp | Ile | Gln | Glu | Glu | Leu | Thr | Asn | 370 |
| 371 | Leu | Asn | Ser | Glu | Leu | Thr | Gln | Glu | Glu | Ile | 380 |
| 381 | Glu | Gln | Ile | Ser | Asn | Leu | Lys | Gly | Tyr | Thr | 390 |
| 391 | Gly | Thr | His | Asn | Leu | Ser | Leu | Lys | Ala | Ile | 400 |
| 401 | Asn | Leu | Ile | Leu | Asp | Glu | Leu | Trp | His | Thr | 410 |

| | | | | | | | | | | | |
|---|---|---|---|---|---|---|---|---|---|---|---|
| 411 | Asn | Asp | Asn | Gln | Ile | Ala | Ile | Phe | Asn | Arg | 420 |
| 421 | Leu | Lys | Leu | Val | Pro | Lys | Lys | Val | Asp | Leu | 430 |
| 431 | Ser | Gln | Gln | Lys | Glu | Ile | Pro | Thr | Thr | Leu | 440 |
| 441 | Val | Asp | Asp | Phe | Ile | Leu | Ser | Pro | Val | Val | 450 |
| 451 | Lys | Arg | Ser | Phe | Ile | Gln | Ser | Ile | Lys | Val | 460 |
| 461 | Ile | Asn | Ala | Ile | Ile | Lys | Lys | Tyr | Gly | Leu | 470 |
| 471 | Pro | Asn | Asp | Ile | Ile | Ile | Glu | Leu | Ala | Arg | 480 |
| 481 | Glu | Lys | Asn | Ser | Lys | Asp | Ala | Gln | Lys | Met | 490 |
| 491 | Ile | Asn | Glu | Met | Gln | Lys | Arg | Asn | Arg | Gln | 500 |
| 501 | Thr | Asn | Glu | Arg | Ile | Glu | Glu | Ile | Ile | Arg | 510 |
| 511 | Thr | Thr | Gly | Lys | Glu | Asn | Ala | Lys | Tyr | Leu | 520 |
| 521 | Ile | Glu | Lys | Ile | Lys | Leu | His | Asp | Met | Gln | 530 |
| 531 | Glu | Gly | Lys | Cys | Leu | Tyr | Ser | Leu | Glu | Ala | 540 |
| 541 | Ile | Pro | Leu | Glu | Asp | Leu | Leu | Asn | Asn | Pro | 550 |
| 551 | Phe | Asn | Tyr | Glu | Val | Asp | His | Ile | Ile | Pro | 560 |
| 561 | Arg | Ser | Val | Ser | Phe | Asp | Asn | Ser | Phe | Asn | 570 |
| 571 | Asn | Lys | Val | Leu | Val | Lys | Gln | Glu | Glu | Asn | 580 |
| 581 | Ser | Lys | Lys | Gly | Asn | Arg | Thr | Pro | Phe | Gln | 590 |
| 591 | Tyr | Leu | Ser | Ser | Ser | Asp | Ser | Lys | Ile | Ser | 600 |
| 601 | Tyr | Glu | Thr | Phe | Lys | Lys | His | Ile | Leu | Asn | 610 |
| 611 | Leu | Ala | Lys | Gly | Lys | Gly | Arg | Ile | Ser | Lys | 620 |
| 621 | Thr | Lys | Lys | Glu | Tyr | Leu | Leu | Glu | Glu | Arg | 630 |
| 631 | Asp | Ile | Asn | Arg | Phe | Ser | Val | Gln | Lys | Asp | 640 |
| 641 | Phe | Ile | Asn | Arg | Asn | Leu | Val | Asp | Thr | Arg | 650 |
| 651 | Tyr | Ala | Thr | Arg | Gly | Leu | Met | Asn | Leu | Leu | 660 |
| 661 | Arg | Ser | Tyr | Phe | Arg | Val | Asn | Asn | Leu | Asp | 670 |
| 671 | Val | Lys | Val | Lys | Ser | Ile | Asn | Gly | Gly | Phe | 680 |
| 681 | Thr | Ser | Phe | Leu | Arg | Arg | Lys | Trp | Lys | Phe | 690 |
| 691 | Lys | Lys | Glu | Arg | Asn | Lys | Gly | Tyr | Lys | His | 700 |
| 701 | His | Ala | Glu | Asp | Ala | Leu | Ile | Ile | Ala | Asn | 710 |
| 711 | Ala | Asp | Phe | Ile | Phe | Lys | Glu | Trp | Lys | Lys | 720 |
| 721 | Leu | Asp | Lys | Ala | Lys | Lys | Val | Met | Glu | Asn | 730 |
| 731 | Gln | Met | Phe | Glu | Glu | Lys | Gln | Ala | Glu | Ser | 740 |
| 741 | Met | Pro | Glu | Ile | Glu | Thr | Glu | Gln | Glu | Tyr | 750 |
| 751 | Lys | Glu | Ile | Phe | Ile | Thr | Pro | His | Gln | Ile | 760 |
| 761 | Lys | His | Ile | Lys | Asp | Phe | Lys | Asp | Tyr | Lys | 770 |
| 771 | Tyr | Ser | His | Arg | Val | Asp | Lys | Lys | Pro | Asn | 780 |

| | | | | | | | | | | | |
|---|---|---|---|---|---|---|---|---|---|---|---|
| 781 | Arg | Glu | Leu | Ile | Asn | Asp | Thr | Leu | Tyr | Ser | 790 |
| 791 | Thr | Arg | Lys | Asp | Asp | Lys | Gly | Asn | Thr | Leu | 800 |
| 801 | Ile | Val | Asn | Asn | Leu | Asn | Gly | Leu | Tyr | Asp | 810 |
| 811 | Lys | Asp | Asn | Asp | Lys | Leu | Lys | Lys | Leu | Ile | 820 |
| 821 | Asn | Lys | Ser | Pro | Glu | Lys | Leu | Leu | Met | Tyr | 830 |
| 831 | His | His | Asp | Pro | Gln | Thr | Tyr | Gln | Lys | Leu | 840 |
| 841 | Lys | Leu | Ile | Met | Glu | Gln | Tyr | Gly | Asp | Glu | 850 |
| 851 | Lys | Asn | Pro | Leu | Tyr | Lys | Tyr | Tyr | Glu | Glu | 860 |
| 861 | Thr | Gly | Asn | Tyr | Leu | Thr | Lys | Tyr | Ser | Lys | 870 |
| 871 | Lys | Asp | Asn | Gly | Pro | Val | Ile | Lys | Lys | Ile | 880 |
| 881 | Lys | Tyr | Tyr | Gly | Asn | Lys | Leu | Asn | Ala | His | 890 |
| 891 | Leu | Asp | Ile | Thr | Asp | Asp | Tyr | Pro | Asn | Ser | 900 |
| 901 | Arg | Asn | Lys | Val | Val | Lys | Leu | Ser | Leu | Lys | 910 |
| 911 | Pro | Tyr | Arg | Phe | Asp | Val | Tyr | Leu | Asp | Asn | 920 |
| 921 | Gly | Val | Tyr | Lys | Phe | Val | Thr | Val | Lys | Asn | 930 |
| 931 | Leu | Asp | Val | Ile | Lys | Lys | Glu | Asn | Tyr | Tyr | 940 |
| 941 | Glu | Val | Asn | Ser | Lys | Cys | Tyr | Glu | Glu | Ala | 950 |
| 951 | Lys | Lys | Leu | Lys | Lys | Ile | Ser | Asn | Gln | Ala | 960 |
| 961 | Glu | Phe | Ile | Ala | Ser | Phe | Tyr | Asn | Asn | Asp | 970 |
| 971 | Leu | Ile | Lys | Ile | Asn | Gly | Glu | Leu | Tyr | Arg | 980 |
| 981 | Val | Ile | Gly | Val | Asn | Asn | Asp | Leu | Leu | Asn | 990 |
| 991 | Arg | Ile | Glu | Val | Asn | Met | Ile | Asp | Ile | Thr | 1000 |
| 1001 | Tyr | Arg | Glu | Tyr | Leu | Glu | Asn | Met | Asn | Asp | 1010 |
| 1011 | Lys | Arg | Pro | Pro | Arg | Ile | Ile | Lys | Thr | Ile | 1020 |
| 1021 | Ala | Ser | Lys | Thr | Gln | Ser | Ile | Lys | Lys | Tyr | 1030 |
| 1031 | Ser | Thr | Asp | Ile | Leu | Gly | Asn | Leu | Tyr | Glu | 1040 |
| 1041 | Val | Lys | Ser | Lys | Lys | His | Pro | Gln | Ile | Ile | 1050 |
| 1051 | Lys | Lys | Gly | | | | | | | | |

The DNA sequence for v3.10 (SAV1) is as follows (SEQ ID NO:131);

1 AAGCGGAACT ACATCCTGGG CCTGGACATC GGCATCACCA GCGTGGGCTA CGGCATCATC
61 GACTACGAGA CACGGGACGT GATCGATGCC GGCGTGCGGC TGTTCAAAGA GGCCAACGTG
121 GAAAACAACG AGGGCAGGCG GAGCAAGAGA GGCGCCAGAA GGCTGAAGCG GCGGAGGCGG
181 CATAGAATCC AGAGAGTGAA GAAGCTGCTG TTCGACTACA ACCTGCTGAC CGACCACAGC
241 GAGCTGAGCG GCATCAACCC CTACGAGGCC AGAGTGAAGG GCCTGAGCCA GAAGCTGAGC
301 GAGGAAGAGT TCTCTGCCGC CCTGCTGCAC CTGGCCAAGA GAAGAGGCGT GCACAACGTG
361 AACGAGGTGG AAGAGGACAC CGGCAACGAG CTGTCCACCA AAGAGCAGAT CAGCCGGAAC
421 AGCAAGGCCC TGGAAGAGAA ATACGTGGCC GAACTGCAGC TGGAACGGCT GAAGAAAGAC

481 GGCGAAGTGC GGGGCAGCAT CAACAGATTC AAGACCAGCG ACTACGTGAA AGAAGCCAAA
541 CAGCTGCTGA AGGTGCAGAA GGCCTACCAC CAGCTGGACC AGAGCTTCAT CGACACCTAC
601 ATCGACCTGC TGGAAACCCG GCGGACCTAC TATGAGGGAC CTGGCGAGGG CAGCCCCTTC
661 GGCTGGAAGG ACATCAAAGA ATGGTACGAG ATGCTGATGG GCCACTGCAC CCACTTCCCC
721 GAGGAACTGC GGAGCGTGAA GTACGCCTAC AACGCCGACC TGTACAACGC CCTGAACGAC
781 CTGAACAATC TCGTGATCAC CAGGGACGAG AACGAGAAGC TGGAATATTA CGAGAAGTTC
841 CAGATCATCG AGAACGTGTT CAAGCAGAAG AAGAAGCCCA CCCTGAAGCA GATCGCCAAA
901 GAAATCCTCG TGAACGAAGA GGATATTAAG GGCTACAGAG TGACCAGCAC CGGCAAGCCC
961 GAGTTCACCA ACCTGAAGGT GTACCACGAC ATCAAGGACA TTACCGCCCG GAAAGAGATT
1021 ATTGAGAACG CCGAGCTGCT GGATCAGATT GCCAAGATCC TGACCATCTA CCAGAGCAGC
1081 GAGGACATCC AGGAAGAACT GACCAATCTG AACTCCGAGC TGACCCAGGA AGAGATCGAG
1141 CAGATCTCTA ATCTGAAGGG CTATACCGGC ACCCACAACC TGAGCCTGAA GGCCATCAAC
1201 CTGATCCTGG ACGAGCTGTG GCACACCAAC GACAACCAGA TCGCTATCTT CGACCGGCTG
1261 AAGCTGGTGC CCAAGAAGGT GGACCTGTCC CAGCAGAAAG AGATCCCCAC CACCCTGGTG
1321 GACGACTTCA TCCTGAGCCC CGTCGTGAAG AGAAGCTTCA TCCAGAGCAT CAAAGTGATC
1381 AACGCCATCA TCAAGAAGTA CGGCCTGCCC AACGACATCA TTATCGAGCT GGCCCGCGAG
1441 AAGAACTCCA AGGACGCCCA GAAAATGATC AACGAGATGC AGAAGCGGAA CCGCCAGCACC
1501 AACGAGCGGA TCGAGGAAAT CATCCGGACC ACCGGCAAAG AGAACGCCAA GTACCTGATC
1561 GAGAAGATCA AGCTGCACGA CATGCAGGAA GGCAAGTGCC TATACAGCCT GGAAGCCATC
1621 CCTCTGGAAG ATCTGCTGAA CAACCCCTTC AACTATGAGG TGGACCACAT CATCCCCAGA
1681 AGCGTGTCCT TCGACAACAG CTTCAACAAC AAGGTGCTCG TGAAGCAGGA AGAAAACAGC
1741 AAGAAGGGCA ACCGGACCCC ATTCCAGTAC CTGAGCAGCA GCGACAGCAA GATCAGCTAC
1801 GAAACCTTCA AGAAGCACAT CCTGAATCTG GCCAAGGGCA AGGGCAGAAT CAGCAAGACC
1861 AAGAAAGAGT ATCTGCTGGA AGAACGGGAC ATCAACAGGT TCTCCGTGCA GAAAGACTTC
1921 ATCAACCGGA ACCTGGTGGA TACCAGACAC GCCACCAGAG GCCTGATGAA CCTGCTGCGG
1981 AGCTACTTCA GAGTGAACAA CCTGGACGTG AAAGTGAAGT CCATCAATGG CGGCTTCACC
2041 AGCTTTCTGC GGCGGAAGTG GAAGTTTAAG AAAGAGCGGA ACAAGGGGTA CAAGCACCAC
2101 GCCGAGGACG CCCTGATCAT TGCCAACGCC GATTTCATCT TCAAAGAGTG GAAGAAACTG
2161 GACAAGGCCA AAAAAGTGAT GGAAAACCAG ATGTTCGAGG AAAAGCAGGC CGAGAGCATG
2221 CCCGAGATCG AAACCGAGCA GGAGTACAAA GAGATCTTCA TCACCCCCCA CCAGATCAAG
2281 CACATTAAGG ACTTCAAGGA CTACAAGTAC AGCCATCGGG TGGACAAGAA GCCTAATAGA
2341 AAGCTGATTA ACGACACCCT GTACTCCACC CGGAAGGACG ACAAGGGCAA CACCCTGATC
2401 GTGAACAATC TGAACGGCCT GTACGACAAG GACAATGACA AGCTGAAAAA GCTGATCAAC
2461 AAGAGCCCCG AAAAGCTGCT GATGTACCAC CACGACCCCC AGACCTACCA GAAACTGAAG
2521 CTGATTATGG AACAGTACGG CGACGAGAAG AATCCCCTGT ACAAGTACTA CGAGGAAACC
2581 GGGAACTACC TGACCAAGTA CTCCAAAAAG GACAACGGCC CCGTGATCAA GAAGATTAAG
2641 TATTACGGCA ACAAACTGAA CGCCCATCTG GACATCACCG ACGACTACCC CAACAGCAGA
2701 AACAAGGTCG TGAAGCTGTC CCTGAAGCCC TACAGATTCG ACGTGTACCT GGACAATGGC
2761 GTGTACAAGT TCGTGACCGT GAAGAATCTG GATGTGATCA AAAAAGAAAA CTACTACGAA
2821 GTGAATAGCA AGTGCTATGA GGAAGCTAAG AAGCTGAAGA AGATCAGCAA CCAGGCCGAG
2881 TTTATCGCCT CCTTCTACAA GAACGATCTG ATCAAGATCA ACGGCGAGCT GTATAGAGTG

2941 ATCGGCGTGA ACAACGACCT GCTGAACCGG ATCGAAGTGA ACATGATCGA CATCACCTAC
3001 CGCGAGTACC TGGAAAACAT GAACGACAAG AGGCCCCCCC ACATCATTAA GACAATCGCC
3061 TCCAAGACCC AGAGCATTAA GAAGTACAGC ACAGACATTC TGGGCAACCT GTATGAAGTG
3121 AAATCTAAGA AGCACCCTCA GATCATCAAA AAGGGC

The amino acid sequence for v3.10 (SAV1) is as follows (SEQ ID NO:80):

| | | | | | | | | | | |
|---|---|---|---|---|---|---|---|---|---|---|
| 1 | Met | Lys | Arg | Asn | Tyr | Ile | Leu | Gly | Leu | Asp | 10 |
| 11 | Ile | Gly | Ile | Thr | Ser | Val | Gly | Tyr | Gly | Ile | 20 |
| 21 | Ile | Asp | Tyr | Glu | Thr | Arg | Asp | Val | Ile | Asp | 30 |
| 31 | Ala | Gly | Val | Arg | Leu | Phe | Lys | Glu | Ala | Asn | 40 |
| 41 | Val | Glu | Asn | Asn | Glu | Gly | Arg | Arg | Ser | Lys | 50 |
| 51 | Arg | Gly | Ala | Arg | Arg | Leu | Lys | Arg | Arg | Arg | 60 |
| 61 | Arg | His | Arg | Ile | Gln | Arg | Val | Lys | Lys | Leu | 70 |
| 70 | Leu | Phe | Asp | Tyr | Asn | Leu | Leu | Thr | Asp | His | 80 |
| 81 | Ser | Glu | Leu | Ser | Gly | Ile | Asn | Pro | Tyr | Glu | 90 |
| 91 | Ala | Arg | Val | Lys | Gly | Leu | Ser | Gln | Lys | Leu | 100 |
| 101 | Ser | Glu | Glu | Glu | Phe | Ser | Ala | Ala | Leu | Leu | 110 |
| 111 | His | Leu | Ala | Lys | Arg | Arg | Gly | Val | His | Asn | 120 |
| 121 | Val | Asn | Glu | Val | Glu | Glu | Asp | Thr | Gly | Asn | 130 |
| 131 | Glu | Leu | Ser | Thr | Lys | Glu | Gln | Ile | Ser | Arg | 140 |
| 141 | Asn | Ser | Lys | Ala | Leu | Glu | Glu | Lys | Tyr | Val | 150 |
| 151 | Ala | Glu | Leu | Gln | Leu | Glu | Arg | Leu | Lys | Lys | 160 |
| 161 | Asp | Gly | Glu | Val | Arg | Gly | Ser | Ile | Asn | Arg | 170 |
| 171 | Phe | Lys | Thr | Ser | Asp | Tyr | Val | Lys | Glu | Ala | 180 |
| 181 | Lys | Gln | Leu | Leu | Lys | Val | Gln | Lys | Ala | Tyr | 190 |
| 191 | His | Gln | Leu | Asp | Gln | Ser | Phe | Ile | Asp | Thr | 200 |
| 201 | Tyr | Ile | Asp | Leu | Leu | Glu | Thr | Arg | Arg | Thr | 210 |
| 211 | Tyr | Tyr | Glu | Gly | Pro | Gly | Glu | Gly | Ser | Pro | 220 |
| 221 | Phe | Gly | Trp | Lys | Asp | Ile | Lys | Glu | Trp | Tyr | 230 |
| 231 | Glu | Met | Leu | Met | Gly | His | Cys | Thr | His | Phe | 240 |
| 241 | Pro | Glu | Glu | Leu | Arg | Ser | Val | Lys | Tyr | Ala | 250 |
| 251 | Tyr | Asn | Ala | Asp | Leu | Tyr | Asn | Ala | Leu | Asn | 260 |
| 261 | Asp | Leu | Asn | Asn | Leu | Val | Ile | Thr | Arg | Asp | 270 |
| 271 | Glu | Asn | Glu | Lys | Leu | Glu | Tyr | Tyr | Glu | Lys | 280 |
| 281 | Phe | Gln | Ile | Ile | Glu | Asn | Val | Phe | Lys | Gln | 290 |
| 291 | Lys | Lys | Lys | Pro | Thr | Leu | Lys | Gln | Ile | Ala | 300 |
| 301 | Lys | Glu | Ile | Leu | Val | Asn | Glu | Glu | Asp | Ile | 310 |
| 311 | Lys | Gly | Tyr | Arg | Val | Thr | Ser | Thr | Gly | Lys | 320 |

| | | | | | | | | | | |
|---|---|---|---|---|---|---|---|---|---|---|
| 321 | Pro | Glu | Phe | Thr | Asn | Leu | Lys | Val | Tyr | His | 330 |
| 331 | Asp | Ile | Lys | Asp | Ile | Thr | Ala | Arg | Lys | Glu | 340 |
| 341 | Ile | Ile | Glu | Asn | Ala | Glu | Leu | Leu | Asp | Gln | 350 |
| 351 | Ile | Ala | Lys | Ile | Leu | Thr | Ile | Tyr | Gln | Ser | 360 |
| 361 | Ser | Glu | Asp | Ile | Gln | Glu | Glu | Leu | Thr | Asn | 370 |
| 371 | Leu | Asn | Ser | Glu | Leu | Thr | Gln | Glu | Glu | Ile | 380 |
| 381 | Glu | Gln | Ile | Ser | Asn | Leu | Lys | Gly | Tyr | Thr | 390 |
| 391 | Gly | Thr | His | Asn | Leu | Ser | Leu | Lys | Ala | Ile | 400 |
| 401 | Asn | Leu | Ile | Leu | Asp | Glu | Leu | Trp | His | Thr | 410 |
| 411 | Asn | Asp | Asn | Gln | Ile | Ala | Ile | Phe | Asp | Arg | 420 |
| 421 | Leu | Lys | Leu | Val | Pro | Lys | Lys | Val | Asp | Leu | 430 |
| 431 | Ser | Gln | Gln | Lys | Glu | Ile | Pro | Thr | Thr | Leu | 440 |
| 441 | Val | Asp | Asp | Phe | Ile | Leu | Ser | Pro | Val | Val | 450 |
| 451 | Lys | Arg | Ser | Phe | Ile | Gln | Ser | Ile | Lys | Val | 460 |
| 461 | Ile | Asn | Ala | Ile | Ile | Lys | Lys | Tyr | Gly | Leu | 470 |
| 471 | Pro | Asn | Asp | Ile | Ile | Ile | Glu | Leu | Ala | Arg | 480 |
| 481 | Glu | Lys | Asn | Ser | Lys | Asp | Ala | Gln | Lys | Met | 490 |
| 491 | Ile | Asn | Glu | Met | Gln | Lys | Arg | Asn | Ala | Ala | 500 |
| 501 | Thr | Asn | Glu | Arg | Ile | Glu | Glu | Ile | Ile | Arg | 510 |
| 511 | Thr | Thr | Gly | Lys | Glu | Asn | Ala | Lys | Tyr | Leu | 520 |
| 521 | Ile | Glu | Lys | Ile | Lys | Leu | His | Asp | Met | Gln | 530 |
| 531 | Glu | Gly | Lys | Cys | Leu | Tyr | Ser | Leu | Glu | Ala | 540 |
| 541 | Ile | Pro | Leu | Glu | Asp | Leu | Leu | Asn | Asn | Pro | 550 |
| 551 | Phe | Asn | Tyr | Glu | Val | Asp | His | Ile | Ile | Pro | 560 |
| 561 | Arg | Ser | Val | Ser | Phe | Asp | Asn | Ser | Phe | Asn | 570 |
| 571 | Asn | Lys | Val | Leu | Val | Lys | Gln | Glu | Glu | Asn | 580 |
| 581 | Ser | Lys | Lys | Gly | Asn | Arg | Thr | Pro | Phe | Gln | 590 |
| 591 | Tyr | Leu | Ser | Ser | Ser | Asp | Ser | Lys | Ile | Ser | 600 |
| 601 | Tyr | Glu | Thr | Phe | Lys | Lys | His | Ile | Leu | Asn | 610 |
| 611 | Leu | Ala | Lys | Gly | Lys | Gly | Arg | Ile | Ser | Lys | 620 |
| 621 | Thr | Lys | Lys | Glu | Tyr | Leu | Leu | Glu | Glu | Arg | 630 |
| 631 | Asp | Ile | Asn | Arg | Phe | Ser | Val | Gln | Lys | Asp | 640 |
| 641 | Phe | Ile | Asn | Arg | Asn | Leu | Val | Asp | Thr | Arg | 650 |
| 651 | His | Ala | Thr | Arg | Gly | Leu | Met | Asn | Leu | Leu | 660 |
| 661 | Arg | Ser | Tyr | Phe | Arg | Val | Asn | Asn | Leu | Asp | 670 |
| 671 | Val | Lys | Val | Lys | Ser | Ile | Asn | Gly | Gly | Phe | 680 |
| 681 | Thr | Ser | Phe | Leu | Arg | Arg | Lys | Trp | Lys | Phe | 690 |

691 Lys Lys Glu Arg Asn Lys Gly Tyr Lys His 700
701 His Ala Glu Asp Ala Leu Ile Ile Ala Asn 710
711 Ala Asp Phe Ile Phe Lys Glu Trp Lys Lys 720
721 Leu Asp Lys Ala Lys Lys Val Met Glu Asn 730
731 Gln Met Phe Glu Glu Lys Gln Ala Glu Ser 740
741 Met Pro Glu Ile Glu Thr Glu Gln Glu Tyr 750
751 Lys Glu Ile Phe Ile Thr Pro His Gln Ile 760
761 Lys His Ile Lys Asp Phe Lys Asp Tyr Lys 770
771 Tyr Ser His Arg Val Asp Lys Lys Pro Asn 780
781 Arg Lys Leu Ile Asn Asp Thr Leu Tyr Ser 790
791 Thr Arg Lys Asp Asp Lys Gly Asn Thr Leu 800
801 Ile Val Asn Asn Leu Asn Gly Leu Tyr Asp 810
811 Lys Asp Asn Asp Lys Leu Lys Lys Leu Ile 820
821 Asn Lys Ser Pro Glu Lys Leu Leu Met Tyr 830
831 His His Asp Pro Gln Thr Tyr Gln Lys Leu 840
841 Lys Leu Ile Met Glu Gln Tyr Gly Asp Glu 850
851 Lys Asn Pro Leu Tyr Lys Tyr Tyr Glu Glu 860
861 Thr Gly Asn Tyr Leu Thr Lys Tyr Ser Lys 870
871 Lys Asp Asn Gly Pro Val Ile Lys Lys Ile 880
881 Lys Tyr Tyr Gly Asn Lys Leu Asn Ala His 890
891 Leu Asp Ile Thr Asp Asp Tyr Pro Asn Ser 900
901 Arg Asn Lys Val Val Lys Leu Ser Leu Lys 910
911 Pro Tyr Arg Phe Asp Val Tyr Leu Asp Asn 920
921 Gly Val Tyr Lys Phe Val Thr Val Lys Asn 930
931 Leu Asp Val Ile Lys Lys Glu Asn Tyr Tyr 940
941 Glu Val Asn Ser Lys Cys Tyr Glu Glu Ala 950
951 Lys Lys Leu Lys Lys Ile Ser Asn Gln Ala 960
961 Glu Phe Ile Ala Ser Phe Tyr Lys Asn Asp 970
971 Leu Ile Lys Ile Asn Gly Glu Leu Tyr Arg 980
981 Val Ile Gly Val Asn Asn Asp Leu Leu Asn 990
991 Arg Ile Glu Val Asn Met Ile Asp Ile Thr 1000
1001 Tyr Arg Glu Tyr Leu Glu Asn Met Asn Asp 1010
1011 Lys Arg Pro Pro His Ile Ile Lys Thr Ile 1020
1021 Ala Ser Lys Thr Gln Ser Ile Lys Lys Tyr 1030
1031 Ser Thr Asp Ile Leu Gly Asn Leu Tyr Glu 1040
1041 Val Lys Ser Lys Lys His Pro Gln Ile Ile 1050
1051 Lys Lys Gly 1060

The DNA sequence for variant 3.16 (SAV2) is as follows (SEQ ID NO:132):

```
1    AAGCGGAACT ACATCCTGGG CCTGGACATC GGCATCACCA GCGTGGGCTA CGGCATCATC
61   GACTACGAGA CACGGGACGT GATCGATGCC GGCGTGCGGC TGTTCAAAGA GGCCAACGTG
121  GAAAACAACG AGGGCAGGCG GAGCAAGAGA GGCGCCAGAA GGCTGAAGCG GCGGAGGCGG
181  CATAGAATCC AGAGAGTGAA GAAGCTGCTG TTCGACTACA ACCTGCTGAC CGACCACAGC
241  GAGCTGAGCG GCATCAACCC CTACGAGGCC AGAGTGAAGG GCCTGAGCCA GAAGCTGAGC
301  GAGGAAGAGT TCTCTGCCGC CCTGCTGCAC CTGGCCAAGA GAAGAGGCGT GCACAACGTG
361  AACGAGGTGG AAGAGGACAC CGGCAACGAG CTGTCCACCA AAGAGCAGAT CAGCCGGAAC
421  AGCAAGGCCC TGGAAGAGAA ATACGTGGCC GAACTGCAGC TGGAACGGCT GAAGAAAGAC
481  GGCGAAGTGC GGGGCAGCAT CAACAGATTC AAGACCAGCG ACTACGTGAA AGAAGCCAAA
541  CAGCTGCTGA AGGTGCAGAA GGCCTACCAC CAGCTGGACC AGAGCTTCAT CGACACCTAC
601  ATCGACCTGC TGGAAACCCG GCGGACCTAC TATGAGGGAC CTGGCGAGGG CAGCCCCTTC
661  GGCTGGAAGG ACATCAAAGA ATGGTACGAG ATGCTGATGG GCCACTGCAC CCACTTCCCC
721  GAGGAACTGC GGAGCGTGAA GTACGCCTAC AACGCCGACC TGTACAACGC CCTGAACGAC
781  CTGAACAATC TCGTGATCAC CAGGGACGAG AACGAGAAGC TGGAATATTA CGAGAAGTTC
841  CAGATCATCG AGAACGTGTT CAAGCAGAAG AAGAAGCCCA CCCTGAAGCA GATCGCCAAA
901  GAAATCCTCG TGAACGAAGA GGATATTAAG GGCTACAGAG TGACCAGCAC CGGCAAGCCC
961  GAGTTCACCA ACCTGAAGGT GTACCACGAC ATCAAGGACA TTACCGCCCG GAAAGAGATT
1021 ATTGAGAACG CCGAGCTGCT GGATCAGATT GCCAAGATCC TGACCATCTA CCAGAGCAGC
1081 GAGGACATCC AGGAAGAACT GACCAATCTG AACTCCGAGC TGACCCAGGA AGAGATCGAG
1141 CAGATCTCTA ATCTGAAGGG CTATACCGGC ACCCACAACC TGAGCCTGAA GGCCATCAAC
1201 CTGATCCTGG ACGAGCTGTG GCACACCAAC GACAACCAGA TCGCTATCTT CGACCGGCTG
1261 AAGCTGGTGC CCAAGAAGGT GGACCTGTCC CAGCAGAAAG AGATCCCCAC CACCCTGGTG
1321 GACGACTTCA TCCTGAGCCC CGTCGTGAAG AGAAGCTTCA TCCAGAGCAT CAAAGTGATC
1381 AACGCCATCA TCAAGAAGTA CGGCCTGCCC AACGACATCA TTATCGAGCT GGCCCGCGAG
1441 AAGAACTCCA AGGACGCCCA GAAAATGATC AACGAGATGC AGAAGCGGAA CCGGCAGACC
1501 AACGAGCGGA TCGAGGAAAT CATCCGGACC ACCGGCAAAG AGAACGCCAA GTACCTGATC
1561 GAGAAGATCA AGCTGCACGA CATGCAGGAA GGCAAGTGCC TATACAGCCT GGAAGCCATC
1621 CCTCTGGAAG ATCTGCTGAA CAACCCCTTC AACTATGAGG TGGACCACAT CATCCCCAGA
1681 AGCGTGTCCT TCGACAACAG CTTCAACAAC AAGGTGCTCG TGAAGCAGGA AGAAAACAGC
1741 AAGAAGGGCA ACCGGACCCC ATTCCAGTAC CTGAGCAGCA GCGACAGCAA GATCAGCTAC
1801 GAAACCTTCA AGAAGCACAT CCTGAATCTG GCCAAGGGCA AGGGCAGAAT CAGCAAGACC
1861 AAGAAAGAGT ATCTGCTGGA AGAACGGGAC ATCAACAGGT TCTCCGTGCA GAAAGACTTC
1921 ATCAACCGGA ACCTGGTGGA TACCAGATAC GCCACCGCCG CCCTGATGAA CCTGCTGCGG
1981 AGCTACTTCA GAGTGAACAA CCTGGACGTG AAAGTGAAGT CCATCAATGG CGGCTTCACC
2041 AGCTTTCTGC GGCGGAAGTG GAAGTTTAAG AAAGAGCGGA ACAAGGGGTA CAAGCACCAC
2101 GCCGAGGACG CCCTGATCAT TGCCAACGCC GATTTCATCT TCAAAGAGTG GAAGAAACTG
2161 GACAAGGCCA AAAAAGTGAT GGAAAACCAG ATGTTCGAGG AAAAGCAGGC CGAGAGCATG
2221 CCCGAGATCG AAACCGAGCA GGAGTACAAA GAGATCTTCA TCACCCCCCA CCAGATCAAG
```

```
2281 CACATTAAGG ACTTCAAGGA CTACAAGTAC AGCCATCGGG TGGACAAGAA GCCTAATAGA
2341 AAGCTGATTA ACGACACCCT GTACTCCACC CGGAAGGACG ACAAGGGCAA CACCCTGATC
2401 GTGAACAATC TGAACGGCCT GTACGACAAG GACAATGACA AGCTGAAAAA GCTGATCAAC
2461 AAGAGCCCCG AAAAGCTGCT GATGTACCAC CACGACCCCC AGACCTACCA GAAACTGAAG
2521 CTGATTATGG AACAGTACGG CGACGAGAAG AATCCCCTGT ACAAGTACTA CGAGGAAACC
2581 GGGAACTACC TGACCAAGTA CTCCAAAAAG GACAACGGCC CCGTGATCAA GAAGATTAAG
2641 TATTACGGCA ACAAACTGAA CGCCCATCTG GACATCACCG ACGACTACCC CAACAGCAGA
2701 AACAAGGTCG TGAAGCTGTC CCTGAAGCCC TACAGATTCG ACGTGTACCT GGACAATGGC
2761 GTGTACAAGT TCGTGACCGT GAAGAATCTG GATGTGATCA AAAAAGAAAA CTACTACGAA
2821 GTGAATAGCA AGTGCTATGA GGAAGCTAAG AAGCTGAAGA AGATCAGCAA CCAGGCCGAG
2881 TTTATCGCCT CCTTCTACAA GAACGATCTG ATCAAGATCA ACGGCGAGCT GTATAGAGTG
2941 ATCGGCGTGA ACAACGACCT GCTGAACCGG ATCGAAGTGA ACATGATCGA CATCACCTAC
3001 CGCGAGTACC TGGAAAACAT GAACGACAAG AGGCCCCCCC ACATCATTAA GACAATCGCC
3061 TCCAAGACCC AGAGCATTAA GAAGTACAGC ACAGACATTC TGGGCAACCT GTATGAAGTG
3121 AAATCTAAGA AGCACCCTCA GATCATCAAA AAGGGC
```

The amino acid sequence for variant 3.16 ("SAV2") is as follows (SEQ ID NO:81):

```
  1  Met Lys Arg Asn Tyr Ile Leu Gly Leu Asp  10
 11  Ile Gly Ile Thr Ser Val Gly Tyr Gly Ile  20
 21  Ile Asp Tyr Glu Thr Arg Asp Val Ile Asp  30
 31  Ala Gly Val Arg Leu Phe Lys Glu Ala Asn  40
 41  Val Glu Asn Asn Glu Gly Arg Arg Ser Lys  50
 51  Arg Gly Ala Arg Arg Leu Lys Arg Arg Arg  60
 61  Arg His Arg Ile Gln Arg Val Lys Lys Leu  70
 70  Leu Phe Asp Tyr Asn Leu Leu Thr Asp His  80
 81  Ser Glu Leu Ser Gly Ile Asn Pro Tyr Glu  90
 91  Ala Arg Val Lys Gly Leu Ser Gln Lys Leu 100
101  Ser Glu Glu Glu Phe Ser Ala Ala Leu Leu 110
.111 His Leu Ala Lys Arg Arg Gly Val His Asn 120
121  Val Asn Glu Val Glu Glu Asp Thr Gly Asn 130
131  Glu Leu Ser Thr Lys Glu Gln Ile Ser Arg 140
141  Asn Ser Lys Ala Leu Glu Glu Lys Tyr Val 150
151  Ala Glu Leu Gln Leu Glu Arg Leu Lys Lys 160
161  Asp Gly Glu Val Arg Gly Ser Ile Asn Arg 170
171  Phe Lys Thr Ser Asp Tyr Val Lys Glu Ala 180
181  Lys Gln Leu Leu Lys Val Gln Lys Ala Tyr 190
191  His Gln Leu Asp Gln Ser Phe Ile Asp Thr 200
201  Tyr Ile Asp Leu Leu Glu Thr Arg Arg Thr 210
```

| | | | | | | | | | | | |
|---|---|---|---|---|---|---|---|---|---|---|---|
| 211 | Tyr | Tyr | Glu | Gly | Pro | Gly | Glu | Gly | Ser | Pro | 220 |
| 221 | Phe | Gly | Trp | Lys | Asp | Ile | Lys | Glu | Trp | Tyr | 230 |
| 231 | Glu | Met | Leu | Met | Gly | His | Cys | Thr | His | Phe | 240 |
| 241 | Pro | Glu | Glu | Leu | Arg | Ser | Val | Lys | Tyr | Ala | 250 |
| 251 | Tyr | Asn | Ala | Asp | Leu | Tyr | Asn | Ala | Leu | Asn | 260 |
| 261 | Asp | Leu | Asn | Asn | Leu | Val | Ile | Thr | Arg | Asp | 270 |
| 271 | Glu | Asn | Glu | Lys | Leu | Glu | Tyr | Tyr | Glu | Lys | 280 |
| 281 | Phe | Gln | Ile | Ile | Glu | Asn | Val | Phe | Lys | Gln | 290 |
| 291 | Lys | Lys | Lys | Pro | Thr | Leu | Lys | Gln | Ile | Ala | 300 |
| 301 | Lys | Glu | Ile | Leu | Val | Asn | Glu | Glu | Asp | Ile | 310 |
| 311 | Lys | Gly | Tyr | Arg | Val | Thr | Ser | Thr | Gly | Lys | 320 |
| 321 | Pro | Glu | Phe | Thr | Asn | Leu | Lys | Val | Tyr | His | 330 |
| 331 | Asp | Ile | Lys | Asp | Ile | Thr | Ala | Arg | Lys | Glu | 340 |
| 341 | Ile | Ile | Glu | Asn | Ala | Glu | Leu | Leu | Asp | Gln | 350 |
| 351 | Ile | Ala | Lys | Ile | Leu | Thr | Ile | Tyr | Gln | Ser | 360 |
| 361 | Ser | Glu | Asp | Ile | Gln | Glu | Glu | Leu | Thr | Asn | 370 |
| 371 | Leu | Asn | Ser | Glu | Leu | Thr | Gln | Glu | Glu | Ile | 380 |
| 381 | Glu | Gln | Ile | Ser | Asn | Leu | Lys | Gly | Tyr | Thr | 390 |
| 391 | Gly | Thr | His | Asn | Leu | Ser | Leu | Lys | Ala | Ile | 400 |
| 401 | Asn | Leu | Ile | Leu | Asp | Glu | Leu | Trp | His | Thr | 410 |
| 411 | Asn | Asp | Asn | Gln | Ile | Ala | Ile | Phe | Asp | Arg | 420 |
| 421 | Leu | Lys | Leu | Val | Pro | Lys | Lys | Val | Asp | Leu | 430 |
| 431 | Ser | Gln | Gln | Lys | Glu | Ile | Pro | Thr | Thr | Leu | 440 |
| 441 | Val | Asp | Asp | Phe | Ile | Leu | Ser | Pro | Val | Val | 450 |
| 451 | Lys | Arg | Ser | Phe | Ile | Gln | Ser | Ile | Lys | Val | 460 |
| 461 | Ile | Asn | Ala | Ile | Ile | Lys | Lys | Tyr | Gly | Leu | 470 |
| 471 | Pro | Asn | Asp | Ile | Ile | Ile | Glu | Leu | Ala | Arg | 480 |
| 481 | Glu | Lys | Asn | Ser | Lys | Asp | Ala | Gln | Lys | Met | 490 |
| 491 | Ile | Asn | Glu | Met | Gln | Lys | Arg | Asn | Arg | Arg | 500 |
| 501 | Thr | Asn | Glu | Arg | Ile | Glu | Glu | Ile | Ile | Arg | 510 |
| 511 | Thr | Thr | Gly | Lys | Glu | Asn | Ala | Lys | Tyr | Leu | 520 |
| 521 | Ile | Glu | Lys | Ile | Lys | Leu | His | Asp | Met | Gln | 530 |
| 531 | Glu | Gly | Lys | Cys | Leu | Tyr | Ser | Leu | Glu | Ala | 540 |
| 541 | Ile | Pro | Leu | Glu | Asp | Leu | Leu | Asn | Asn | Pro | 550 |
| 551 | Phe | Asn | Tyr | Glu | Val | Asp | His | Ile | Ile | Pro | 560 |
| 561 | Arg | Ser | Val | Ser | Phe | Asp | Asn | Ser | Phe | Asn | 570 |
| 571 | Asn | Lys | Val | Leu | Val | Lys | Gln | Glu | Glu | Asn | 580 |

| | | | | | | | | | | | |
|---|---|---|---|---|---|---|---|---|---|---|---|
| 581 | Ser | Lys | Lys | Gly | Asn | Arg | Thr | Pro | Phe | Gln | 590 |
| 591 | Tyr | Leu | Ser | Ser | Ser | Asp | Ser | Lys | Ile | Ser | 600 |
| 601 | Tyr | Glu | Thr | Phe | Lys | Lys | His | Ile | Leu | Asn | 610 |
| 611 | Leu | Ala | Lys | Gly | Lys | Gly | Arg | Ile | Ser | Lys | 620 |
| 621 | Thr | Lys | Lys | Glu | Tyr | Leu | Leu | Glu | Glu | Arg | 630 |
| 631 | Asp | Ile | Asn | Arg | Phe | Ser | Val | Gln | Lys | Asp | 640 |
| 641 | Phe | Ile | Asn | Arg | Asn | Leu | Val | Asp | Thr | Arg | 650 |
| 651 | Tyr | Ala | Thr | Ala | Ala | Leu | Met | Asn | Leu | Leu | 660 |
| 661 | Arg | Ser | Tyr | Phe | Arg | Val | Asn | Asn | Leu | Asp | 670 |
| 671 | Val | Lys | Val | Lys | Ser | Ile | Asn | Gly | Gly | Phe | 680 |
| 681 | Thr | Ser | Phe | Leu | Arg | Arg | Lys | Trp | Lys | Phe | 690 |
| 691 | Lys | Lys | Glu | Arg | Asn | Lys | Gly | Tyr | Lys | His | 700 |
| 701 | His | Ala | Glu | Asp | Ala | Leu | Ile | Ile | Ala | Asn | 710 |
| 711 | Ala | Asp | Phe | Ile | Phe | Lys | Glu | Trp | Lys | Lys | 720 |
| 721 | Leu | Asp | Lys | Ala | Lys | Lys | Val | Met | Glu | Asn | 730 |
| 731 | Gln | Met | Phe | Glu | Glu | Lys | Gln | Ala | Glu | Ser | 740 |
| 741 | Met | Pro | Glu | Ile | Glu | Thr | Glu | Gln | Glu | Tyr | 750 |
| 751 | Lys | Glu | Ile | Phe | Ile | Thr | Pro | His | Gln | Ile | 760 |
| 761 | Lys | His | Ile | Lys | Asp | Phe | Lys | Asp | Tyr | Lys | 770 |
| 771 | Tyr | Ser | His | Arg | Val | Asp | Lys | Lys | Pro | Asn | 780 |
| 781 | Arg | Lys | Leu | Ile | Asn | Asp | Thr | Leu | Tyr | Ser | 790 |
| 791 | Thr | Arg | Lys | Asp | Asp | Lys | Gly | Asn | Thr | Leu | 800 |
| 801 | Ile | Val | Asn | Asn | Leu | Asn | Gly | Leu | Tyr | Asp | 810 |
| 811 | Lys | Asp | Asn | Asp | Lys | Leu | Lys | Lys | Leu | Ile | 820 |
| 821 | Asn | Lys | Ser | Pro | Glu | Lys | Leu | Leu | Met | Tyr | 830 |
| 831 | His | His | Asp | Pro | Gln | Thr | Tyr | Gln | Lys | Leu | 840 |
| 841 | Lys | Leu | Ile | Met | Glu | Gln | Tyr | Gly | Asp | Glu | 850 |
| 851 | Lys | Asn | Pro | Leu | Tyr | Lys | Tyr | Tyr | Glu | Glu | 860 |
| 861 | Thr | Gly | Asn | Tyr | Leu | Thr | Lys | Tyr | Ser | Lys | 870 |
| 871 | Lys | Asp | Asn | Gly | Pro | Val | Ile | Lys | Lys | Ile | 880 |
| 881 | Lys | Tyr | Tyr | Gly | Asn | Lys | Leu | Asn | Ala | His | 890 |
| 891 | Leu | Asp | Ile | Thr | Asp | Asp | Tyr | Pro | Asn | Ser | 900 |
| 901 | Arg | Asn | Lys | Val | Val | Lys | Leu | Ser | Leu | Lys | 910 |
| 911 | Pro | Tyr | Arg | Phe | Asp | Val | Tyr | Leu | Asp | Asn | 920 |
| 921 | Gly | Val | Tyr | Lys | Phe | Val | Thr | Val | Lys | Asn | 930 |
| 931 | Leu | Asp | Val | Ile | Lys | Lys | Glu | Asn | Tyr | Tyr | 940 |
| 941 | Glu | Val | Asn | Ser | Lys | Cys | Tyr | Glu | Glu | Ala | 950 |

| | | | | | | | | | | | |
|---|---|---|---|---|---|---|---|---|---|---|---|
| 951 | Lys | Lys | Leu | Lys | Lys | Ile | Ser | Asn | Gln | Ala | 960 |
| 961 | Glu | Phe | Ile | Ala | Ser | Phe | Tyr | Lys | Asn | Asp | 970 |
| 971 | Leu | Ile | Lys | Ile | Asn | Gly | Glu | Leu | Tyr | Arg | 980 |
| 981 | Val | Ile | Gly | Val | Asn | Asn | Asp | Leu | Leu | Asn | 990 |
| 991 | Arg | Ile | Glu | Val | Asn | Met | Ile | Asp | Ile | Thr | 1000 |
| 1001 | Tyr | Arg | Glu | Tyr | Leu | Glu | Asn | Met | Asn | Asp | 1010 |
| 1011 | Lys | Arg | Pro | Pro | His | Ile | Ile | Lys | Thr | Ile | 1020 |
| 1021 | Ala | Ser | Lys | Thr | Gln | Ser | Ile | Lys | Lys | Tyr | 1030 |
| 1031 | Ser | Thr | Asp | Ile | Leu | Gly | Asn | Leu | Tyr | Glu | 1040 |
| 1041 | Val | Lys | Ser | Lys | Lys | His | Pro | Gln | Ile | Ile | 1050 |
| 1051 | Lys | Lys | Gly | | | | | | | | 1060 |

According to some embodiments, the wild-type Cas9 is derived from *Micrococcus, Staphylococcus, Planoeoccus, Streptococcus, Leuconostoc, Pediococcus, Aerococcus or Gemella. Preferably, Staphylococcus includes Staphylococcus aureus, Staphylococcus epidermidis, Staphylococcus haemolyticu, Staphylococcus stimulans, Staphylococcus sp. HMSC061G12, and Staphylococcus saprophyticus. Preferably, Streptococcus includes Streptococcus pyogenes, Streptococcus equismilis, Streptococcus zooepidemicus, Streptococcus equi, Streptococcus dysgalactiae, Streptococcus sanguis, Streptococcus pneumoniae, Streptococcus anginosus, Streptococcus agalactiae, Streptococcus acidominimus, Streptococcus salivarius, Streptococcus mitis, Streptococcus bovis, Streptococcus equinus, Streptococcus thermophilus, Streptococcus faecalis, Streptococcus faecium, Streptococcus avium, Streptococcus uberis, Streptococcus lactis, Streptococcus cremoris and Streptococcus canis.* Preferably, the wild-type Cas9 is derived from *Staphylococcus aureus* (i.e., SaCas9).

According to some embodiments, the ortholog of *Staphylococcus aureus* is selected from *Absiella dolichum, Clostridium coleatum, Veillonella parvula, Alkalibacterium gilvum, Alkalibacterium sp. 20, Lacticigenium naphtae, Alkalibacterium subtropicum, Carnobacterium iners, Carnobacterium viridans, Jeotgalibaca sp., Listeria ivanovii sp. londoniensis, Bacillus massilionigeriensis, Bacillus niameyensis, Ureibacillus thermosphaericus-1, Ureibacillus thermosphaericus-2, Halakalibacillus halophilus, Paraliobacillus ryukyuensis, Sediminibacillus albus, Virgibacillus senegalensis, Pelagirhabdus alkalitolerans, Massilibacterium senegalense, Macrococcus* sp., *Staphylococcus* (from multispecies) , *Staphylococcus simulans, Staphylococcus sp., Staphylococcus massiliensis, Staphylococcus microti, Staphylococcus haemolyticus, Staphylococcus sp., Staphylococcus warneri, Staphylococcus schleiferi, Staphylococcus agnetis and Staphylococcus lutrae.*

A Cas9 endonuclease variant may comprise an amino acid sequence that is at least about 80% to 95% identical to the amino acid sequence of the parent Cas9 endonuclease.

Given that certain amino acids share similar structural and/or charge features with each other (i.e., conserved), the amino acid at each position in a Cas9 can be as provided in the disclosed sequences or substituted with a radical amino acid residue ("radical amino acid substitution" or "radical amino acid replacement"). A radical amino acid substitution is an amino acid replacement that exchanges an amino acid by a final amino acid with different physiochemical properties and typically include substitutions of amino acids in the groups below with an amino acid from outside of that group: glycine, alanine; valine, isoleucine, leucine; histidine; aspartic acid, glutamic acid, asparagine, glutamine; serine, threonine; lysine, arginine; and phenylalanine, tyrosine.

In some embodiments, the KKH-SaCas9 variants can include mutations at one or more of the following positions: T238, Y239, R245, N260, T392, N394, N413, Q414, N419, R499, Q500, Y651, R654 or G655. In some embodiments, the SaCas9 variants include one or more of the following mutations: T238A, Y239H, Y239R, R245A, R245K, N260D, T392A, N394T, N394A, N413A, Q414R, N419A, N419D, N419S, N419G, R499A, Q500A, Y651H, R654A and G655A. In some embodiments, the SaCas9 variants are at least 80% to 95% identical to the amino acid sequence of SEQ ID NO: 1 with mutations at one or more of the following positions: T238, Y239, R245, T392, N394, Q414, N419, R499, Q500, Y651, R654 or G655. In preferred embodiments, the variant retains desired activity of the parent, e.g., the nuclease activity and/or the ability to interact with a guide RNA and target DNA.

In some embodiments, the SaCas9-KKH variant includes an amino acid sequence that is at least 95% identical to the amino acid sequence of a wild-type Cas9. In some embodiments, the SaCas9-KKH variant includes a Cas9; a protospacer adjacent motif (PAM) interaction region, and having at least 80% sequence identity, and preferably 95% sequence identity, to the PAM interaction regions of the ortholog of the wild-type Cas9; Cas9; wherein an N-terminus of the PAM interaction region is connected to a C-terminus of the first backbone region, and a C-terminus of the PAM interaction region is connected to an N-terminus of the second backbone region, and wherein the Cas9 variant has recognition capability at the PAM sequence "NNNRRT" where N is adenine (A), thymine (T), cytosine (C) or guanine (G) and R is an adenine (A) or guanine (G).

In some embodiments, the SaCas9 variants include one of the following sets of mutations: Y239H/N419D/R499A/Q500A/Y651H/E782K/N968K/R1015H (SAV1 variant); Y239H/N419D/R654A/G655A/E782K/N968K/R1015H (SAV2 variant); or R245A/N413A/N419A/R654A (SaCas9-HF variant).

To determine the percent identity of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes is at least 80% of the length of the reference sequence, and in some embodiments is at least 90% or 100%. The nucleotides at corresponding amino acid positions or nucleotide positions are then compared. When a position in the first sequence is occupied by the same nucleotide as the corresponding position in the second sequence, then the molecules are identical at that position (as used herein nucleic acid "identity" is equivalent to nucleic acid "homology"). The percent identity between the two sequences is a function of the number of identical positions shared by the sequences, taking into account the number of gaps, and the length of each gap, which need to be introduced for optimal alignment of the two sequences. Percent identity between two polypeptides or nucleic acid sequences is determined in various ways that are within the skill in the art, for instance, using publicly available computer software such as Smith Waterman Alignment (Smith, T. F. and M. S. Waterman (1981) J Mol Biol 147:195-7); "BestFit" (Smith and Waterman, Advances in Applied Mathematics, 482-489 (1981)) as incorporated into GeneMatcher Plus, Schwarz and Dayhof (1979) Atlas of Protein Sequence and Structure, Dayhof, M. O., Ed, pp 353-358; BLAST program (Basic Local Alignment Search Tool; (Altschul, S. F., W. Gish, et al. (1990) J Mol Biol 215: 403-10), BLAST-2, BLAST-P, BLAST-N, BLAST-X, WU-BLAST-2, ALIGN, ALIGN-2, CLUSTAL, Geneious or Megalign (DNASTAR) software. In addition, those skilled in the art can determine appropriate parameters for measuring alignment, including any algorithms needed to achieve maximal alignment over the length of the sequences being compared. In general, for proteins or nucleic acids, the length of comparison can be any length, up to and including full length (e.g., 5%, 10%, 20%, 30%, 40%, 50%, 60%, 70%, 80%, 90%, 95%, or 100%). For purposes of the present compositions and methods, at least 80% of the full length of the sequence is aligned using the BLAST algorithm and the default parameters.

For purposes of the present disclosure, the comparison of sequences and determination of percent identity between two sequences can be accomplished using the following scoring matrix:

$$\frac{(Number\ of\ homologous\ nucleotide\ or\ amino\ acids)}{Number\ of\ total\ sequence\ length}\ x\ 100\%$$

### 1. Expression Vectors

In some embodiments, vectors can be designed for the expression of Cas9 variant transcripts from nucleic acid transcripts, proteins or enzymes encoding them in prokaryotic and/or eukaryotic cells. This can be done in various ways. For example, nucleic acid transcripts encoding Cas9 variants can be expressed in bacterial cells such as *Escherichia coli,* insect cells (using *baculovirus* expression vectors), yeast cells or mammalian cells.

In some embodiments, nucleic acids encoding the Cas9 variants can be cloned into an intermediate vector for transformation into a prokaryotic or eukaryotic cell for replication and/or expression (e.g., amplifying a plasmid as part of a viral vector packaging system). In some embodiments, the nucleic acid encoding the Cas9 variant can also be cloned into an expression vector for administration to plant cells, animal cells, mammalian or human cells, fungal cells, bacterial cells, or protozoal cells. Preferably, the nucleic acid encoding the Cas9 variant is cloned into an expression vector for administration to human cells.

Expression of genetically engineered proteins in prokaryotes is most often carried out in *Escherichia coli, Baccillus sp.* and *Salmonella* with vectors containing a transcription unit or expression cassette with all the elements required for the expression of the nucleic acid in host cells. Preferably, the expression vector is *Escherichia coli.* A typical expression cassette thus contains a promoter operably linked, e.g., to the nucleic acid sequence encoding the Cas9 variant, and any signals required, e.g., for efficient polyadenylation of the transcript, transcriptional termination, ribosome binding sites, or translation termination. Additional elements of the cassette may include, e.g., enhancers, and heterologous spliced intronic signals.

The promoter used to direct expression of a nucleic acid depends on the application. For example, a strong constitutive promoter is typically used for expression and purification of fusion proteins. In contrast, when the Cas9 variant is to be administered in vivo for gene regulation, either a constitutive or an inducible promoter can be used, depending on the particular use of the Cas9 variant. In addition, a preferred promoter for administration of the Cas9 variant can be a weak promoter, such as HSV TK or a promoter having similar activity. The promoter can also include elements that are responsive to transactivation, e.g., hypoxia response elements, Gal4 response elements, lac repressor response element, and small molecule control systems such as tetracycline-regulated systems and the RU-486 system (see, e.g., Gossen & Bujard, 1992, Proc. Natl. Acad. Sci. USA, 89:5547; Oligino et al., 1998, Gene Ther., 5:491-496; Wang et al., 1997, Gene Ther., 4:432-441; Neering et al., 1996, Blood, 88:1147-55; and Rendahl et al., 1998, Nat. Biotechnol., 16:757-761). Other expression systems can also be used, such as HRE, Lac, and Tet.

Disclosed are constructs encoding any of the disclosed variants for expression of the variant in a host of interest. In some forms, the construct can comprise sequences for expression of the variant in the host of interest. In some forms, the construct can further encode an sgRNA targeting a sequence of interest and sequences for expression of the sgRNA in the host of interest. In some forms, the construct can be comprised in a virus vector. In some forms, the virus vector can be an adeno-associated virus vector.

The disclosed compositions and methods are applicable to numerous areas including, but not limited to, gene targeting and editing to activate and/or repress genes, thereby regulating gene function. Other uses include medicine e.g., gene therapy, prognostic and predictive biomarker identification and drug development, biotechnology e.g., production of genetically modified plants and food such as stress-resistant crops, and research e.g., altering the epigenetic landscape and production of relevant animal models. Other uses are disclosed, apparent from the disclosure, and/or will be understood by those in the art.

**Methods**

The disclosed variants can be used for any suitable purpose and in any suitable method. Generally, the disclosed variants can be used to cleave target DNA of interest. Such cleavage is preferably used in a method of editing the target DNA of interest. For example, the disclosed variants can be used for and in any known methods of DNA editing, including in vitro and in vivo DNA editing. RNA-guided endonucleases, of which the disclosed variants are new forms, can be and have been used for various DNA cleavage and editing methods and the disclosed variants can be used as the RNA-guided endonuclease in any of these methods uses. For example, the disclosed variants can be used for altering the genome of a cell. Various methods for selectively altering the genome of a cell using RNA-guided endonucleases are described in the following exemplary U.S. Patent documents: U.S. Patent Nos. 8,993,233, 9,023,649, and 8,697,359 and U.S. Patent Application Publication Nos. 20140186958, 20160024529, 20160024524, 20160024523, 20160024510, 20160017366, 20160017301, 20150376652, 20150356239, 20150315576, 20150291965, 20150252358, 20150247150, 20150232883, 20150232882, 20150203872, 20150191744, 20150184139, 20150176064, 20150167000, 20150166969, 20150159175, 20150159174, 20150093473, 20150079681, 20150067922, 20150056629, 20150044772, 20150024500, 20150024499, 20150020223, 20140356867, 20140295557, 20140273235, 20140273226, 20140273037, 20140189896, 20140113376, 20140093941, 20130330778, 20130288251, 20120088676, 20110300538, 20110236530, 20110217739, 20110002889, 20100076057, 20110189776, 20110223638, 20130130248, 20150050699, 20150071899, 20150050699, 20150045546, 20150031134, 20150024500, 20140377868, 20140357530, 20140349400, 20140335620, 20140335063, 20140315985, 20140310830, 20140310828, 20140309487, 20140304853, 20140298547, 20140295556, 20140294773, 20140287938, 20140273234, 20140273232, 20140273231, 20140273230, 20140271987, 20140256046, 20140248702, 20140242702, 20140242700, 20140242699, 20140242664, 20140234972, 20140227787, 20140212869, 20140201857, 20140199767, 20140189896, 20140186958, 20140186919, 20140186843, 20140179770, 20140179006, 20140170753, and 20150071899, each of which is incorporated by reference herein, and in particular for their description of the uses of RNA-guided endonucleases.

Various methods for selectively altering the genome of a cell using RNA-guided endonucleases are described in the following exemplary publications: WO 2014/099744; WO 2014/089290; WO 2014/144592; WO 2014/004288; WO 2014/204578; WO 2014/152432; WO 2015/099850; WO 2008/108989; WO 2010/054108; WO 2012/164565; WO 2013/098244; WO 2013/176772; Makarova et al., "Evolution and classification of the CRISPR-Cas systems" 9(6) Nature Reviews Microbiology 467-477 (1-23) (June 2011); Wiedenheft et al., "RNA-guided genetic silencing systems in bacteria and archaea" 482 Nature 331-338 (Feb. 16, 2012); Gasiunas et al., "Cas9-crRNA ribonucleoprotein complex mediates specific DNA cleavage for adaptive immunity in bacteria" 109(39) Proceedings of the National Academy of Sciences USA E2579-E2586 (Sep. 4, 2012); Jinek et al., "A Programmable Dual-RNA-Guided DNA Endonuclease in Adaptive Bacterial Immunity" 337 Science 816-821 (Aug. 17, 2012); Carroll, "A CRISPR Approach to Gene Targeting" 20(9) Molecular Therapy 1658-1660 (September 2012); U.S. Appl. No. 61/652,086, filed May 25, 2012; Al-Attar et al., Clustered Regularly Interspaced Short Palindromic Repeats (CRISPRs): The Hallmark of an Ingenious Antiviral Defense Mechanism in Prokaryotes, Biol Chem. (2011) vol. 392, Issue 4, pp. 277-289; Hale et al., Essential Features and Rational Design of CRISPR RNAs That Function With the Cas RAMP Module Complex to Cleave RNAs, Molecular Cell, (2012) vol. 45, Issue 3, 292-302.

Disclosed are methods used to generate the SaCas9 variant constructs. SaCas9 variants were generated by applying repeating point mutations to DNA vectors harboring the KKH-SaCas9. A DNA vector fragment harboring part of the SaCas9 sequence was produced by digesting DNA vectors harboring the wild-type KKH-SaCas9 with specific restriction enzymes generating flanking sequences. A DNA insert fragment harboring part of the SaCas9 sequence which is not found in the digested vector fragment was a result of PCR amplification from the DNA vector harboring wild-type KKH-SaCas9 with primer DNA sequences harboring point mutations and digestion with restriction enzymes generating flanking sequences. The generation of intermediate SaCas9 variant vectors carried out by ligase conjunction with the above vector fragment and insert fragment. The SaCas9 variant constructs were generated by repeating digestion the intermediate SaCas9 variant vectors, PCR amplification from wild-type KKH-SaCas9 sequences with primer DNA with point mutations and ligation between intermediate SaCas9 variant vector and insert fragments until the sequence of SaCas9 variants are completed. The vectors containing completed sequence of SaCas9 variants were introduced to cells, OVCAR8-ADR, SK-N-MC, and MHCC97L for further characterization on their activities.

Disclosed are methods of editing a sequence of interest. In some forms, the method comprises contacting a disclosed construct with the host of interest, where the host of interest harbors the sequence of interest and where the cell expresses the construct to produce variant and the sgRNA. In some forms, the method comprises contacting a disclosed construct with the host of interest, where the host of interest harbors a sequence of interest and where the cell expresses the construct to produce the variant. In some forms, the method comprises contacting the sequence of interest with a disclosed mixture, whereby the variant edits the sequence of interest targeted by the sgRNA.

In some forms, the method can further comprises causing an sgRNA targeting the sequence of interest to be present in the host of interest with the produced variant, whereby the produced variant edits the sequence of interest targeted by the sgRNA.

### A. Administration/Contacting

The term "hit" refers to a test compound or material that shows desired properties in an assay. The terms "test compound" and "test material" refer to a compound or material to be tested by one or more screening method(s) for a desired activity. A test compound or a test material can be any compound or material such as an inorganic compound, an organic compound, a protein, a peptide, a carbohydrate, a lipid, a material, or a combination thereof. Usually, various predetermined concentrations of test compounds or test materials are used for screening, such as 0.01 micromolar, 1 micromolar and 10 micromolar. Test compound and test material controls can include the measurement of a signal in the absence of the test compound or comparison to a compound known to modulate the target.

The terms "high," "higher," "increases," "elevates," or "elevation" refer to increases above basal levels, e.g., as compared to a control. The terms "low," "lower," "reduces," or "reduction" refer to decreases below basal levels, e.g., as compared to a control.

The term "modulate" as used herein refers to the ability of a compound or material to change an activity in some measurable way as compared to an appropriate control. As a result of the presence of compounds or materials in the assays, activities can increase or decrease as compared to controls in the absence of these compounds or materials. Preferably, an increase in activity is at least 25%, more preferably at least 50%, most preferably at least 100% compared to the level of activity in the absence of the compound. Similarly, a decrease in activity is preferably at least 25%, more preferably at least 50%, most preferably at least 100% compared to the level of activity in the absence of the compound. A compound or material that increases a known activity is an "agonist." One that decreases, or prevents, a known activity is an "antagonist."

The term "inhibit" means to reduce or decrease in activity or expression. This can be a complete inhibition of activity or expression, or a partial inhibition. Inhibition can be compared to a control or to a standard level. Inhibition can be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64,65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%.

The term "monitoring" as used herein refers to any method in the art by which an activity can be measured.

The term "providing" as used herein refers to any means of adding a compound or material to something known in the art. Examples of providing can include the use of pipettes, pipettemen, syringes, needles, tubing, guns, etc. This can be manual or automated. It can include transfection by any mean or any other means of providing nucleic acids to dishes, cells, tissue, cell-free systems and can be *in vitro* or *in vivo*.

The term "contacting" refers to causing two or more objects to become in proximity to one another such that the objects are, or can come into, contact. The objects can be any compound, composition, material, component, etc. Mixing objects in a container, solution, or suspension can be a form of contacting. Administering an object to a subject can be considered contacting the object and subject. Similarly, administering an object to a subject such that the object can come into contact with a particular tissue, cell type, cell structure, protein, or other molecule in the subject can be a form of contacting.

The term "preventing" as used herein refers to administering a compound or material prior to the onset of clinical symptoms of a disease or conditions so as to prevent a physical manifestation of aberrations associated with the disease or condition.

The term "in need of treatment" as used herein refers to a judgment made by a caregiver (e.g. physician, nurse, nurse practitioner, or individual in the case of humans; veterinarian in the case of animals, including non-human mammals) that a subject requires or will benefit from treatment. This judgment is made based on a variety of factors that are in the realm of a care giver's expertise, but that include the knowledge that the subject is ill, or will be ill, as the result of a condition that is treatable by the disclosed variants and compositions and other materials comprising, containing, or embodying the variant.

As used herein, "subject" includes, but is not limited to, animals, plants, bacteria, viruses, parasites and any other organism or entity. The subject can be a vertebrate, more specifically a mammal (e.g., a human, horse, pig, rabbit, dog, sheep, goat, non-human primate, cow, cat, guinea pig or rodent), a fish, a bird or a reptile or an amphibian. The subject can be an invertebrate, more specifically an arthropod (e.g., insects and crustaceans). The term does not denote a particular age or sex. Thus, adult and newborn subjects, as well as fetuses, whether male or female, are intended to be covered. A patient refers to a subject afflicted with a disease or disorder. The term "patient" includes human and veterinary subjects.

By "treatment" and "treating" is meant the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitiative terms. Thus, for example, characteristics or features of a disease, pathological condition, or disorder and/or symptoms of a disease, pathological condition, or disorder can be reduced to any effect or to any amount.

A cell can be *in vitro.* Alternatively, a cell can be *in vivo* and can be found in a subject. A "cell" can be a cell from any organism including, but not limited to, a bacterium.

In one aspect, the disclosed variants and compositions and other materials comprising, containing, or embodying the variant can be administered to a subject comprising a human or an animal including, but not limited to, a mouse, dog, cat, horse, bovine or ovine and the like, that is in need of alleviation or amelioration from a recognized medical condition.

By the term "effective amount" of a compound or material as provided herein is meant a nontoxic but sufficient amount of the compound or material to provide the desired result. As will be pointed out below, the exact amount required will vary from subject to subject, depending on the species, age, and general condition of the subject, the severity of the disease that is being treated, the particular compound or material used, its mode of administration, and the like. Thus, it is not possible to specify an exact "effective amount." However, an appropriate effective amount can be determined by one of ordinary skill in the art using only routine experimentation.

The dosages or amounts of the compounds and materials described herein are large enough to produce the desired effect in the method by which delivery occurs. The dosage should not be so large as to cause adverse side effects, such as unwanted cross-reactions, anaphylactic reactions, and the like. Generally, the dosage will vary with the age, condition, sex and extent of the disease in the subject and can be determined by one of skill in the art. The dosage can be adjusted by the individual physician based on the clinical condition of the subject involved. The dose, schedule of doses and route of administration can be varied.

The efficacy of administration of a particular dose of the compounds or materials according to the methods described herein can be determined by evaluating the particular aspects of the medical history, signs, symptoms, and objective laboratory tests that are known to be useful in evaluating the status of a subject in need. These signs, symptoms, and objective laboratory tests will vary, depending upon the particular disease or condition being treated or prevented, as will be known to any clinician who treats such patients or a researcher conducting experimentation in this field. For example, if, based on a comparison with an appropriate control group and/or knowledge of the normal progression of the disease in the general population or the particular individual: (1) a subject's physical condition is shown to be improved (e.g., a tumor has partially or fully regressed), (2) the progression of the disease or condition is shown to be stabilized, or slowed, or reversed, or (3) the need for other medications for treating the disease or condition is lessened or obviated, then a particular treatment regimen will be considered efficacious.

By "pharmaceutically acceptable" is meant a material that is not biologically or otherwise undesirable, i.e., the material can be administered to a subject along with the selected compound or material without causing any undesirable biological effects or interacting in a deleterious manner with any of the other components of the pharmaceutical composition in which it is contained.

Any of the compounds or materials can be used therapeutically in combination with a pharmaceutically acceptable carrier. The compounds and materials described herein can be conveniently formulated into pharmaceutical compositions composed of one or more of the compounds in association with a pharmaceutically acceptable carrier. See, e.g., Remington's Pharmaceutical Sciences, latest edition, by E.W. Martin Mack Pub. Co., Easton, PA, which discloses typical carriers and conventional methods of preparing pharmaceutical compositions that can be used in conjunction with the preparation of formulations of the compounds described herein. These most typically would be standard carriers for administration of compositions to humans. In one aspect, humans and non-humans, including solutions such as sterile water, saline, and buffered solutions at physiological pH. Other compounds and materials can be administered according to standard procedures used by those skilled in the art.

The pharmaceutical compositions described herein can include, but are not limited to, carriers, thickeners, diluents, buffers, preservatives, surface active agents and the like in addition to the molecule of choice. Pharmaceutical compositions can also include one or more active ingredients such as antimicrobial agents, antiinflammatory agents, anesthetics, and the like.

The compounds, materials, and pharmaceutical compositions described herein can be administered to the subject in a number of ways depending on whether local or systemic treatment is desired, and on the area to be treated. Thus, for example, a compound, material, or pharmaceutical composition described herein can be administered as an ophthalmic solution and/or ointment to the surface of the eye. Moreover, a compound, material, or pharmaceutical composition can be administered to a subject vaginally, rectally, intranasally, orally, by inhalation, or parenterally, for example, by intradermal, subcutaneous, intramuscular, intraperitoneal, intrarectal, intraarterial, intralymphatic, intravenous, intrathecal and intratracheal routes. Parenteral administration, if used, is generally characterized by injection. Injectables can be prepared in conventional forms, either as liquid solutions or suspensions, solid forms suitable for solution or suspension in liquid prior to injection, or as emulsions. A more recently revised approach for parenteral administration involves use of a slow release or sustained release system such that a constant dosage is maintained. See, e.g., U.S. Patent No. 3,610,795.

Preparations for parenteral administration include sterile aqueous or non-aqueous solutions, suspensions, and emulsions which can also contain buffers, diluents and other suitable additives. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives can also be present such as, for example, antimicrobials, antioxidants, chelating agents, and inert gases and the like.

Formulations for topical administration can include ointments, lotions, creams, gels, drops, suppositories, sprays, liquids and powders. Conventional pharmaceutical carriers, aqueous, powder or oily bases, thickeners and the like can be necessary or desirable.

Compositions for oral administration can include powders or granules, suspensions or solutions in water or non-aqueous media, capsules, sachets, or tablets. Thickeners, flavorings, diluents, emulsifiers, dispersing aids or binders can be desirable.

The disclosed compositions and methods can be further understood through the following numbered paragraphs.

1. A SaCas9 variant comprising the mutation Y239H and not comprising the mutation R245A.
2. The variant of paragraph 1 further comprising the mutations E782K, N968K, and R1015H.
3. The variant of paragraph 1 or 2, wherein the variant does not include any other mutation or combination of other mutations such that the variant has greater off-target activity than SaCas9 variant v3.2 in a GFP disruption assay at 15 days in OVCAR8-ADR cells, SK-N-MC cells, or MHCC97L cells harboring a reporter construct expressing an off-target sgRNA having the sequence CACCTACGGCAATCTGACCCTGAAGT (SEQ ID NO: 1), wherein the SaCas9 variant v3.2 has only the mutations N419D, R654A, G655A, E782K, N968K, and R1015H.
4. The variant of any one of paragraphs 1-3, wherein the variant does not include any other mutation or combination of other mutations such that the variant has on-target activity less than 0.5 of the on-target activity of SaCas9 variant KKH-SaCas9 in a GFP disruption assay at 15 days in OVCAR8-ADR cells, SK-N-MC cells, or MHCC97L cells harboring a reporter construct expressing an on-target sgRNA having the sequence CACCTACGGCAAGCTGACCCTGAAGT (SEQ ID NO:2), wherein the SaCas9 variant KKH-SaCas9 has only the mutations E782K, N968K, and R1015H.

5. The variant of any one of paragraphs 1-4 further comprising one or more mutations selected from the group consisting of T238A, T392A, N394T, N394A, N413A, Q414R, N419A, N419D, N419S, N419G, R499A, Q500A, Y651H, R654A, and G655A.

6. The variant of any one of paragraphs 1-5 including the mutation N419D.

7. The variant of any one of paragraphs 1-5 including the mutation N419S.

8. The variant of any one of paragraphs 1-5 including the mutation N419G.

9. The variant of any one of paragraphs 1-8 including the mutation R499A.

10. The variant of any one of paragraphs 1-9 including the mutation Q500A.

11. The variant of any one of paragraphs 1-10 including the mutation Y651H.

12. The variant of any one of paragraphs 1-11 including the mutation R654A.

13. The variant of any one of paragraphs 1-12 including the mutation G655A.

14. The variant of any one of paragraphs 1-13 including the mutation Q414R.

15. The variant of any one of paragraphs 1-14 including the mutation N394T.

16. The variant of any one of paragraphs 1-14 including the mutation N394A.

17. The variant of any one of paragraphs 1-16 including the mutation T392A.

18. The variant of any one of paragraphs 1-17 including the mutation T238A.

19. The variant of any one of paragraphs 1-5 including one or more mutations selected from the group consisting of R499A, Q500A, Y651H, R654A, and G655A,

20. The variant of any one of paragraphs 1-5, wherein the variant is v3.18, v3.8, v3.22, v3.16, v3.10, v3.24, or v3.19.

21. A construct encoding the variant of any one of paragraphs 1-20 for expression of the variant in a host of interest.

22. The construct of paragraphs 21 comprising sequences for expression of the variant in the host of interest.

23. The construct of paragraph 21 or 22 further encoding an sgRNA targeting a sequence of interest and sequences for expression of the sgRNA in the host of interest.

24. The construct of any one of paragraphs 21-23 comprised in a virus vector.

25. The construct of paragraph 24, wherein the virus vector is an adeno-associated virus vector.

26. A method of editing a sequence of interest, the method comprising contacting the construct of any one of paragraphs 23-25 with the host of interest, wherein the host of interest harbors the sequence of interest, wherein the cell expresses the construct to produce variant and the sgRNA.

27. A method of editing a sequence of interest, the method comprising contacting the construct of paragraph 21 or 22 with the host of interest, wherein the host of interest harbors a sequence of interest, wherein the cell expresses the construct to produce the variant.

28. The method of paragraph 27 further comprising causing an sgRNA targeting the sequence of interest to be present in the host of interest with the produced variant, whereby the produced variant edits the sequence of interest targeted by the sgRNA.

29. A mixture comprising the variant of any one of paragraphs 1-20 and an sgRNA targeting a sequence of interest.

30. The mixture of paragraph 29 comprised in a delivery particle.

31. The mixture of paragraph 29 comprised in a cell containing the sequence of interest.

32. A method of editing a sequence of interest, the method comprising contacting the sequence of interest with a mixture of any one of paragraphs 29-31, whereby the variant edits the sequence of interest targeted by the sgRNA.

## Examples

The following examples as well as the figures are included to demonstrate preferred embodiments of the invention. Those of skill in the art will recognize or be able to ascertain using no more than routine experimentation, many equivalents to the specific embodiments of the method and compositions described herein. Such equivalents are intended to be encompassed by the claims.

## Materials and Methods

### *Construction of DNA vectors*

The vector constructs used in this study (Table 1) were generated using standard molecular cloning techniques, including PCR, restriction enzyme digestion, ligation, and Gibson assembly. Custom oligonucleotides were purchased from Genewiz. To create the expression vector encoding KKH-SaCas9-HF, KKH-efSaCas9, and KKH-eSaCas9, the SaCas9 sequences were amplified/mutated from Addgene #61591 and #117552 by PCR and cloned into the pFUGW lentiviral vector backbone. To construct the expression vector containing U6 promoter-driven expression of a sgRNA that targeted a specific locus, oligo pairs with the gRNA target sequences were synthesized, annealed, and cloned in the pFUGW-based vector. The gRNA spacer sequences are listed in Table 2. The constructs were transformed into *E. coli* strain DH5α, and 50 μg/ml of carbenicillin/ampicillin was used to isolate colonies harboring the constructs. DNA was extracted and purified using Plasmid Mini (Takara) or Midi (Qiagen) kits. Sequences of the vector constructs were verified with Sanger sequencing.

Table 1: List of Constructs Used in the Current Study

| Plasmid name | Description | Reference |
|---|---|---|
| AWp124 | KKH-SaCas9 expression vector | pFUGW-EFS-SaCas9 KKH-NheI-2A-BFP-BamHI-EcoRI |
| ZRp7b | GFP_on 1 sgRNA expression vector | pFUGW-UbC-RFP-CMV-EGFP-BamHI-U6-EGFP sg b-Sacas9 scaffold-EcoR1 |
| ZRp7g | GFP_on 2 sgRNA expression vector | pFUGW-UbC-RFP-CMV-EGFP-BamHI-U6-EGFP sg g-Sacas9 scaffold-EcoR1 |
| ZRp7L | GFP_off 1 sgRNA expression vector | pFUGW-UbC-RFP-CMV-EGFP-BamHI-U6-EGFP sg b off 1-Sacas9 scaffold-EcoR1 |
| ZRp7M | GFP_off 2 sgRNA expression vector | pFUGW-UbC-RFP-CMV-EGFP-BamHI-U6-EGFP sg b off 2-Sacas9 scaffold-EcoR1 |
| ZRp7N | GFP_off 3 sgRNA expression vector | pFUGW-UbC-RFP-CMV-EGFP-BamHI-U6-EGFP sg b off 3-Sacas9 scaffold-EcoR1 |
| ZRp9-Ver.3 | Mutated KKH-SaCas9 variants expression vector | pFUGW-SaCas9 Nter-mutations-SaCas9-Cter-NheI-2A-BFP-SA157-EcoRI |
| ZRp 15 | dSaCas9 expression vector | pFUGW-EFS-dSaCas9-NheI-2A-BFP-BamHI-EcoRI |
| ZRp19 | Mutated KKH-SaCas9 variants expression vector | pFUGW-SaCas9_2nd_mut(A to G)-NheI-2A-BFP |
| ZRp45-1 | SaCas9-HF expression vector | pFUGW-SaCas9_HF-NheI-2A-BFP |
| ZRp46-1 | KKH-SaCas9-HF expression vector | pFUGW-KKH_SaCas9_HF-NheI-2A-BFP |
| ZRp47(B)-1 | KKH-SaCas9-HF expression vector (no R245A) | pFUGW-KKH_SaCas9_HF-removed R245A-NheI-2A-BFP |
| ZRp48-2 | KKH-SaCas9-HF expression vector (no R245A, replace with Y239H) | pFUGW-KKH_SaCas9_HF-R245A replaced with Y239H-NheI-2A-BFP |
| ZRp49-2 | KKH-SaCas9-HF expression vector (addition with Y239H) | pFUGW-KKH_SaCas9_HF+Y239H-NheI-2A-BFP |
| ZRp9_v3_16 | KKH-SaCas9-SAV1 expression vector | pFUGW-KKH-SaCas9 Nter-ZRp9v3_16-Cter-NheI-2A-BFP-SA157-EcoRI-BC4-BC3-BC2-BC1 |
| ZRp9_v3_10 | KKH-SaCas9-SAV2 expression vector | pFUGW-KKH-SaCas9 Nter-ZRp9v3_10-Cter-NheI-2A-BFP-SA157-EcoRI-BC4-BC3-BC2-BC1 |
| ZRp50-3 | KKH-SaCas9-SAV2 expression vector (removed Y239H) | pFUGW-KKH_SaCas9_ZRp9v3-10-no Y239H-NheI-2A-BFP |
| ZRp51-2 | KKH-SaCas9-SAV2 expression vector (no Y239H, replace with R245A) | pFUGW-KKH_SaCas9_ZRp9v3-10-R245A replace Y239H-NheI-2A-BFP |
| CKp 19 | KKH-SaCas9-SAV1 + N260D expression vector | pFUGW-KKH_SaCas9_v3_16+N260N-2A-BFP |

(continued)

| Plasmid name | Description | Reference |
|---|---|---|
| CKp18 | KKH-SaCas9-SAV2 + N260D expression vector | pFUGW-KKH_SaCas9_v3_10+N260N-2A-BFP |
| ZRp54 | KKH-efSaCas9 expression vector | pFUGW-KKH-efSaCas9-NheI-2A-BFP |
| ZRp55 | efSaCas9 expression vector | pFUGW-efSaCas9-NheI-2A-BFP |
| CKP14 | KKH-dSaCas9-KRAB expression vector | pFUGW-EFSp-KKH-dSaCas9(D 10A/N580A)-BFP-KRAB |
| CKP15 | KKH-dSaCas9-SAV1-KRAB expression vector (dSAV1-KRAB) | pFUGW-EFSp-KKH-dSaCas9 v3 16 (D10A/N580A)-BFP-KRAB |
| CKP16 | KKH-dSaCas9-SAV2-KRAB expression vector (dSAV2-KRAB) | pFUGW-EFSp-KKH-dSaCas9_v3_10 (D10A/N580A)-BFP-KRAB |
| CKP17 | KKH-deSaCas9-KRAB expression vector | pFUGW-EFSp-KKH-deSaCas9(D 10A/N580A)-BFP-KRAB |
| ZRp66_M1 | GFP single mismatch sgRNA_1 expression vector | pFUGW-UbC-RFP-CMV-EGFP-BamHI-U6-EGFP sgb M1-Sacas9 scaffold-EcoR1 |
| ZRp66_M2 | GFP single mismatch sgRNA_2 expression vector | pFUGW-UbC-RFP-CMV-EGFP-BamHI-U6-EGFP sgb M2-Sacas9 scaffold-EcoR1 |
| ZRp66_M3 | GFP single mismatch sgRNA_3 expression vector | pFUGW-UbC-RFP-CMV-EGFP-BamHI-U6-EGFP sgb M3-Sacas9 scaffold-EcoR1 |
| ZRp66_M4 | GFP single mismatch sgRNA_4 expression vector | pFUGW-UbC-RFP-CMV-EGFP-BamHI-U6-EGFP sgb M4-Sacas9 scaffold-EcoR1 |
| ZRp66_M5 | GFP single mismatch sgRNA_5 expression vector | pFUGW-UbC-RFP-CMV-EGFP-BamHI-U6-EGFP sgb M5-Sacas9 scaffold-EcoR1 |
| ZRp66_M6 | GFP single mismatch sgRNA_6 expression vector | pFUGW-UbC-RFP-CMV-EGFP-BamHI-U6-EGFP sgb M6-Sacas9 scaffold-EcoR1 |
| ZRp66_M7 | GFP single mismatch sgRNA_7 expression vector | pFUGW-UbC-RFP-CMV-EGFP-BamHI-U6-EGFP sgb M7-Sacas9 scaffold-EcoR1 |
| ZRp66_M8 | GFP single mismatch sgRNA_8 expression vector | pFUGW-UbC-RFP-CMV-EGFP-BamHI-U6-EGFP sgb M8-Sacas9 scaffold-EcoR1 |
| ZRp66_M9 | GFP single mismatch sgRNA_9 expression vector | pFUGW-UbC-RFP-CMV-EGFP-BamHI-U6-EGFP sgb M9-Sacas9 scaffold-EcoR1 |
| ZRp66_M10 | GFP single mismatch sgRNA_10 expression vector | pFUGW-UbC-RFP-CMV-EGFP-BamHI-U6-EGFP sgb M10-Sacas9 scaffold-EcoR1 |
| ZRp66_M11 | GFP single mismatch sgRNA_11 expression vector | pFUGW-UbC-RFP-CMV-EGFP-BamHI-U6-EGFP sgb M11-Sacas9 scaffold-EcoR1 |
| ZRp66_M12 | GFP single mismatch sgRNA_12 expression vector | pFUGW-UbC-RFP-CMV-EGFP-BamHI-U6-EGFP sgb M12-Sacas9 scaffold-EcoR1 |
| ZRp66_M13 = ZRp7N | GFP single mismatch sgRNA_13 expression vector | pFUGW-UbC-RFP-CMV-EGFP-BamHI-U6-EGFP sgb off1_7N-Sacas9 scaffold-EcoR1 |
| ZRp66_M14 | GFP single mismatch sgRNA_14 expression vector | pFUGW-UbC-RFP-CMV-EGFP-BamHI-U6-EGFP sgb M14-Sacas9 scaffold-EcoR1 |
| ZRp66_M15 | GFP single mismatch sgRNA_15 expression vector | pFUGW-UbC-RFP-CMV-EGFP-BamHI-U6-EGFP sgb M15-Sacas9 scaffold-EcoR1 |
| ZRp66_M16 | GFP single mismatch sgRNA_16 expression vector | pFUGW-UbC-RFP-CMV-EGFP-BamHI-U6-EGFP sgb M16-Sacas9 scaffold-EcoR1 |

(continued)

| Plasmid name | Description | Reference |
|---|---|---|
| ZRp66_M17 | GFP single mismatch sgRNA_17 expression vector | pFUGW-UbC-RFP-CMV-EGFP-BamHI-U6-EGFP sgb M17-Sacas9 scaffold-EcoR1 |
| ZRp66_M18 | GFP single mismatch sgRNA_18 expression vector | pFUGW-UbC-RFP-CMV-EGFP-BamHI-U6-EGFP sgb M18-Sacas9 scaffold-EcoR1 |
| ZRp66_M19 | GFP single mismatch sgRNA_19 expression vector | pFUGW-UbC-RFP-CMV-EGFP-BamHI-U6-EGFP sgb M19-Sacas9 scaffold-EcoR1 |
| ZRp66_M20 | GFP single mismatch sgRNA_20 expression vector | pFUGW-UbC-RFP-CMV-EGFP-BamHI-U6-EGFP sgb M20-Sacas9 scaffold-EcoR1 |
| ZRp67_M17/18 | GFP single mismatch sgRNA_17/18 expression vector | pFUGW-UbC-RFP-CMV-EGFP-BamHI-U6-EGFP sgb M17/18-Sacas9 scaffold-EcoR1 |
| ZRp67_M18/19 | GFP single mismatch sgRNA_18/19 expression vector | pFUGW-UbC-RFP-CMV-EGFP-BamHI-U6-EGFP sgb M18/19-Sacas9 scaffold-EcoR1 |
| ZRp67_M19/20 | GFP single mismatch sgRNA_19/20 expression vector | pFUGW-UbC-RFP-CMV-EGFP-BamHI-U6-EGFP sgb M19/20-Sacas9 scaffold-EcoR1 |
| ZRp33B | EMX1_1 sgRNA expression vector | pFUGW-CMV-GFP-hU6-EMX1_1 gRNA-SaCas9 scaffold-BamHI-EcoRI |
| ZRp34B | EMX1_4 sgRNA expression vector | pFUGW-CMV-GFP-hU6-EMX1_4 gRNA-SaCas9 scaffold-BamHI-EcoRI |
| ZRp35B | EMX1_6 sgRNA expression vector | pFUGW-CMV-GFP-hU6-EMX1_6 gRNA-SaCas9 scaffold-BamHI-EcoRI |
| ZRp36B | EMX1_10 sgRNA expression vector | pFUGW-CMV-GFP-hU6-EMX1_10 gRNA-SaCas9 scaffold-BamHI-EcoRI |
| ZRp37B | CMV-GFP exprgfpsion vector | pFUGW-CMV-GFP-hU6-EMX1_sg2 gRNA-SaCas9 scaffold-BamHI-EcoRI |
| ZRp38B | CMV-GFP exprgfpsion vector | pFUGW-CMV-GFP-hU6-EMX1_sg3 gRNA-SaCas9 scaffold-BamHI-EcoRI |
| ZRp39B | CMV-GFP exprgfpsion vector | pFUGW-CMV-GFP-hU6-EMX1_sg7 gRNA-SaCas9 scaffold-BamHI-EcoRI |
| ZRp40B | VEGFA_3 sgRNA expression vector | pFUGW-CMV-GFP-hU6-VEGFA_3 gRNA-SaCas9 scaffold-BamHI-EcoRI |
| ZRp41B | VEGFA_8 sgRNA expression vector | pFUGW-CMV-GFP-hU6-VEGFA_8 gRNA-SaCas9 scaffold-BamHI-EcoRI |
| ZRp85_M1 | VEGFA_8 single mismatch sgRNA M1 expression vector | pFUGW-CMV-GFP-hU6-VEGFA_8 gRNA M1-SaCas9 scaffold-BamHI-EcoRI |
| ZRp85_M2 | VEGFA_8 single mismatch sgRNA M2 expression vector | pFUGW-CMV-GFP-hU6-VEGFA_8 gRNA M2-SaCas9 scaffold-BamHI-EcoRI |
| ZRp85_M3 | VEGFA_8 single mismatch sgRNA M3 expression vector | pFUGW-CMV-GFP-hU6-VEGFA_8 gRNA M3-SaCas9 scaffold-BamHI-EcoRI |
| ZRp85_M4 | VEGFA_8 single mismatch sgRNA M4 expression vector | pFUGW-CMV-GFP-hU6-VEGFA_8 gRNA M4-SaCas9 scaffold-BamHI-EcoRI |
| ZRp85_M5 | VEGFA_8 single mismatch sgRNA M5 expression vector | pFUGW-CMV-GFP-hU6-VEGFA_8 gRNA M5-SaCas9 scaffold-BamHI-EcoRI |
| ZRp85_M6 | VEGFA_8 single mismatch sgRNA M6 expression vector | pFUGW-CMV-GFP-hU6-VEGFA_8 gRNA M6-SaCas9 scaffold-BamHI-EcoRI |

(continued)

| Plasmid name | Description | Reference |
|---|---|---|
| ZRp85_M7 | VEGFA_8 single mismatch sgRNA M7 expression vector | pFUGW-CMV-GFP-hU6-VEGFA_8 gRNA M7-SaCas9 scaffold-BamHI-EcoRI |
| ZRp85_M8 | VEGFA_8 single mismatch sgRNA M8 expression vector | pFUGW-CMV-GFP-hU6-VEGFA_8 gRNA M8-SaCas9 scaffold-BamHI-EcoRI |
| ZRp85_M9 | VEGFA_8 single mismatch sgRNA M9 expression vector | pFUGW-CMV-GFP-hU6-VEGFA_8 gRNA M9-SaCas9 scaffold-BamHI-EcoRI |
| ZRp85_M10 | VEGFA_8 single mismatch sgRNA M10 expression vector | pFUGW-CMV-GFP-hU6-VEGFA_8 gRNA M10-SaCas9 scaffold-BamHI-EcoRI |
| ZRp85_M11 | VEGFA_8 single mismatch sgRNA M11 expression vector | pFUGW-CMV-GFP-hU6-VEGFA_8 gRNA M11-SaCas9 scaffold-BamHI-EcoRI |
| ZRp85_M12 | VEGFA_8 single mismatch sgRNA M12 expression vector | pFUGW-CMV-GFP-hU6-VEGFA_8 gRNA M12-SaCas9 scaffold-BamHI-EcoRI |
| ZRp85_M13 | VEGFA_8 single mismatch sgRNA M13 expression vector | pFUGW-CMV-GFP-hU6-VEGFA_8 gRNA M13-SaCas9 scaffold-BamHI-EcoRI |
| ZRp85_M14 | VEGFA_8 single mismatch sgRNA M14 expression vector | pFUGW-CMV-GFP-hU6-VEGFA_8 gRNA M14-SaCas9 scaffold-BamHI-EcoRI |
| ZRp85_M15 | VEGFA_8 single mismatch sgRNA M15 expression vector | pFUGW-CMV-GFP-hU6-VEGFA_8 gRNA M15-SaCas9 scaffold-BamHI-EcoRI |
| ZRp85_M16 | VEGFA_8 single mismatch sgRNA M16 expression vector | pFUGW-CMV-GFP-hU6-VEGFA_8 gRNA M16-SaCas9 scaffold-BamHI-EcoRI |
| ZRp85_M17 | VEGFA_8 single mismatch sgRNA M17 expression vector | pFUGW-CMV-GFP-hU6-VEGFA_8 gRNA M17-SaCas9 scaffold-BamHI-EcoRI |
| ZRp85_M18 | VEGFA_8 single mismatch sgRNA M18 expression vector | pFUGW-CMV-GFP-hU6-VEGFA_8 gRNA M18-SaCas9 scaffold-BamHI-EcoRI |
| ZRp85_M19 | VEGFA_8 single mismatch sgRNA M19 expression vector | pFUGW-CMV-GFP-hU6-VEGFA_8 gRNA M19-SaCas9 scaffold-BamHI-EcoRI |
| ZRp85_M20 | VEGFA_8 single mismatch sgRNA M20 expression vector | pFUGW-CMV-GFP-hU6-VEGFA_8 gRNA M20-SaCas9 scaffold-BamHI-EcoRI |
| ZRp85_M21 | VEGFA_8 single mismatch sgRNA M21 expression vector | pFUGW-CMV-GFP-hU6-VEGFA_8 gRNA M21-SaCas9 scaffold-BamHI-EcoRI |
| ZRp42B | FANCF_13 sgRNA expression vector | pFUGW-CMV-GFP-hU6-FANCF_13 gRNA-SaCas9 scaffold-BamHI-EcoRI |
| ZRp86_M1 | FANCF_13 single mismatch sgRNA M1 expression vector | pFUGW-CMV-GFP-hU6-FANCF_13 gRNA M1-SaCas9 scaffold-BamHI-EcoRI |
| ZRp86_M2 | FANCF_13 single mismatch sgRNA M2 expression vector | pFUGW-CMV-GFP-hU6-FANCF_13 gRNA M2-SaCas9 scaffold-BamHI-EcoRI |
| ZRp86_M3 | FANCF_13 single mismatch sgRNA M3 expression vector | pFUGW-CMV-GFP-hU6-FANCF_13 gRNA M3-SaCas9 scaffold-BamHI-EcoRI |
| ZRp86_M4 | FANCF_13 single mismatch sgRNA M4 expression vector | pFUGW-CMV-GFP-hU6-FANCF_13 gRNA M4-SaCas9 scaffold-BamHI-EcoRI |
| ZRp86_M5 | FANCF_13 single mismatch sgRNA M5 expression vector | pFUGW-CMV-GFP-hU6-FANCF_13 gRNA M5-SaCas9 scaffold-BamHI-EcoRI |
| ZRp86_M6 | FANCF_13 single mismatch sgRNA M6 expression vector | pFUGW-CMV-GFP-hU6-FANCF_13 gRNA M6-SaCas9 scaffold-BamHI-EcoRI |

(continued)

| Plasmid name | Description | Reference |
|---|---|---|
| ZRp86_M7 | FANCF_13 single mismatch sgRNA M7 expression vector | pFUGW-CMV-GFP-hU6-FANCF_13 gRNA M7-SaCas9 scaffold-BamHI-EcoRI |
| ZRp86_M8 | FANCF_13 single mismatch sgRNA M8 expression vector | pFUGW-CMV-GFP-hU6-FANCF_13 gRNA M8-SaCas9 scaffold-BamHI-EcoRI |
| ZRp86_M9 | FANCF_13 single mismatch sgRNA M9 expression vector | pFUGW-CMV-GFP-hU6-FANCF_13 gRNA M9-SaCas9 scaffold-BamHI-EcoRI |
| ZRp86_M10 | FANCF_13 single mismatch sgRNA M10 expression vector | pFUGW-CMV-GFP-hU6-FANCF_13 gRNA M10-SaCas9 scaffold-BamHI-EcoRI |
| ZRp86_M11 | FANCF_13 single mismatch sgRNA M11 expression vector | pFUGW-CMV-GFP-hU6-FANCF_13 gRNA M11-SaCas9 scaffold-BamHI-EcoRI |
| ZRp86_M12 | FANCF_13 single mismatch sgRNA M12 expression vector | pFUGW-CMV-GFP-hU6-FANCF_13 gRNA M12-SaCas9 scaffold-BamHI-EcoRI |
| ZRp86_M13 | FANCF_13 single mismatch sgRNA M13 expression vector | pFUGW-CMV-GFP-hU6-FANCF_13 gRNA M13-SaCas9 scaffold-BamHI-EcoRI |
| ZRp86_M14 | FANCF_13 single mismatch sgRNA M14 expression vector | pFUGW-CMV-GFP-hU6-FANCF_13 gRNA M14-SaCas9 scaffold-BamHI-EcoRI |
| ZRp86_M15 | FANCF_13 single mismatch sgRNA M15 expression vector | pFUGW-CMV-GFP-hU6-FANCF_13 gRNA M15-SaCas9 scaffold-BamHI-EcoRI |
| ZRp86_M16 | FANCF_13 single mismatch sgRNA M16 expression vector | pFUGW-CMV-GFP-hU6-FANCF_13 gRNA M16-SaCas9 scaffold-BamHI-EcoRI |
| ZRp86_M17 | FANCF_13 single mismatch sgRNA M17 expression vector | pFUGW-CMV-GFP-hU6-FANCF_13 gRNA M17-SaCas9 scaffold-BamHI-EcoRI |
| ZRp86_M18 | FANCF_13 single mismatch sgRNA M18 expression vector | pFUGW-CMV-GFP-hU6-FANCF_13 gRNA M18-SaCas9 scaffold-BamHI-EcoRI |
| ZRp86_M19 | FANCF_13 single mismatch sgRNA M19 expression vector | pFUGW-CMV-GFP-hU6-FANCF_13 gRNA M19-SaCas9 scaffold-BamHI-EcoRI |
| ZRp86_M20 | FANCF_13 single mismatch sgRNA M20 expression vector | pFUGW-CMV-GFP-hU6-FANCF_13 gRNA M20-SaCas9 scaffold-BamHI-EcoRI |
| ZRp86_M21 | FANCF_13 single mismatch sgRNA M21 expression vector | pFUGW-CMV-GFP-hU6-FANCF_13 gRNA M21-SaCas9 scaffold-BamHI-EcoRI |
| ZRp43B | AAVS1_4 sgRNA expression vector | pFUGW-CMV-GFP-hU6-AAVS 1_4 gRNA-SaCas9 scaffold-BamHI-EcoRI |
| ZRp44B | CCR5_2 sgRNA expression vector | pFUGW-CMV-GFP-hU6-CCRS _ 2 gRNA-SaCas9 scaffold-BamHI-EcoRI |
| ZRp71 | KKH-eSaCas9 expression vector | pFUGW-KKH-eSaCas9-NheI-2A-BFP-SA 157-EcoRI-B C4-B C3-B C2-B Cl |
| ZRp72 | DNMT1_sg1 sgRNA expression vector | pFUGW-CMV-GFP-hU6-DNMT1_sg1-SaCas9 scaffold-BamHI-EcoRI |
| ZRp73 | DNMT1_sg2 sgRNA expression vector | pFUGW-CMV-GFP-hU6-DNMT1_ sg2-SaCas9 scaffold-BamHI-EcoRI |
| ZRp74 | FGFR3_sg1 sgRNA expression vector | pFUGW-CMV-GFP-hU6-FGFR3_sg1-SaCas9 scaffold-BamHI-EcoRI |
| CKp21 | DNMT1i_sg1 sgRNA expression vector | pFUGW-CMV-GFP-hU6-DNMT1i_sg1-SaCas9 scaffold-BamHI-EcoRI |

(continued)

| Plasmid name | Description | Reference |
|---|---|---|
| CKp21_M12 | DNMT1_isingle mismatch sg1_M12 sgRNA expression vector | pFUGW-CMV-GFP-hU6-DNMT1i_sg1 M12-SaCas9 scaffold-BamHI-EcoRI |
| CKp21_M18 | DNMT1_isingle mismatch sg1_M18 sgRNA expression vector | pFUGW-CMV-GFP-hU6-DNMT1i_sg1 M18-SaCas9 scaffold-BamHI-EcoRI |
| CKp23 | EGFRi_sg1 sgRNA expression vector | pFUGW-CMV-GFP-hU6-EGFRi_sg1-SaCas9 scaffold-BamHI-EcoRI |
| CKp23_M12 | EGFRi single mismatch sg1_M12 sgRNA expression vector | pFUGW-CMV-GFP-hU6-EGFRi_sg1 M12-SaCas9 scaffold-BamHI-EcoRI |

**Table 2:** List of sgRNA Protospacer Sequences Used in the Current Study

| sgRNA ID | 3' end of U6 | 5' G (*) | sgRNA protospacer sequence (*) | PAM |
|---|---|---|---|---|
| GFP_On 1 | CACC | G | CACCTACGGCAAGCTGACCC (SEQ ID NO:129) | TGAAGT |
| GFP_On 2 | CACC | G | GGCGAGGAGCTGTTCACCGG (SEQ ID NO:3) | GGTGGT |
| GFP_Off 1 | CACC | G | CACCTACGGCAA**T**CTGACCC (SEQ ID NO:130) | TGAAGT |
| GFP_Off 2 | CACC | G | CA**A**CTACGGCAAGCTGACCC (SEQ ID NO:4) | TGAAGT |
| GFP_Off 3 | CACC | G | CACCTAC**A**GCAAGCTGACCC (SEQ ID NO:5) | TGAAGT |
| GFP_On1_M1 | CACC | G | CACCTACGGCAAGCTGACC**T** (SEQ ID NO:7) | TGAAGT |
| GFP_On1_M2 | CACC | G | CACCTACGGCAAGCTGAC**T**C (SEQ ID NO:8) | TGAAGT |
| GFP_On1_M3 | CACC | G | CACCTACGGCAAGCTGA**T**CC (SEQ ID NO:9) | TGAAGT |
| GFP_On1_M4 | CACC | G | CACCTACGGCAAGCTG**G**CCC (SEQ ID NO: 10) | TGAAGT |
| GFP_On1_M5 | CACC | G | CACCTACGGCAAGCT**A**ACCC (SEQ ID NO:11) | TGAAGT |
| GFP_On1_M6 | CACC | G | CACCTACGGCAAGC**C**GACCC (SEQ ID NO:12) | TGAAGT |
| GFP_On1_M7 | CACC | G | CACCTACGGCAAG**T**TGACCC (SEQ ID NO:13) | TGAAGT |
| GFP_On1_M8 | CACC | G | CACCTACGGCAA**A**CTGACCC (SEQ ID NO:14) | TGAAGT |
| GFP_On1_M9 | CACC | G | CACCTACGGCA**G**GCTGACCC (SEQ ID NO:15) | TGAAGT |
| GFP_On1_M10 | CACC | G | CACCTACGGC**G**AGCTGACCC (SEQ ID NO:16) | TGAAGT |
| GFP_On1_M11 | CACC | G | CACCTACGG**T**AAGCTGACCC (SEQ ID NO:17) | TGAAGT |
| GFP_On1_M12 | CACC | G | CACCTACGACAAGCTGACCC (SEQ ID NO:18) | TGAAGT |
| GFP_On1_M13 | CACC | G | CACCTAC**A**GCAAGCTGACCC (SEQ ID NO:19) | TGAAGT |
| GFP_On1_M14 | CACC | G | CACCTA**T**GGCAAGCTGACCC (SEQ ID NO:20) | TGAAGT |
| GFP_On1_M15 | CACC | G | CACCT**G**CGGCAAGCTGACCC (SEQ ID NO:21) | TGAAGT |
| GFP_On1_M16 | CACC | G | CACC**C**ACGGCAAGCTGACCC (SEQ ID NO:22) | TGAAGT |
| GFP_On1_M17 | CACC | G | CAC**T**TACGGCAAGCTGACCC (SEQ ID NO:23) | TGAAGT |
| GFP_On1_M18 | CACC | G | CA**T**CTACGGCAAGCTGACCC (SEQ ID NO:24) | TGAAGT |
| GFP_On1_M19 | CACC | G | C**G**CCTACGGCAAGCTGACCC (SEQ ID NO:25) | TGAAGT |
| GFP_On1_M20 | CACC | G | **T**ACCTACGGCAAGCTGACCC (SEQ ID NO:26) | TGAAGT |
| GFP_On1_M17/18 | CACC | G | CA**TT**TACGGCAAGCTGACCC (SEQ ID NO:27) | TGAAGT |
| GFP_On1_M18/19 | CACC | G | C**GT**CTACGGCAAGCTGACCC (SEQ ID NO:28) | TGAAGT |
| GFP_On1_M19/20 | CACC | G | **TG**CCTACGGCAAGCTGACCC (SEQ ID NO:29) | TGAAGT |

(continued)

| sgRNA ID | 3' end of U6 | 5' G (*) | sgRNA protospacer sequence (*) | PAM |
|---|---|---|---|---|
| EMX1_1 | CACC | G | GTGTGGTTCCAGAACCGGAGGA (SEQ ID NO:82) | CA**A**AGT |
| EMX1_4 | CACC | G | GCTCAGCCTGAGTGTTGAGGC (SEQ ID NO:83) | CC**C**AGT |
| EMX1_6 | CACC | G | GCAACCACAAACCCACGAGGG (SEQ ID NO:101) | CAGAGT |
| EMX1_10 | CACC | G | GGCTCTCCGAGGAGAAGGCCA (SEQ ID NO:102) | AG**T**GGT |
| EMX1_sg2 | CACC | G | TGGCCAGGCTTTGGGGAGGCC (SEQ ID NO:30) | TGGAGT |
| EMX1_sg7 | CACC | G | GGCCAGGCTTTGGGGAGGCC (SEQ ID NO:32) | TGGAGT |
| VEGFA_3 | CACC | G | GAGAGGGACACACAGATCTAT (SEQ ID NO:33) | TGGAAT |
| AAVS1_4 | CACC | G | GACTAGGAAGGAGGAGGCCT (SEQ ID NO:31) | AAGGAT |
| CCR5_2 | CACC | G | GTTGCCCTAAGGATTAAATGA (SEQ ID NO:34) | ATGAAT |
| DNMT1_sg1 | CACC | G | AAACGACCCCCAAAGAACCGT (SEQ ID NO:103) | AAGAAT |
| DNMT1_sg2 | CACC | G | AGTCTGAAAGAGCCAAATCGG (SEQ ID NO:104) | ATGAGT |
| FGFR3_sg1 | CACC | G | GTATGCAGGCATCCTCAGCTA (SEQ ID NO:105) | CGGGGT |
| VEGFA_8 | CACC | G | GGGTGAGTGAGTGTGTGCGTG (SEQ ID NO:35) | TGGGGT |
| VEGFA_8_M1 | CACC | G | GGGTGAGTGAGTGTGTGCGT**A** (SEQ ID NO:36) | TGGGGT |
| VEGFA_8_M2 | CACC | G | GGGTGAGTGAGTGTGTGCG**C**G (SEQ ID NO:37) | TGGGGT |
| VEGFA_8_M3 | CACC | G | GGGTGAGTGAGTGTGTGC**A**TG (SEQ ID NO:38) | TGGGGT |
| VEGFA_8_M4 | CACC | G | GGGTGAGTGAGTGTGTG**T**GTG (SEQ ID NO:39) | TGGGGT |
| VEGFA_8_M5 | CACC | G | GGGTGAGTGAGTGTGT**A**CGTG (SEQ ID NO:40) | TGGGGT |
| VEGFA_8_M6 | CACC | G | GGGTGAGTGAGTGTG**C**GCGTG (SEQ ID NO:41) | TGGGGT |
| VEGFA_8_M7 | CACC | G | GGGTGAGTGAGTGT**A**TGCGTG (SEQ ID NO:42) | TGGGGT |
| VEGFA_8_M8 | CACC | G | GGGTGAGTGAGTG**C**GTGCGTG (SEQ ID NO:43) | TGGGGT |
| VEGFA_8_M9 | CACC | G | GGGTGAGTGAGT**A**TGTGCGTG (SEQ ID NO:44) | TGGGGT |
| VEGFA_8_M10 | CACC | G | GGGTGAGTGAG**C**GTGTGCGTG (SEQ ID NO:45) | TGGGGT |
| VEGFA_8_M11 | CACC | G | GGGTGAGTGA**A**TGTGTGCGTG (SEQ ID NO:46) | TGGGGT |
| VEGFA_8_M12 | CACC | G | GGGTGAGTG**G**GTGTGTGCGTG (SEQ ID NO:47) | TGGGGT |
| VEGFA_8_M13 | CACC | G | GGGTGAGT**A**AGTGTGTGCGTG (SEQ ID NO:48) | TGGGGT |
| VEGFA_8_M14 | CACC | G | GGGTGAG**C**GAGTGTGTGCGTG (SEQ ID NO:49) | TGGGGT |
| VEGFA_8_M15 | CACC | G | GGGTGA**A**TGAGTGTGTGCGTG (SEQ ID NO:50) | TGGGGT |
| VEGFA_8_M16 | CACC | G | GGGTG**G**GTGAGTGTGTGCGTG (SEQ ID NO:51) | TGGGGT |
| VEGFA_8_M17 | CACC | G | GGGT**A**AGTGAGTGTGTGCGTG (SEQ ID NO:52) | TGGGGT |
| VEGFA_8_M18 | CACC | G | GGG**C**GAGTGAGTGTGTGCGTG (SEQ ID NO:53) | TGGGGT |
| VEGFA_8_M19 | CACC | G | GG**A**TGAGTGAGTGTGTGCGTG (SEQ ID NO:54) | TGGGGT |
| VEGFA_8_M20 | CACC | G | G**A**GTGAGTGAGTGTGTGCGTG (SEQ ID NO:55) | TGGGGT |
| VEGFA_8_M21 | CACC | G | **A**GGTGAGTGAGTGTGTGCGTG (SEQ ID NO:56) | TGGGGT |
| FANCF_13 | CACC | G | GCAAGGCCCGGCGCACGGTGG (SEQ ID NO:57) | CGGGGT |
| FANCF_13_M1 | CACC | G | GCAAGGCCCGGCGCACGGTG**A** (SEQ ID NO:58) | CGGGGT |
| FANCF_13_M2 | CACC | G | GCAAGGCCCGGCGCACGGT**A**G (SEQ ID NO:59) | CGGGGT |
| FANCF_13_M3 | CACC | G | GCAAGGCCCGGCGCACGG**C**GG (SEQ ID NO:60) | CGGGGT |

(continued)

| sgRNA ID | 3' end of U6 | 5' G (*) | sgRNA protospacer sequence (*) | PAM |
|---|---|---|---|---|
| FANCF_13_M4 | CACC | G | GCAAGGCCCGGCGCACG**A**TGG (SEQ ID NO:61) | CGGGGT |
| FANCF_13_M5 | CACC | G | GCAAGGCCCGGCGCAC**A**GTGG (SEQ ID NO:62) | CGGGGT |
| FANCF_13_M6 | CACC | G | GCAAGGCCCGGCGCA**T**GGTGG (SEQ ID NO:63) | CGGGGT |
| FANCF_13_M7 | CACC | G | GCAAGGCCCGGCGC**G**CGGTGG (SEQ ID NO:64) | CGGGGT |
| FANCF_13_M8 | CACC | G | GCAAGGCCCGGCG**T**ACGGTGG (SEQ ID NO:65) | CGGGGT |
| FANCF_13_M9 | CACC | G | GCAAGGCCCGGC**A**CACGGTGG (SEQ ID NO:66) | CGGGGT |
| FANCF_13_M10 | CACC | G | GCAAGGCCCGG**T**GCACGGTGG (SEQ ID NO:67) | CGGGGT |
| FANCF_13_M11 | CACC | G | GCAAGGCCCG**A**CGCACGGTGG (SEQ ID NO:68) | CGGGGT |
| FANCF_13_M12 | CACC | G | GCAAGGCCC**A**GCGCACGGTGG (SEQ ID NO:69) | CGGGGT |
| FANCF_13_M13 | CACC | G | GCAAGGCC**T**GGCGCACGGTGG (SEQ ID NO:70) | CGGGGT |
| FANCF_13_M14 | CACC | G | GCAAGGC**T**CGGCGCACGGTGG (SEQ ID NO:71) | CGGGGT |
| FANCF_13_M15 | CACC | G | GCAAGG**T**CCGGCGCACGGTGG (SEQ ID NO:72) | CGGGGT |
| FANCF_13_M16 | CACC | G | GCAAG**A**CCCGGCGCACGGTGG (SEQ ID NO:73) | CGGGGT |
| FANCF_13_M17 | CACC | G | GCAA**A**GCCCGGCGCACGGTGG (SEQ ID NO:74) | CGGGGT |
| FANCF_13_M18 | CACC | G | GCA**G**GGCCCGGCGCACGGTGG (SEQ ID NO:75) | CGGGGT |
| FANCF_13_M19 | CACC | G | GC**G**AGGCCCGGCGCACGGTGG (SEQ ID NO:76) | CGGGGT |
| FANCF_13_M20 | CACC | G | G**T**AAGGCCCGGCGCACGGTGG (SEQ ID NO:77) | CGGGGT |
| FANCF_13_M21 | CACC | G | **A**CAAGGCCCGGCGCACGGTGG (SEQ ID NO:78) | CGGGGT |
| DNMT1i_sg1 | CACC | G | GCCTGCGGACATCGTCGGGCA (SEQ ID NO:106) | GCGAGT |
| DNMT1i_sg1 M12 | CACC | G | GCCTGCGGA**T**ATCGTCGGGCA (SEQ ID NO:107) | GCGAGT |
| DNMT1i_sg1 M18 | CACC | G | GCC**C**GCGGACATCGTCGGGCA (SEQ ID NO:108) | GCGAGT |
| EGFRi_sg1 | CACC | G | CGTCGGGCGCTCACACCGTGC (SEQ ID NO:109) | GGGGGT |
| EGFR1_sg1 M12 | CACC | G | CGTCGGGCG**T**TCACACCGTGC (SEQ ID NO:110) | GGGGGT |

A library of KKH-SaCas9 variants with combinations of substitution mutations was constructed. Based on predictions from protein structure models, 12 amino acid residues that were predicted to make contacts with or be in close proximity to the DNA and sgRNA backbones were focused on, and modified to harbor specified substitutions (Table 3). Some of those mutations are present in SaCas9-HF (Tan, Y., et al. (2019) Proc Natl Acad Sci USA, Vol. 116, pages 20969-20976) and eSaCas9 (Slaymaker, et al. (2016) Science, Vol. 351, 84-88 doi: 10.1126/science.aad5227). It was hypothesized that specific combinations of these mutations in KKH-SaCas9 could reduce its undesirable off-target activity, while maximizing its on-target editing efficiency. To assemble the KKH-SaCas9 variants with combinations of substitution mutations, the KKH-SaCas9 sequence is modularized into four parts (P1 to P4). The modularized parts with specific mutations were generated by PCR or synthesis, and each of them was flanked by a pair of type IIS restriction enzyme cut sites on their two ends. The variants within each part were pooled together. Type IIS restriction enzymes were used to iteratively digest and ligate to the subsequent pool of DNA parts in a lentiviral vector to generate higher-order combination mutants. Since digestion with type IIS restriction enzymes generates compatible overhangs that are originated from the protein-coding sequence, no fusion scar is formed in the ligation reactions. A set of 27 variants (i.e., v3.1-20, v3.22-25, and v3.27-29) were randomly sampled from the combination mutant library of KKH-SaCas9 and their editing activities were individually characterized using multiple sgRNA reporter lines.

Table 3:

| Amino acid residue(s) | Domain location | Being mutated in other SaCas9 variants? | Selected substitutions | Reason for selection | Reference |
|---|---|---|---|---|---|
| T238 | REC | No | Alanine | T238 is predicted to interact with the sgRNA backbone, and the interaction could be abolished by an alanine substitution. | Nishimasu et al., Cell, 2015 (PMID: 26317473) |
| Y239 | REC | No | Histidine | Y239H is predicted to weaken the enzyme's interaction of the sgRNA backbone, while maintaining the stacking interaction with its F418. | Nishimasu et al., Cell, 2015 (PMID: 26317473) |
| R245 | REC | Yes; in SaCas9-HF | Alanine | R245A was reported in SaCas9-HF that shows reduced off-target editing, and an alanine substitution may abolish the enzyme's contacts with the DNA backbone. | Tan et al., PNAS, 2019 (PMID: 31570596) |
| T392 | REC | No | Alanine | T392 is predicted to make contact with the phosphate backbone of the target DNA strand, and the interaction could be abolished by an alanine substitution. | Nishimasu et al., Cell, 2015 (PMID: 26317473) |
| N394 | REC | No | Alanine/ Threonine | N394 is predicted to interact with the sgRNA backbone, and N394A and N394T may abolish and weaken this interaction, respectively. | Nishimasu et al., Cell, 2015 (PMID: 26317473) |
| Q414 | REC | No | Arginine | Q414 is predicted to interact with the sgRNA backbone and the 3rd base of the sgRNA distal to the PAM. Q414R could remove the enzyme's non-base specific interaction with the 3rd base of the sgRNA, while maintaining the enzyme's interaction the sgRNA backbone. Q414R may thus encourage specificity to be determined more stringently by Watson-Crick base pairing between the sgRNA and the target DNA. | Nishimasu et al., Cell, 2015 (PMID: 26317473) |
| N419 | REC | Yes; in SaCas9-HF | Aspartic acid/ Serine/ Glycine | N419 is predicted to interact with the backbone of the target DNA strand. Switching from N to D does not change the distance from the enzyme's side chain to the DNA backbone, but the negative-charged side chain of D may repel the backbone more than N. Switching from N to S and G may lessen the enzyme's interaction with the backbone. | Tan et al., PNAS, 2019 (PMID: 31570596) |

(continued)

| Amino acid residue(s) | Domain location | Being mutated in other SaCas9 variants? | Selected substitutions | Reason for selection | Reference |
|---|---|---|---|---|---|
| R499/Q500 | L1 linker | Yes; in eSaCas9 | Alanine | R499A/Q500A was reported in eSaCas9 that shows reduced off-target editing. R499 potentially interacts with the DNA backbone, and R499A may abolish the interaction. G500A may help maintain the shape of the helix for R499A. | Slay maker et al., Science, 2016 (PMID: 26628643) |
| Y651 | RuvC | No | Histidine | Y651 is predicted to interact with the DNA backbone, and is positioned near R654. R654A potentially loses interactions with both DNA and sgRNA backbones (see below). Introducing Y651H may increase the interaction with the DNA backbone, which could compensate for the loss of the enzyme's interaction with DNA backbone in variants harboring R654A. This may allow evaluation of how balancing interactions with the sgRNA and DNA backbones may fine-tune the enzyme's specificity and activity. | Nishimasu et al., Cell, 2015 (PMID: 26317473) |
| R654/G655 | RuvC | Yes; in SaCas9-HF and eSaCas9 | Alanine | R654A and R654A/G655A were reported in SaCas9-HF and eSaCas9, respectively, that show reduced off-target editing. R654 potentially interacts with the sgRNA backbone and the DNA backbone. R654A is predicted to abolish both interactions. G655 is next to R654 and in a helix region. It does not have predicted interactions with sgRNA and DNA, but G655A may help maintain the shape of the helix. | Tan et al., PNAS, 2019 (PMID: 31570596); Slay maker et al., Science, 2016 (PMID: 26628643) |

***Human cell culture***

HEK293T and SK-N-MC cells were obtained from American Type Culture Collection (ATCC). MHCC97L cells were obtained from The University of Hong Kong). OVCAR8-ADR cells were obtained from the Japanese National Cancer Center Research Institute, and the identity of the OVCAR8-ADR cells was confirmed by a cell line authentication test (Genetica DNA Laboratories). OVCAR8-ADR cells were transduced with lentiviruses encoding RFP and GFP genes expressed from UBC and CMV promoters, respectively, and a tandem U6 promoter-driven expression cassette of sgRNA targeting GFP site. ON1 and ON2 lines harbor sgRNA's spacer that matches completely with the target sites on GFP, while OFF1, OFF2, and OFF3 lines harbor single-base mismatches to the targets site. To generate cell lines stably expressing SaCas9 protein, cells were infected with a lentiviral expression vector encoding KKH-SaCas9, KKH-SaCas9-SAV1, and SAV2, followed by P2A-BFP. These cells were sorted using a Becton Dickinson BD Influx cell sorter. HEK293T, SK-N-MC, and MHCC97L cells were cultured in Dulbecco's Modified Eagle Medium (DMEM). supplemented with 10% heat-inactivated FBS and 1× antibiotic-antimycotic (ThermoFisher Scientific) at 37°C with 5% $CO_2$. OVCAR8-ADR cells were cultured in RPMI supplemented with 10% heat-inactivated FBS and 1× antibiotic-antimycotic (ThermoFisher Scientific) at 37°C with 5% $CO_2$. Cells were regularly tested for mycoplasma contamination and were confirmed to be negative. Lentivirus production and transduction were performed as previously described (Choi, G.C.G., et al. (2019) Nat Methods, Vol. 16, pages 722-730).

### *Fluorescent protein disruption assay*

Fluorescent protein disruption assays were performed to evaluate DNA cleavage and indel-mediated disruption at the target site of the fluorescent protein (i.e., GFP) brought by SaCas9 and gRNA expressions, which results in loss of cell fluorescence. Cells harboring an integrated green fluorescent protein (GFP) and red fluorescent protein (RFP) reporter gene and together with SaCas9 and sgRNA were washed and resuspended with 1× PBS supplemented with 2% heat inactivated FBS, and assayed with a Becton Dickinson LSR Fortessa Analyzer or ACEA NovoCyte Quanteon. Cells were gated on forward and side scatter. At least $1 \times 10^4$ cells were recorded per sample in each data set.

### *Immunoblot analysis*

Immunoblotting experiments were carried out as previously described (Choi, G.C.G., et al. (2019) Nat Methods, Vol. 16, pages 722-730). Primary antibodies used were anti-SaCas9 (1:1,000, Cell Signaling Cat. #85687) and anti-GAPDH (1:5,000, Cell Signaling Cat. #2118). Secondary antibody used was HRP-linked anti-mouse IgG (1:10,000, Cell Signaling Cat. #7076) and HRP-linked anti-rabbit IgG (1:20,000, Cell Signaling Cat. #7074).

### *T7 Endonuclease I assay*

T7 endonuclease I assay was carried out as previously described (Choi, G.C.G., et al. (2019) Nat Methods, Vol. 16, pages 722-730). Amplicons harboring the targeted loci were generated by PCR. The PCR primer sequences are listed in Table 4. Quantification was based on relative band intensities measured using ImageJ. Editing efficiency was estimated using the formula:

$$100 \times (1 - (1 - (b + c)/(a + b + c))1/2)$$

as previously described (Guschin, D.Y., et al. (2010) Methods Mol Biol, Vol. 649, pages 247-256), wherein "a" is the integrated intensity of the uncleaved PCR product, and "b" and "c" are the integrated intensities of each cleavage product. Normalized editing efficiency brought by the KKH-SaCas9 variants to those by wild-type are calculated for each sgRNA.

$$100 \text{ x} (1 - \left(1 - \frac{(\textit{cleaved band intensity})}{(\text{total band intensity})}\right) \text{^}0.5)$$

Table 4: List of Primers Used in T7E1 Assay

| Target gene | Forward primer (5' to 3') | Reverse primer (5' to 3') |
|---|---|---|
| EMX1_1 | GGAGCAGCTGGTCAGAGGGG (SEQ ID NO: 113) | CCATAGGGAAGGGGGACACTGG (SEQ ID NO:114) |
| EMX1_4 | GGAGCAGCTGGTCAGAGGGG (SEQ ID NO: 113) | CCATAGGGAAGGGGGACACTGG (SEQ ID NO: 114) |
| EMX1_6 | GGAGCAGCTGGTCAGAGGGG (SEQ ID NO: 113) | CCATAGGGAAGGGGGACACTGG (SEQ ID NO: 114) |
| VEGFA_8 | TCCAGATGGCACATTGTCAG (SEQ ID NO: 115) | AGGGAGCAGGAAAGTGAGGT (SEQ ID NO: 116) |
| FANCF_13 | GGGCCGGGAAAGAGTTGCTG (SEQ ID NO: 117) | GCCCTACATCTGCTCTCCCTCC (SEQ ID NO: 118) |
| EMX1_10 | GGAGCAGCTGGTCAGAGGGG (SEQ ID NO: 113) | CCATAGGGAAGGGGGACACTGG (SEQ ID NO: 114) |
| EMX1_sg2 | GGAGCAGCTGGTCAGAGGGG (SEQ ID NO: 113) | CCATAGGGAAGGGGGACACTGG (SEQ ID NO: 114) |
| EMX1_sg7 | GGAGCAGCTGGTCAGAGGGG (SEQ ID NO: 113) | CCATAGGGAAGGGGGACACTGG (SEQ ID NO: 114) |

(continued)

| Target gene | Forward primer (5' to 3') | Reverse primer (5' to 3') |
|---|---|---|
| VEGFA_3 | TCCAGATGGCACATTGTCAG (SEQ ID NO: 115) | AGGGAGCAGGAAAGTGAGGT (SEQ ID NO: 116) |
| AAVS1_4 | GACTCAAACCCAGAAGCCCA (SEQ ID NO: 119) | AGCCACCTCTCCATCCTCTT (SEQ ID NO: 120) |
| CCR5_2 | CCGGCCATTTCACTCTGACT (SEQ ID NO: 121) | TTGCTGCTAGCTTCCCTGTC (SEQ ID NO: 122) |
| DNMT1_sg1 | GAGGTGTCCACTTACACAGGAA (SEQ ID NO:123) | TCAAAATCGCCCCTGTGAGG (SEQ ID NO: 124) |
| DNMT1_sg2 | ACAGACAGCGTTTGGTTGAC (SEQ ID NO: 125) | GAGCCCTAGACAGGGTTTTTAT (SEQ ID NO: 126) |
| FGFR3 | TCACTGGCGTTACTGACTGC (SEQ ID NO: 127) | TGTGTTGGAGCTCATGGACG (SEQ ID NO: 128) |

***GUIDE-seq***

Genome-wide off-targets were accessed using the GUIDE-seq method (20). Experimental procedures for preparing sequencing libraries were carried out as previously described (Choi, G.C.G., et al. (2019) Nat Methods, Vol. 16, pages 722-730). For each GUIDE-seq sample, 1.6 million MHCC97L cells infected with SaCas9 variants and sgRNAs (EMX1-sg2, EMX1-sg7, VEGFA-sg3, AAVS1-sg4, and CCR5-sg2) were electroporated with 1,100 pmol freshly annealed GUIDE-seq end-protected dsODN using 100 μl Neon tips (ThermoFisher Scientific) according to the manufacturer's protocol. The dsODN oligonucleotides used for annealing were 5'-P-G*T*TTAATTGAGTTGTCATATGTTAATAACGGT*A*T-3' (SEQ ID NO:111) and 5'-P-A*T*ACCGTTATTAACATATGACAACTCAATTAA*A*C-3' (SEQ ID NO:112), where P represents 5' phosphorylation and asterisks indicate a phosphorothioate linkage. The electroporation parameters used were 1100 volts, 20 pulse width, and pulse 3. Similarly, 1.5 million OVCAR8-ADR cells infected with SaCas9 variants and the VEGFA-sg8 sgRNA were electroporated with the dsODN. Sequencing libraries were sequenced on Illumina NextSeq System and analysed using the GUIDE-seq software (Tsai, S.Q., et al. (2016) Nat Biotechnol, Vol. 34(5), article 483, DOI: 10.1038/nbt.3534). The updated GUIDE-seq software is based on tsailabSJ/guideseq with the following modifications: 1) changes to make it compatible with python 3.8; 2) configurable UMI length and sample index length; 3) configurable PAM sequence; 4) tox automated testing for python 3.8 to test against alignment data generated by bwa-0.7.17.

***Deep sequencing***

Deep sequencing was carried out as previously described (Wong, A.S., et al. (2016) Proc Natl Acad Sci USA, Vol. 113(9), pages 2544-2549, doi: 10.1073/pnas.1517883113). OVCAR8-ADR cells were infected with SaCas9 variants and the sgRNAs bearing perfectly matched or single-base-pair-mismatched protospacer sequences. Amplicons harboring the targeted loci were generated by PCR. ~1 million reads per sample on average were used to evaluate the editing consequences of >10,000 cells. Indel quantification around the protospacer regions was conducted using CRISPresso2 (Clement, K., et al. (2019) NatBiotechnol, Vol. 37(3), pages 224-226, doi: 10.103 8/s415 87-019-0032-3).

***Reverse transcription quantitative PCR (RT-qPCR)***

OVCAR8-ADR cells were transduced by BFP-tagged KKH-dSaCas9-KRAB variants and then by GFP-marked sgRNA lentiviral vectors three days after. Co-infected cells were sorted by BD FACSAria SORP based on the fluorescent signals 7 days post-sgRNA infection. Total RNA was extracted from the sorted cells and reverse transcription were done via MiniBEST Universal RNA Extraction Kit and PrimeScript™ RT Reagent Kit (TaKaRa), respectively, according to the manufacturer's instructions. qPCR was performed using TB Green Premix Ex Taq (TaKaRa), with the standard PCR protocol. Relative gene expressions were determined relative to GAPDH using standard ΔΔCt method (2-ΔΔCt). qPCR primers used are listed in Table 4.

### *Molecular modelling*

Molecular dynamic simulations were conducted on the variants using DynaMut (Rodrigues, C.H., et al. (2018) Nucleic Acids Res, Vol. 46(W1), W350-W355, doi: 10.1093/nar/gky300; (Biswas, S., et al. (2021) Nat Methods, Vol. 18, pages 389-396; Wu, Z., et al. (2019) Proc NatlAcad Sci USA, Vol. 116, pages 8852-8858)). The variants mutations were singly inputted into the webserver, and the structural outputs were then aligned with the crystal structure of SaCas9 (PDB: 5CZZ) on PyMol. The predicted rotamer of the mutations as indicated by DynaMut was then used to replace the amino acid positions on the SaCas9 crystal structure. The predicted interactions determined by DynaMut and Pymol were then drawn on the crystal structure to provide a putative representation of the SaCas9 variants. Chimera v.1.4 was used for intermolecular contacts estimation, atom-atom distance calculation, and visualization of the protein model.

## Results

### *Identification of KKH-SaCas9 SAV1 and SAV2 variants with enhanced accuracy*

Reduction of KKH-SaCas9's activity by more than 50% on editing three out of five endogenous loci when SaCas9-HF mutations (i.e., R245A/N413A/N419A/R654A) was directly grafted onto KKH-SaCas9 was observed (Tan, et al. Proc Natl Acad Sci U.S.A, 116, 20969-20976, doi: 10.1073/pnas. 1906843116 (2019)). This observation was confirmed by evaluating the editing efficiency of KKH-SaCas9-HF against additional endogenous loci and 88% of reduction (averaged from nine sgRNAs) in its on-target activity when compared to KKH-SaCas9 was detected (**Figure1**; **Figure 16**). Attempts were made to re-engineer KKH-SaCas9 with low off-target and high on-target editing activities. It is possible that specific mutations found on SaCas9-HF may be detrimental to KKH-SaCas9's overall activity. It was hypothesized that alternative residues and substitutions could be exploited to more optimally engineer the enzyme. Protein structure analyses of multiple SaCas9 residues, including those scattered over its REC and RuvC domains, were predicted to interact with the DNA and sgRNA backbones (Table 3). To determine which residues are important in affecting KKH-SaCas9's activity, variants containing combinations of 12 substitution mutations located at the different regions of the protein were assembled and randomly sampled (*see* Material and Methods above). The on- and off-target editing activities of the combination mutations and their variants were measured.

An initial set of 27 variants (i.e., v3.1-20, v3.22-25, and v3.27-29) carrying different sets of the substitution mutations were individually constructed and characterized using two sgRNAs targeting a GFP reporter. Among the variants analyzed, a stark contrast was observed in on-target activities between variants with and without R245A. Variants harboring R245A showed >60% of reduction (and in most cases >80% reduction) in at least one of the two tested sgRNAs at day 15 post-transduction in the green fluorescent protein (GFP) disruption assays. R245A-containing KKH-SaCas9-HF showed a similar decrease in edits when targeting the two same sequences **(Figure 3**). Based on molecular modelling, it was predicted that R245 makes multiple contacts with the DNA backbone **(Figure 6**). Losing too many interactions with the DNA may explain the incompatibility of adding R245A to N413A/N419A/R654A mutations that were found on SaCas9-HF in the KKH-SaCas9 setting. Indeed, grafting only three mutations N413A/N419A/R654A but not including R245A completely restored the on-target activities of KKH-SaCas9 **(Figure 3**). However, a minimal reduction of its off-target activities was also observed **(Figure 3**), indicating the need for alternative mutations to improve KKH-SaCas9's editing accuracy.

Maintaining the core stability of the Cas9 protein and the intricate balance of contacts between sgRNA and DNA is crucial for retaining the on-target activities while reducing off-targeting (Vakulskas, C.A., et al. (2018) Nat Med, Vol. 24(8), pages 1216-1224, doi: 10.1038/s41591-018-0137-0; Choi, G.C.G., et al. (2019) Nat Methods, Vol. 16, pages 722-730). Promising KKH-SaCas9 variants with high activity and targeting accuracy were identified among the variants analyzed. Eight of the variants (i.e., v3.18, v3.8, v3.22, v3.24, v3.19, v3.16, v3.10, v3.2) demonstrated high on-target activities (with >60% of KKH-SaCas9 activity at day 15 post-transduction, averaged from two sgRNAs). Seven of the variants demonstrated significantly reduced off-target activities (decreased by >90%; being characterized using 3 individual sgRNAs each bearing a single-base-pair-mismatched protospacer sequence) (**Figure 3). ** In particular, the variant v3.16 harboring Y239H/N419D/R499A/Q500A/Y651H mutations generated the fewest off-target edits after 15 days post-transduction (reduced by >95%) and resulted in an average of ~70% of on-target activity, when compared to KKH-SaCas9 (**Figure 3; Figure 17**). The variant v3.16 demonstrated better discrimination of most of the single-base-pair mismatches between the DNA target and the tested sgRNA, which spanned the entirety of the protospacer sequence (**Figures 7A and 7B; Figures 18A-18D**). This super-accurate variant was designated KKH-SaCas9-SAV1 (hereinafter also referred to as "SAV1"). Another second highly accurate variant was identified, variant v3.10 (hereinafter referred to as KKH-SaCas9-SAV2 or "SAV2") harboring Y239H/N419D/R654A/G655A mutations. The SAV2 variant exhibited very few off-target edits at day 7 post-transduction, similar to SAV1, and exhibited comparable on-target activity (an average of ~80%) to KKH-SaCas9 (**Figures 3** and 1D; **Figure 17**; **Figures 18A-18D**). Variants harboring additional substitution(s) over the quadruple mutant SAV2 exhibited lower on-target activities (**Figures 3 and 8A-8C**). A third variant, v3.1, which was like SAV2, but carried R245A instead of Y239H, was also tested. Variant v3.1 also demonstrated less on-target edits than SAV2 (**Figure 3**). The R245-containing v3.1 when added with Y239H (as well as other tested variants containing both Y239H and R245A) exhibited reduced editing efficiency more consistently across the two on-target sites tested (**Figure 3**). These results suggest that Y239H and R245A co-mutation is particularly detrimental to the enzyme's activity. SAV1 and SAV2 were thus selected for further characterization.

***Y239H is important for KKH-SaCas9-SAV2's editing specificity and activity.***

Attempts were made to gain structural insights regarding why KKH-SaCas9-SAV1 and SAV2 exhibited low off-target and high on-target activities. The results revealed a pivotal role of the Y239H substitution in determining target accuracy while maintaining the activity of KKH-SaCas9. It was found that SAV2 lacking Y239H (i.e., variant v3.2) generated significantly increased off-target edits (**Figure 3; Figure 17**). Therefore, the triple mutant combination (N419D/R654A/G655A) alone is insufficient to minimize off-target editing. Replacing Y239H with R245A in the SAV2 mutant increased off-target edits, but this variant also exhibited reduced on-target activity (**Figure 3**). Based on molecular modelling assessments, it was predicted that mutating Y239 into histidine could weaken the enzyme's bonding with the sgRNA backbone to reduce off-target editing, while preserving the $\pi$-$\pi$ interactions with its F418 **(Figures 10A-10C).** The Y239 residue was also mutated into arginine to facilitate maintenance of its bonding with the sgRNA backbone but form a cation-$\pi$ interaction with F418 **(Figures 10A-10C).** The variant bearing Y239R instead of Y239H generated less overall activity with a more similar on-to-off targeting ratio as KKH-SaCas9 **(Figure 8A-8C).** This observation supports the involvement of the $\pi$-$\pi$ interaction in maintaining the enzyme's structural stability and activity.

It was observed that adding substitutions including N394T (i.e., v3.24 in **Figure 3)** or T392A/N394T **(Figure 8A-8C)** to SAV2 decreased its activity. N394T was modelled to reduce interaction with the sgRNA backbone at the side opposite to where Y239 interacts **(Figure 19).** Losing multiple interactions with the sgRNA backbone may account for the drop in SAV2's activity when these mutations were added. The observations that SAV2 with Y239R instead of its original Y239H mutation rendered SAV2 less susceptible to the drop in activity brought by T392A/N394T addition suggested that Y239R does not lose its interaction with the sgRNA backbone. Therefore, tuning the enzyme's interaction with the sgRNA backbone to reduce off-target editing, while maintaining high on-target activity, requires optimal engineering at specific site(s).

In addition, the replacement of R245A with Y239H in KKH-SaCas9-HF was tested for improved activity and target accuracy. It was found that such replacement resulted in fewer off-target edits as well as partial restoration of the enzyme's on-target activity **(Figure 3; Figure 17).** Nonetheless, this variant demonstrated significantly decreased on-target activity and increased off-target activity than SAV2, suggesting that Y239H collaborates with the off-target-reducing N419D/R654A/G655A mutations (i.e., variant v3.2, **Figure 3)** to achieve optimal editing performance for SAV2.

***Comparison of on- and off- target activities of KKH-SaCas9 variants***

The on- and off-target activities of SAV1 and SAV2 were compared with existing/candidate high-fidelity variants of KKH-SaCas9. The results from GFP disruption assays revealed that SAV1 and SAV2 have higher on-target editing activity than KKH-SaCas9-HF, (i.e., ~70%, ~85%, and ~40% of KKH-SaCas9 activity for SAV1, SAV2, and KKH-SaCas9-HF, respectively), and generated significantly less off-target edits (i.e., reduced by >98%, -95%, and ~60% for SAV1, SAV2, and KKH-SaCas9-HF, respectively, when compared to KKH-SaCas9) **(Figure 11).** A candidate N260D substitution was more recently reported to reduce the off-target activity of SaCas9 (Xie, H., et al. (2020) PLoS Biol, Vol. 18 (7), e3000747, doi:10.1371/journal.pbio.3000747). This mutation was grafted onto KKH-SaCas9 to generate KKH-efSaCas9. Although this variant exhibited comparable on-target activity to KKH-SaCas9 and SAV2, it also generated a high frequency of off-target edits (i.e., reduced by only -40% for KKH-efSaCas9 versus ~95% for SAV2, when compared to KKH-SaCas9) **(Figure 11).** Adding N260D to mutations on SAV1 and SAV2 greatly reduced their on-target activities **(Figures 20A and 20B).** Another variant with mutations were grafted from eSaCas9 (Slaymaker, et al. (2016) Science, Vol. 351, 84-88 doi: 10.1126/science.aad5227) onto KKH-SaCas9. It was observed that this mutant generated fewer off-target edits than that generated by wild-type but more off-target edits generated by SAV2, but also produced more on-target edits with the two tested sgRNAs **(Figure 12).** The differences in the observed editing efficiencies were not due to the discrepancy in protein expression levels of the variants **(Figure 2).** These results indicated that SAV1 and SAV2, along with KKH-eSaCas9, showed superior fidelity and efficiency compared to other existing variants **(Figure 9).**

***Evaluation of endogenous on-target and genome-wide off-target activities of KKH-SaCas9 variants.***

The performance of SAV1, SAV2, and KKH-eSaCas9 in editing endogenous genomic loci was further characterized. T7 Endonuclease I mismatch detection assays, Genome-wide unbiased identification of double-strand breaks enabled by sequencing (GUIDE-seq), and deep sequencing were performed to evaluate the on- and off-target activities in three cell lines - OVCAR8-ADR, SK-N-MC, and MHCC97L cells. Multiple endogenous loci (Kleinstiver, B.P., et al. (2015) Nat Biotechnol, Vol. 33, pages 1293-1298, doi: 10.1038/nbt.3404; Tan, et al. Proc NatlAcadSci U.S.A, 116, 20969-20976, doi: 10.1073/pnas. 1906843116 (2019)) were assayed. It was demonstrated that SAV1, SAV2, and KKH-eSaCas9 exhibited a median editing efficiency of 51%, 72%, and 87% of KKH-SaCas9's activity, respectively **(Figures 4A-4C; Figures 21A-21C)** in OVCAR8-ADR cells, which was significantly higher than KKH-SaCas9-HF **(Figure 1).** Comparison of the variants' on-target activity was extended to SK-N-MC and MHCC97L cells, and the results revealed a similar trend. The normalized median editing efficiency for each variant was: 43% for SAV1, 65% for SAV2, and 111% for KKH-eSaCas9 in SK-N-MC cells, while 65% for SAV1, 81% for SAV2, and 110% for KKH-eSaCas9 in MHCC97L cells **(Figures 4A-4C).** GUIDE-seq results indicated that SAV1 and SAV2 resulted in higher on- to off-targeting ratio than wild-type in all tested loci **(Figure 4B; Figures 14A, 14D, and 14E).**

***KKH-SaCas9- SA V1 and SAV2 showed improved single-base mismatch discrimination***

The variants' ability to discriminate target sequences with single-base mismatches was further evaluated. Deep sequencing analysis was performed using a panel of sgRNAs that are perfectly matched or carry a single-base mismatch to the target sequences (i.e., VEGFA and FANCF). Compared with wild-type and KKH-eSaCas9, SAV1 and SAV2 demonstrated significantly better discrimination of the single-base mismatches, including those located distal to the PAM region, between the endogenous target and the sgRNAs **(Figures 15A and 15B).** These results corroborate the on- and off-target activities observed for the SAV1 and SAV2 variants in the above-described experiments using GFP disruption assays. The results also confirm the high fidelity of KKH-SaCas9-SAV1 and KKH-SaCas9-SAV2 for efficiently generating more accurate genomic edits against sequences with a single-base mismatch.

**Figure 2** shows the on-target and off target editing efficiencies of KKH-SaCas9-SAV1 and SAV2. SAV1 and SAV2 had varying levels of indel frequencies for the 11 loci tested, with an average of ~60% and -77% of KKH-SaCas9's editing activity for SAV1 and SAV2, respectively **(Figure 13).** SAV1 and SAV2's editing activity was also much higher than KKH-SaCas9-HF **(Figure 1). Figures 14A-14E** shows the GUIDE-seq genome-wide specificity profiles for the KKH-SaCas9 variants paired with the indicated sgRNAs targeted to five endogenous gene sites containing NNGRRT PAMS. Mismatched positions in off-target sites are in grey boxes, and GUIDE-seq read counts were used as a measure of the cleavage efficiency at a given site. GUIDE-seq results indicated that SAV1 and SAV2 had much higher on- to off-targeting ratio, inducing nearly identical GUIDE-seq tag integration rates and on-target cleavage frequencies for all five sites **(Figures 14A-14E).** For example, SAV2 and SAV1 showed little to no detectable off-target editing while maintaining a high on- to off-target read ratio for *EMX1-sg2, AAVSl-sg4* and *VEGF-sg3* compared to WT KKH-SaCas9 *(see* EMX1_sg2, AAVS1_sg4 and VEGFA_sg3, **Figures 14A, 14B and 14D).** Also, SaCas9, SAV1 and SAV2 had 42, 4 and 2 off-target sites respectively, detected in the PAM site EMX1_sg7 (see EMX1_sg7, **Figure 14C).** These results corroborate the on- and off-target activities observed for these variants in the experiments using GFP disruption assays **(Figure 1)** and confirm the high fidelity of KKH-SaCas9-SAV1 and KKH-SaCas9-SAV2 for efficiently generating accurate genomic edits.

The disclosed SaCas9 protein variants can also edit target more distal from the PAM than SaCas9. As shown in **Figure 15,** SAV1 and SAV2 show good editing efficiency even with single-nucleotide differences located distantly from the PAM. Prior strategies to target mutant allele using SaCas9 require the pathogenic single-nucleotide polymorphism (SNP) or mutation to be located within the seed region of the sgRNA or using an SNP-derived PAM to achieve SNP-specific targeting without cleaving the wild-type allele. However, these do not apply to SNPs that are located outside of the seed region or those that do not generate a new PAM for SaCas9 targeting. The unique ability of SAV1 and SAV2 in distinguishing a broader range of single-nucleotide mismatches expands the scope and capabilities of genome editing at loci with SNPs and mutations located further away from the PAM, which has not been previously achieved.

**Summary and Conclusions**

In summary, through combinatorial mutagenesis, KKH-SaCas9-SAV1 and KKH-SaCas9-SAV2 variants were successfully identified. These variants harbor new sets of mutations that confer KKH-SaCas9 with high editing accuracy and efficiency. The work of the current study addresses the unmet need for highly specific and efficient variants of KHH-SaCas9 that can make edits across a broad range of genomic targets (i.e., with "NNNRRT" PAM), including sites harboring "NHHRRT" PAM that could not be targeted by other high-fidelity SpCas9 variants that recognize "NGG" PAM. The results of the current study also reveal that SAV1 and SAV2 have an enhanced ability to distinguish targets with single-nucleotide differences including those located distantly from the PAM. Current strategies to target mutant allele using SaCas9 requires the pathogenic single-nucleotide polymorphism (SNP) or mutation to be located within the seed region of the sgRNA or using an SNP-derived PAM to achieve SNP-specific targeting without cleaving the wild-type allele. However, these do not apply to SNPs that are located outside of the seed region or those that do not generate a new PAM for SaCas9 targeting. The unique ability of SAV1 and SAV2 in distinguishing a broader range of single-nucleotide mismatches could expand the scope and capabilities of genome editing at loci with SNPs and mutations located further away from the PAM, which has not been previously achieved. When compared to wild-type KKH-SaCas9, it was observed that some of the endogenous target loci demonstrated greater reduction in editing efficiency when SAV1 and SAV2 were used. Such variability of the relative editing efficiency among loci was also previously reported for other high-fidelity SpCas9 variants (Chen, J.S., et al. (2017) Nature, Vol. 550, 407-410; Kulcsar, P.I., et al. (2020) Nat Commun, Vol. 11, Article 1223). This could be due to the sgRNA/target sequence dependencies for each variant because each variant was engineered with mutations that interact with different regions of the DNA and/or sgRNA backbone(s).

Screening combinatorial mutations have been technically challenging due to the vast combinatorial space within which to search, and only a limited number of mutants could be characterized in practice. For example, performing a saturated mutagenesis screen on 12 amino acid residues requires $20^{12}$ (i.e., $4 \times 10^{15}$) variants to be screened, which is practically infeasible. In the current study, a structure-guided approach was applied to rationally select mutations for engineering and testing. The results demonstrate the feasibility of engineering highly accurate KKH-SaCas9 enzyme via mutating multiple DNA- and sgRNA- interacting residues that span over the different parts of the protein. Notably, it was observed that grafting SAV1 and SAV2 mutations onto the nuclease-dead version of KKH-SaCas9 showed comparable gene knockdown efficiency and specificity to their wild type and KKH-eSaCas9 counterparts **(Figures 22A-22D).** This suggests that the enhanced editing specificity of SAV1 and SAV2 nucleases is more likely dictated at the DNA cleavage level, rather than the DNA binding level. The mutations, including Y239H, identified in this work can be used as building blocks for further engineering of KKH-SaCas9 nucleases.

## ASPECTS

The following numbered paragraphs which are not the claims also define the invention

1. A SaCas9 variant comprising the mutation Y239H and not comprising the mutation R245A.
2. The variant of paragraph 1 further comprising the mutations E782K, N968K, and R1015H.
3. The variant of paragraph 1 or 2, wherein the variant does not include any other mutation or combination of other mutations such that the variant has greater off-target activity than SaCas9 variant v3.2 in a GFP disruption assay at 15 days in OVCAR8-ADR cells, SK-N-MC cells, and/or MHCC97L cells harboring a reporter construct expressing an off-target sgRNA having the sequence CACCTACGGCAATCTGACCCTGAAGT (SEQ ID NO:1), wherein the SaCas9 variant v3.2 has only the mutations N419D, R654A, G655A, E782K, N968K, and R1015H.
4. The variant of any one of paragraphs 1-3, wherein the variant does not include any other mutation or combination of other mutations such that the variant has on-target activity less than 0.5 of the on-target activity of SaCas9 variant KKH-SaCas9 in a GFP disruption assay at 15 days in OVCAR8-ADR cells, SK-N-MC cells, and/or MHCC97L cells harboring a reporter construct expressing an on-target sgRNA having the sequence CACCTACGGCAAGCTGACCCTGAAGT (SEQ ID NO:2), wherein the SaCas9 variant KKH-SaCas9 has only the mutations E782K, N968K, and R1015H.
5. The variant of any one of paragraphs 1-4 further comprising one or more mutations selected from the group consisting of T238A, T392A, N394T, N394A, N413A, Q414R, N419A, N419D, N419S, N419G, R499A, Q500A, Y651H, R654A, and G655A.
6. The variant of any one of paragraphs 1-5 including the mutation N419D.
7. The variant of any one of paragraphs 1-5 including the mutation N419S.
8. The variant of any one of paragraphs 1-5 including the mutation N419G.
9. The variant of any one of paragraphs 1-8 including the mutation R499A.
10. The variant of any one of paragraphs 1-9 including the mutation Q500A.
11. The variant of any one of paragraphs 1-10 including the mutation Y651H.
12. The variant of any one of paragraphs 1-11 including the mutation R654A.
13. The variant of any one of paragraphs 1-12 including the mutation G655A.
14. The variant of any one of paragraphs 1-13 including the mutation Q414R.
15. The variant of any one of paragraphs 1-14 including the mutation N394T.
16. The variant of any one of paragraphs 1-14 including the mutation N394A.
17. The variant of any one of paragraphs 1-16 including the mutation T392A.
18. The variant of any one of paragraphs 1-17 including the mutation T238A.
19. The variant of any one of paragraphs 1-5 including one or more mutations selected from the group consisting of R499A, Q500A, Y651H, R654A, and G655A,
20. The variant of any one of paragraphs 1-5, wherein the variant is v3.18, v3.8, v3.22, v3.16, v3.10, v3.24, or v3.19.
21. A construct encoding the variant of any one of paragraphs 1-20 for expression of the variant in a host of interest.
22. The construct of paragraph 21 comprising sequences for expression of the variant in the host of interest.
23. The construct of paragraph 21 or 22 further encoding an sgRNA targeting a sequence of interest and sequences for expression of the sgRNA in the host of interest.
24. The construct of any one of paragraphs 21-23 comprised in a virus vector.

25. The construct of paragraph 24, wherein the virus vector is an adeno-associated virus vector.

26. A method of editing a sequence of interest, the method comprising contacting the construct of any one of paragraphs 23-25 with the host of interest, wherein the host of interest harbors the sequence of interest, wherein the cell expresses the construct to produce variant and the sgRNA.

27. A method of editing a sequence of interest, the method comprising contacting the construct of paragraphs 21 or 22 with the host of interest, wherein the host of interest harbors a sequence of interest, wherein the cell expresses the construct to produce the variant.

28. The method of paragraph 27 further comprising causing an sgRNA targeting the sequence of interest to be present in the host of interest with the produced variant, whereby the produced variant edits the sequence of interest targeted by the sgRNA.

29. A mixture comprising the variant of any one of paragraphs 1-20 and an sgRNA targeting a sequence of interest.

30. The mixture of paragraph 29 comprised in a delivery particle.

31. The mixture of paragraph 29 comprised in a cell containing the sequence of interest.

32. A method of editing a sequence of interest, the method comprising contacting the sequence of interest with a mixture of any one of paragraphs 29-31, whereby the variant edits the sequence of interest targeted by the sgRNA.

## Claims

1. A SaCas9 variant comprising the mutation Y239H and not comprising the mutation R245A.

2. The variant of claim 1,

   (a) further comprising the mutations E782K, N968K, and R1015H; and/or
   (b) wherein the variant does not include any other mutation or combination of other mutations such that the variant has greater off-target activity than SaCas9 variant v3.2 in a GFP disruption assay at 15 days in OVCAR8-ADR cells, SK-N-MC cells, and/or MHCC97L cells harboring a reporter construct expressing an off-target sgRNA having the sequence CACCTACGGCAATCTGACCCTGAAGT (SEQ ID NO:1), wherein the SaCas9 variant v3.2 has only the mutations N419D, R654A, G655A, E782K, N968K, and R1015H; and/or
   (c) wherein the variant does not include any other mutation or combination of other mutations such that the variant has on-target activity less than 0.5 of the on-target activity of SaCas9 variant KKH-SaCas9 in a GFP disruption assay at 15 days in OVCAR8-ADR cells, SK-N-MC cells, and/or MHCC97L cells harboring a reporter construct expressing an on-target sgRNA having the sequence CACCTACGGCAAGCTGACCCTGAAGT (SEQ ID NO:2), wherein the SaCas9 variant KKH-SaCas9 has only the mutations E782K, N968K, and R1015H; and/or
   (d) further comprising one or more mutations selected from the group consisting of T238A, T392A, N394T, N394A, N413A, Q414R, N419A, N419D, N419S, N419G, R499A, Q500A, Y651H, R654A, and G655A.

3. The variant of claim 1 or 2 including the mutation N419D, the mutation N419S, or the mutation N419G.

4. The variant of any one of claims 1-3 including the mutation R499A, the mutation Q500A, the mutation Y651H, the mutation R654A, the mutation G655A, and/or
   the mutation Q414R.

5. The variant of any one of claims 1-4 including the mutation N394T or the mutation N394A.

6. The variant of any one of claims 1-5 including the mutation T392A and/or
   the mutation T238A.

7. The variant of claim 1 or 2 including one or more mutations selected from the group consisting of R499A, Q500A, Y651H, R654A, and G655A,

8. The variant of claim 1 or 2, wherein the variant is v3.18, v3.8, v3.22, v3.16, v3.10, v3.24, or v3.19.

9. A construct encoding the variant of any one of claims 1-8 for expression of the variant in a host of interest.

10. The construct of claim 9,

    (a) comprising sequences for expression of the variant in the host of interest, and/or

(b) further encoding an sgRNA targeting a sequence of interest and sequences for expression of the sgRNA in the host of interest; and/or
(c) comprised in a virus vector, optionally wherein the virus vector is an adeno-associated virus vector.

**11.** A method of editing a sequence of interest, the method comprising contacting the construct of any one of claims 10(b)-10(c) with the host of interest, wherein the host of interest harbors the sequence of interest, wherein the cell expresses the construct to produce variant and the sgRNA.

**12.** A method of editing a sequence of interest, the method comprising contacting the construct of claim 9 or 10(a) with the host of interest, wherein the host of interest harbors a sequence of interest, wherein the cell expresses the construct to produce the variant.

**13.** The method of claim 12 further comprising causing an sgRNA targeting the sequence of interest to be present in the host of interest with the produced variant, whereby the produced variant edits the sequence of interest targeted by the sgRNA.

**14.** A mixture comprising the variant of any one of claims 1-8 and an sgRNA targeting a sequence of interest, optionally wherein
the mixture is (i) comprised in a delivery particle or (ii) comprised in a cell containing the sequence of interest.

**15.** A method of editing a sequence of interest, the method comprising contacting the sequence of interest with a mixture of claim 14, whereby the variant edits the sequence of interest targeted by the sgRNA.

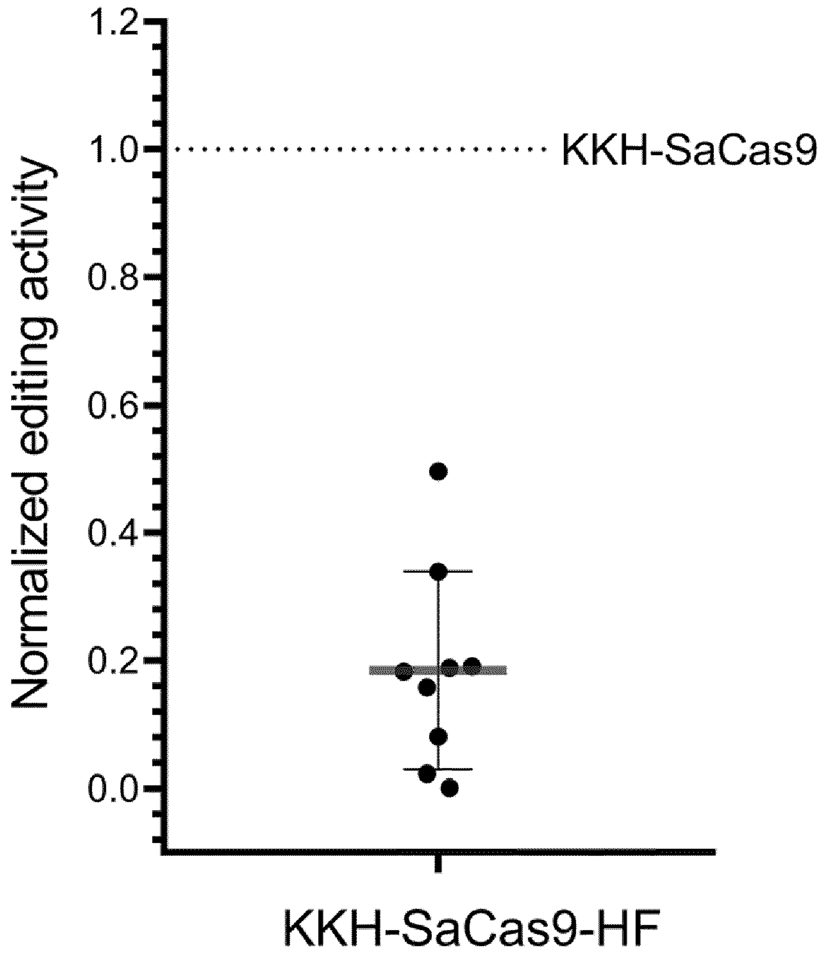
1.2
1.0
0.8
0.6
0.4
0.2
0.0
Normalized editing activity
KKH-SaCas9
KKH-SaCas9-HF

FIG. 1A

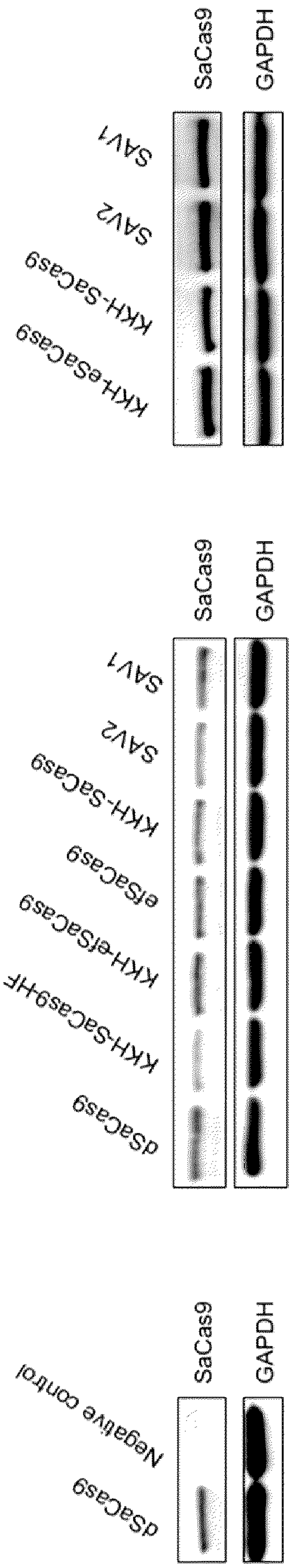
SaCas9
GAPDH
SAV1
SAV2
KKH-SaCas9
KKH-eSaCas9
efSaCas9
KKH-efSaCas9
KKH-SaCas9-HF
dSaCas9
Negative control

FIG. 2

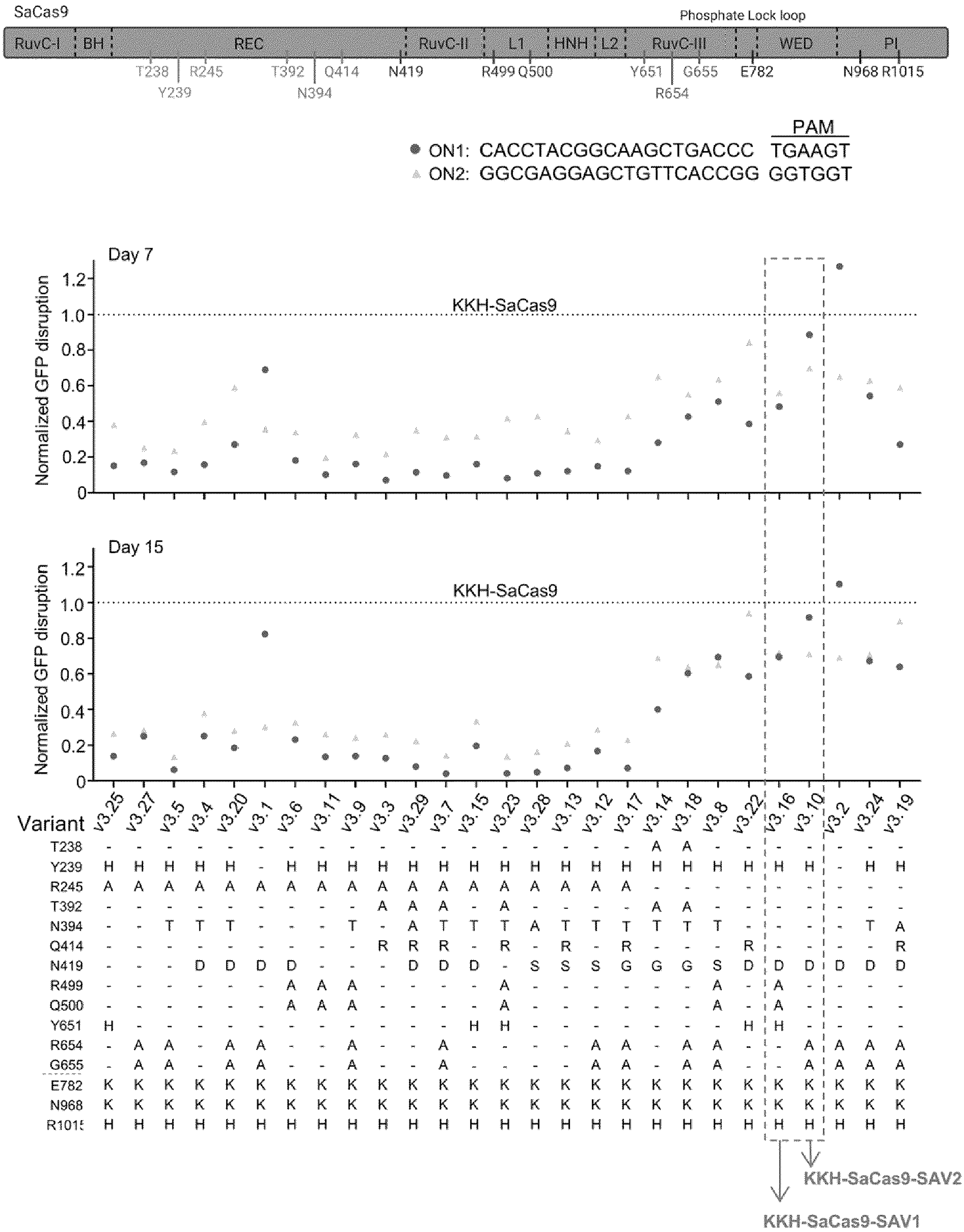
SaCas9
Phosphate Lock loop
RuvC-I BH REC RuvC-II L1 HNH L2 RuvC-III WED PI
T238 R245 T392 Q414 N419 R499 Q500 Y651 G655 E782 N968 R1015
Y239 N394 R654
PAM
ON1: CACCTACGGCAAGCTGACCC TGAAGT
ON2: GGCGAGGAGCTGTTCACCGG GGTGGT
Day 7
Day 15
Normalized GFP disruption
KKH-SaCas9
Variant v3.25 v3.27 v3.5 v3.4 v3.20 v3.1 v3.6 v3.11 v3.9 v3.3 v3.29 v3.7 v3.15 v3.23 v3.28 v3.13 v3.12 v3.17 v3.14 v3.18 v3.8 v3.22 v3.16 v3.10 v3.2 v3.24 v3.19
T238 - - - - - - - - - - - - - - - - - - A A - - - - - - -
Y239 H H H H H - H H H H H H H H H H H H H H H H H H - H H
R245 A A A A A A A A A A A A A A A A A A - - - - - - - - -
T392 - - - - - - - - - A A A - A - - - - A A - - - - - - -
N394 - - T T T - - - T - A T T T A T T T T T T - - - - T A
Q414 - - - - - - - - - R R R - R - R - R - - - R - - - - R
N419 - - - D D D D - - - D D D - S S S G G G S D D D D D D
R499 - - - - - - A A A - - - - A - - - - - - A - A - - - -
Q500 - - - - - - A A A - - - - A - - - - - - A - A - - - -
Y651 H - - - - - - - - - - - H H - - - - - - - H H - - - -
R654 - A A - A A - - A - - A - - - - A A - A A - - A A A A
G655 - A A - A A - - A - - A - - - - A A - A A - - A A A A
E782 K K K K K K K K K K K K K K K K K K K K K K K K K K K
N968 K K K K K K K K K K K K K K K K K K K K K K K K K K K
R1015 H H H H H H H H H H H H H H H H H H H H H H H H H H H
KKH-SaCas9-SAV2
KKH-SaCas9-SAV1

FIG. 3

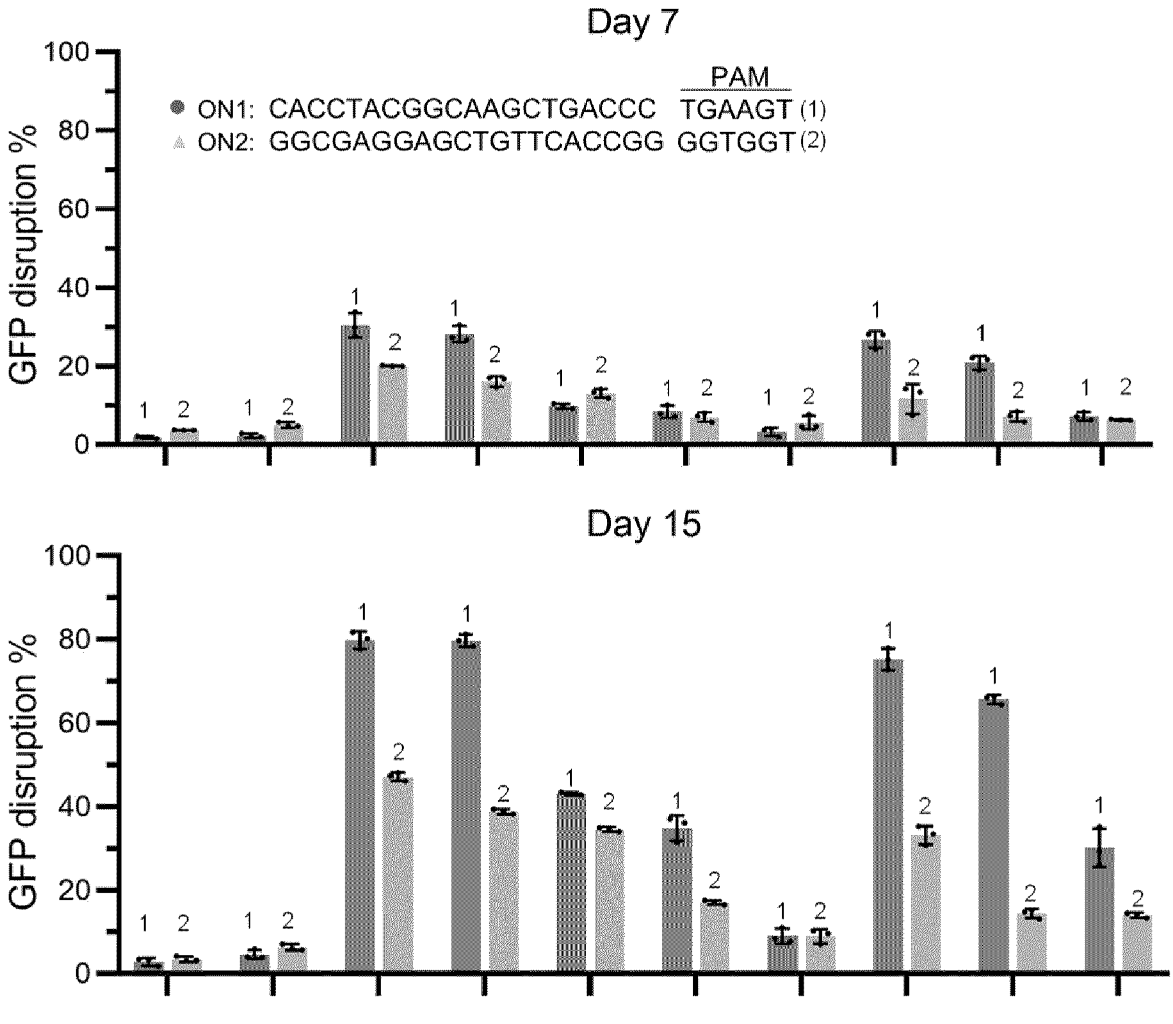
Day 7
GFP disruption %
100
80
60
40
20
0
PAM
ON1: CACCTACGGCAAGCTGACCC TGAAGT (1)
ON2: GGCGAGGAGCTGTTCACCGG GGTGGT (2)
Day 15
GFP disruption %
100
80
60
40
20
0

FIG. 4A

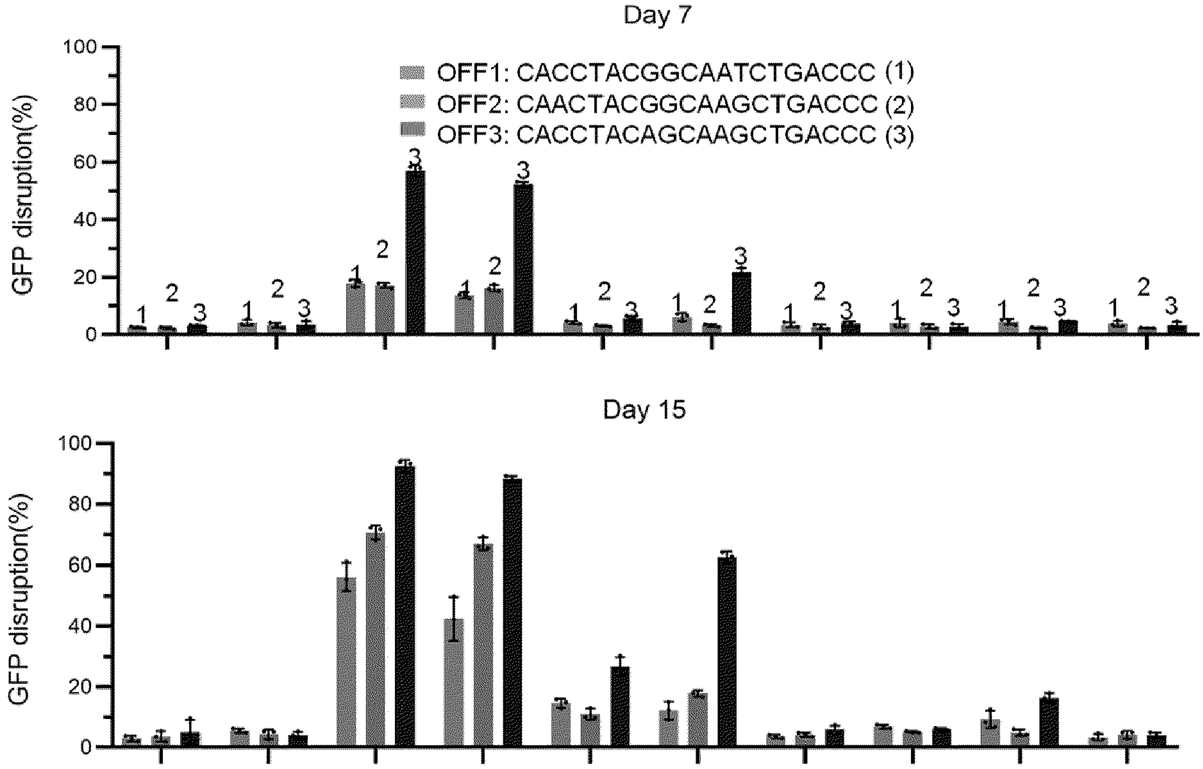
Day 7
OFF1: CACCTACGGCAATCTGACCC (1)
OFF2: CAACTACGGCAAGCTGACCC (2)
OFF3: CACCTACAGCAAGCTGACCC (3)
GFP disruption(%)
Day 15
GFP disruption(%)

FIG. 4B

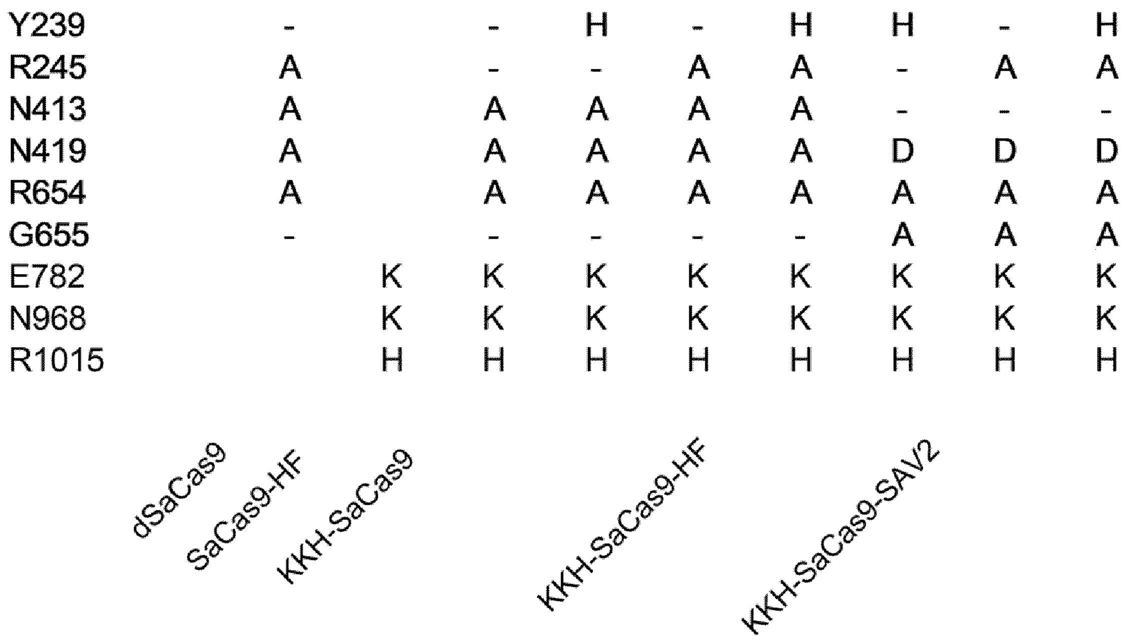
Y239 - - H - H H - H
R245 A - - A A - A A
N413 A A A A A - - -
N419 A A A A A D D D
R654 A A A A A A A A
G655 - - - - - A A A
E782 K K K K K K K K
N968 K K K K K K K K
R1015 H H H H H H H H
dSaCas9
SaCas9-HF
KKH-SaCas9
KKH-SaCas9-HF
KKH-SaCas9-SAV2

FIG. 4C

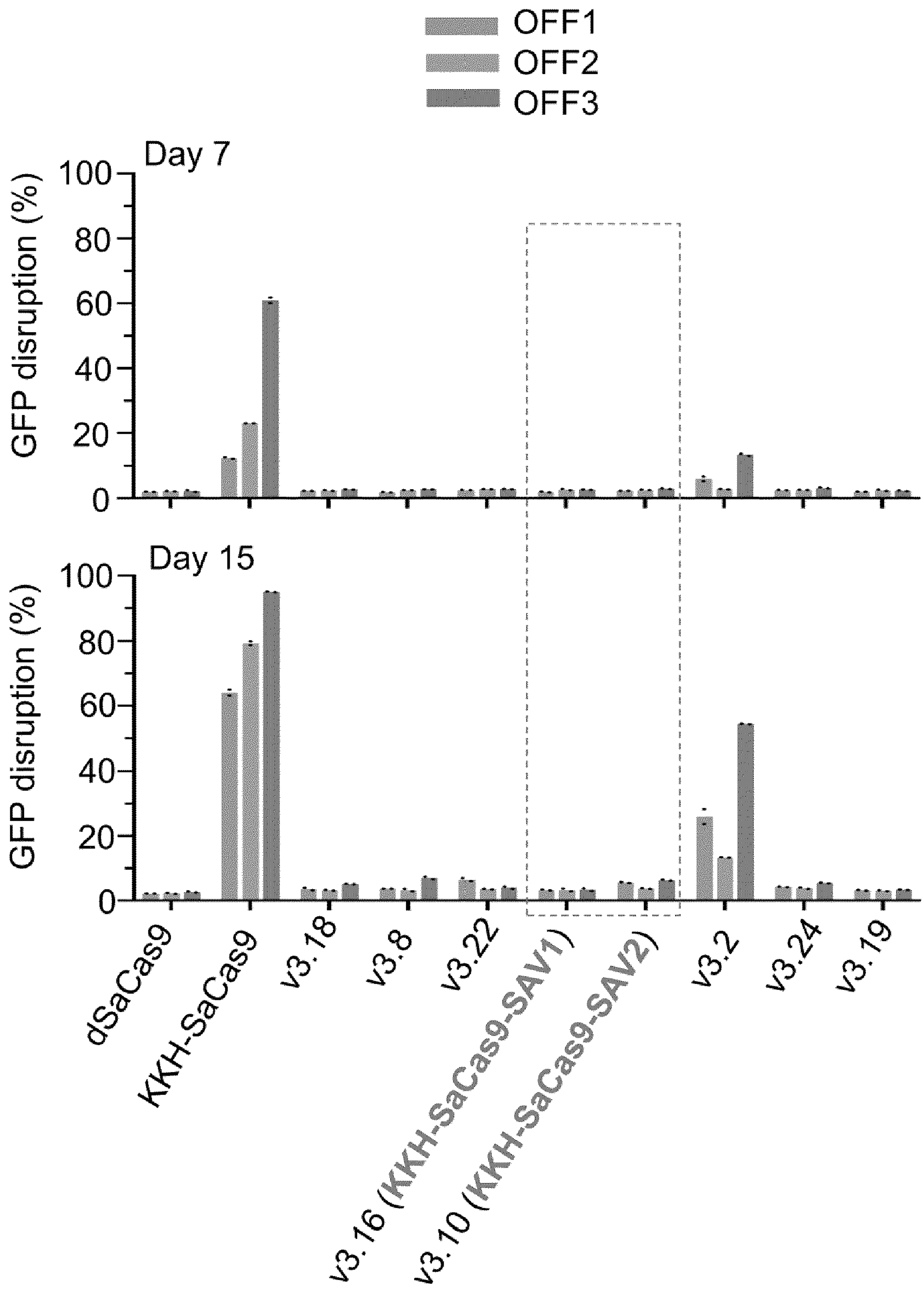
OFF1
OFF2
OFF3
Day 7
100
80
60
40
20
0
GFP disruption (%)
Day 15
100
80
60
40
20
0
GFP disruption (%)
dSaCas9
KKH-SaCas9
v3.18
v3.8
v3.22
v3.16 (KKH-SaCas9-SAV1)
v3.10 (KKH-SaCas9-SAV2)
v3.2
v3.24
v3.19

**FIG. 5**

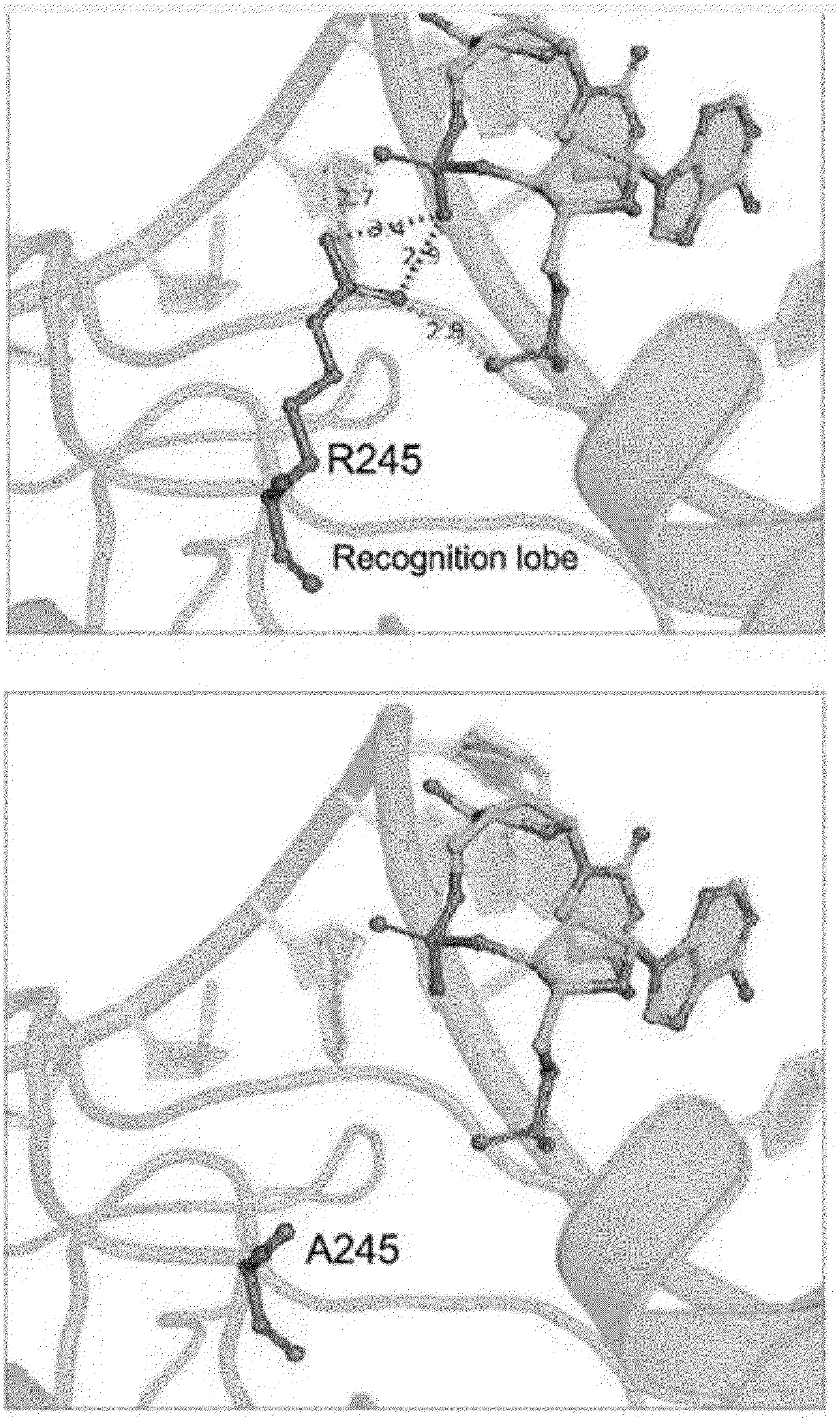


FIG. 6

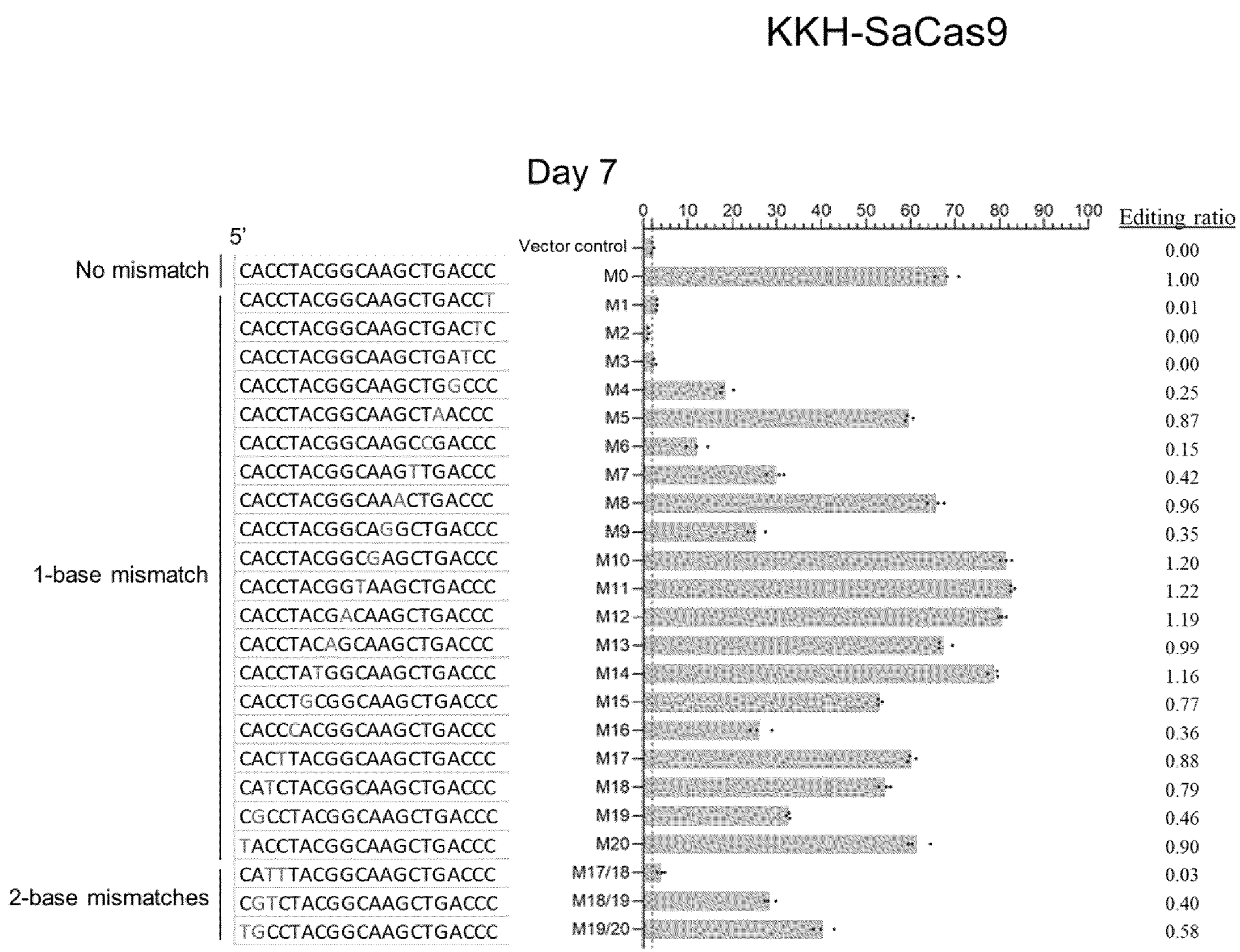
KKH-SaCas9
Day 7
5'
No mismatch
1-base mismatch
2-base mismatches
CACCTACGGCAAGCTGACCC
CACCTACGGCAAGCTGACCT
CACCTACGGCAAGCTGACTC
CACCTACGGCAAGCTGATCC
CACCTACGGCAAGCTGGCCC
CACCTACGGCAAGCTAACCC
CACCTACGGCAAGCCGACCC
CACCTACGGCAAGTTGACCC
CACCTACGGCAAACTGACCC
CACCTACGGCAGGCTGACCC
CACCTACGGCGAGCTGACCC
CACCTACGGTAAGCTGACCC
CACCTACGACAAGCTGACCC
CACCTACAGCAAGCTGACCC
CACCTATGGCAAGCTGACCC
CACCTGCGGCAAGCTGACCC
CACCCACGGCAAGCTGACCC
CACTTACGGCAAGCTGACCC
CATCTACGGCAAGCTGACCC
CGCCTACGGCAAGCTGACCC
TACCTACGGCAAGCTGACCC
CATTTACGGCAAGCTGACCC
CGTCTACGGCAAGCTGACCC
TGCCTACGGCAAGCTGACCC
Vector control
M0
M1
M2
M3
M4
M5
M6
M7
M8
M9
M10
M11
M12
M13
M14
M15
M16
M17
M18
M19
M20
M17/18
M18/19
M19/20
0 10 20 30 40 50 60 70 80 90 100
Editing ratio
0.00
1.00
0.01
0.00
0.00
0.25
0.87
0.15
0.42
0.96
0.35
1.20
1.22
1.19
0.99
1.16
0.77
0.36
0.88
0.79
0.46
0.90
0.03
0.40
0.58

FIG. 7A

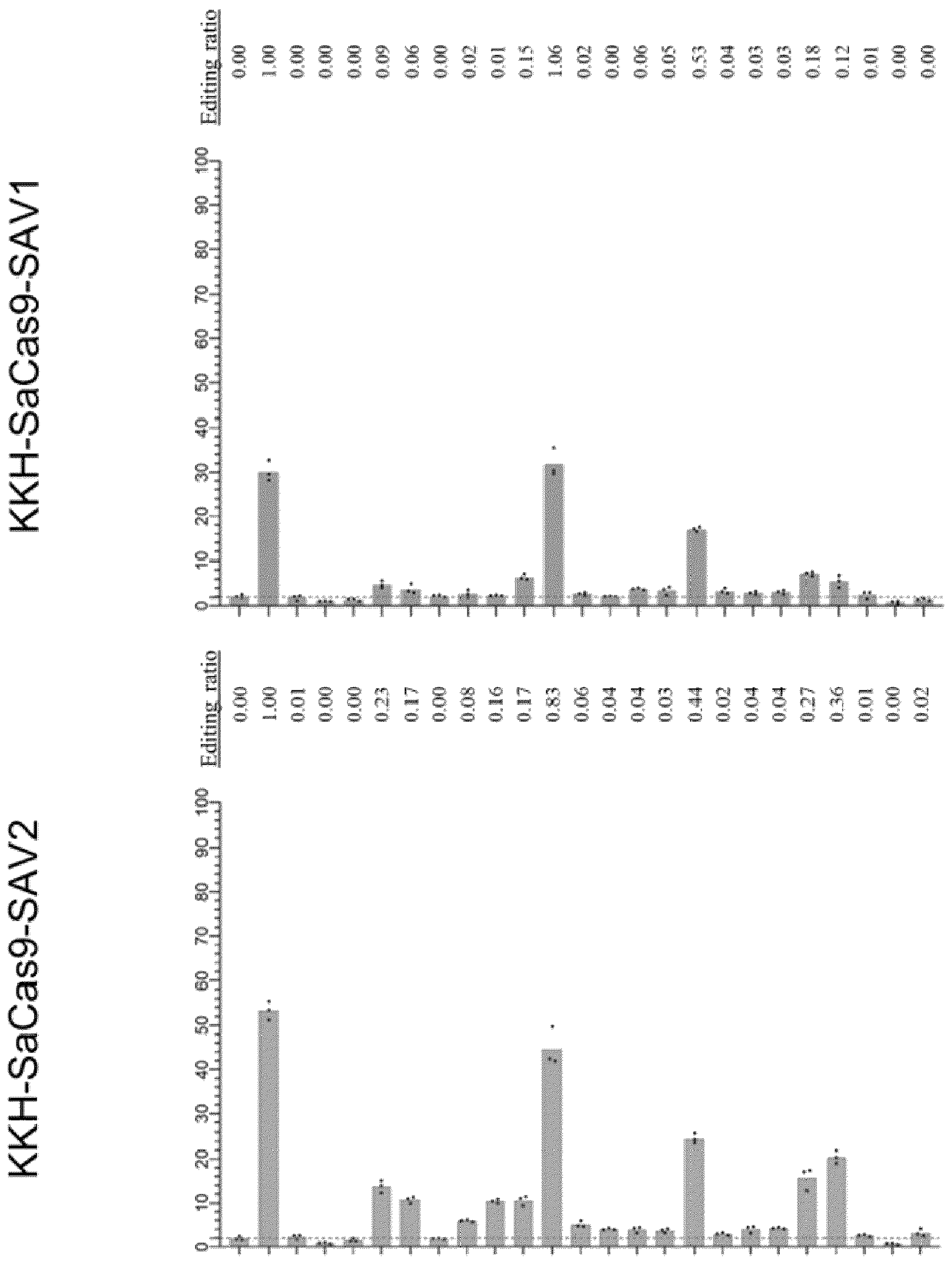
KKH-SaCas9-SAV1
Editing_ratio
0.00
1.00
0.00
0.00
0.00
0.09
0.06
0.00
0.02
0.01
0.15
1.06
0.02
0.00
0.06
0.05
0.53
0.04
0.03
0.03
0.18
0.12
0.01
0.00
0.00
KKH-SaCas9-SAV2
Editing_ratio
0.00
1.00
0.01
0.00
0.00
0.23
0.17
0.00
0.08
0.16
0.17
0.83
0.06
0.04
0.04
0.03
0.44
0.02
0.04
0.04
0.27
0.36
0.01
0.00
0.02
0
10
20
30
40
50
60
70
80
90
100

FIG. 7B

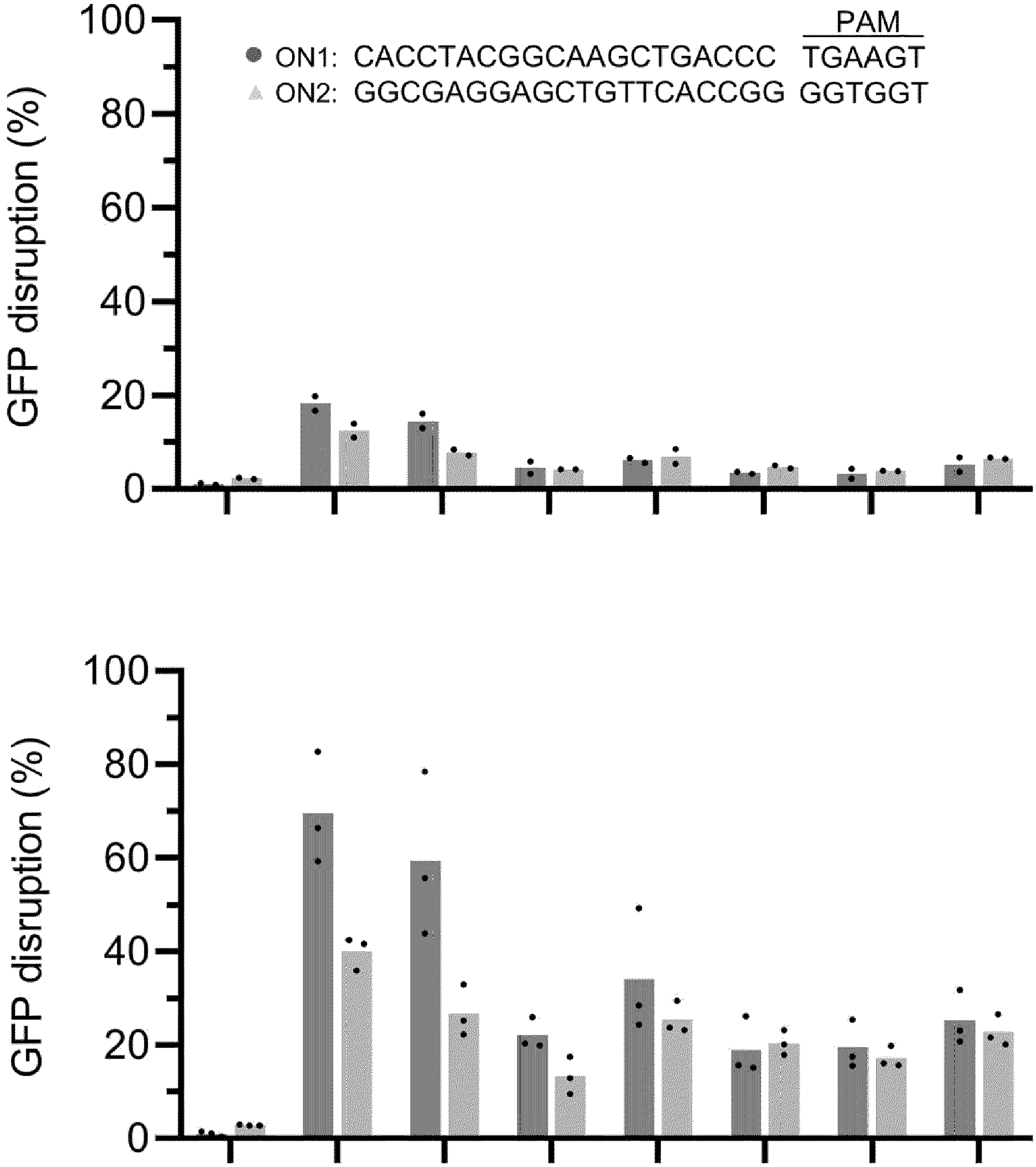
100
80
60
40
20
0
GFP disruption (%)
PAM
ON1: CACCTACGGCAAGCTGACCC TGAAGT
ON2: GGCGAGGAGCTGTTCACCGG GGTGGT
100
80
60
40
20
0
GFP disruption (%)

FIG. 8A

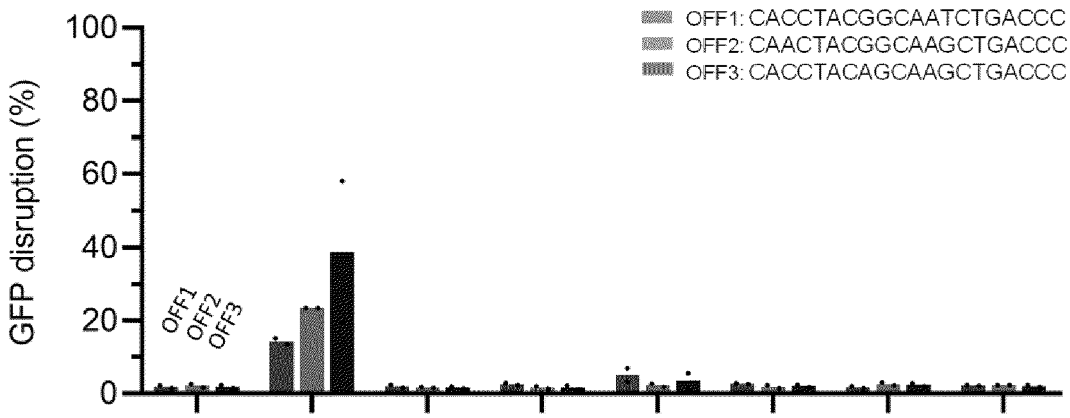
OFF1: CACCTACGGCAATCTGACCC
OFF2: CAACTACGGCAAGCTGACCC
OFF3: CACCTACAGCAAGCTGACCC
GFP disruption (%)
100
80
60
40
20
0
OFF1
OFF2
OFF3

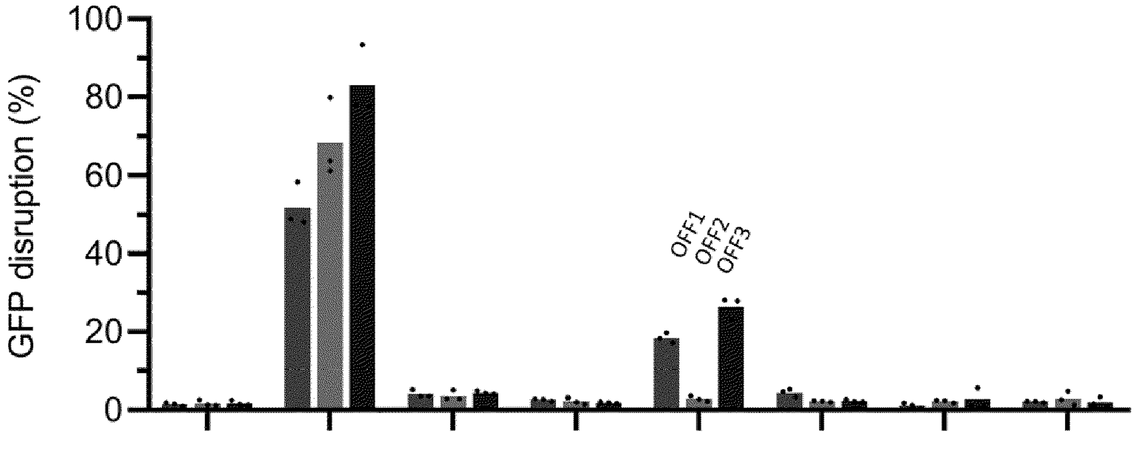
GFP disruption (%)
100
80
60
40
20
0
OFF1
OFF2
OFF3

FIG. 8B

| | dSaCas9 | KKH-SaCas9 | KKH-SaCas9-SAV2 | | | | | |
|---|---|---|---|---|---|---|---|---|
| Y239 | - | - | H | H | R | R | H | R |
| R245 | - | - | - | A | - | K | - | - |
| T392 | - | - | - | - | - | - | A | A |
| N394 | - | - | - | - | - | - | T | T |
| N419 | - | - | D | D | D | D | D | D |
| R654 | - | - | A | A | A | A | A | A |
| G655 | - | - | A | A | A | A | A | A |
| E782 | - | K | K | K | K | K | K | K |
| N968 | - | K | K | K | K | K | K | K |
| R1015 | - | H | H | H | H | H | H | H |

**FIG. 8C**

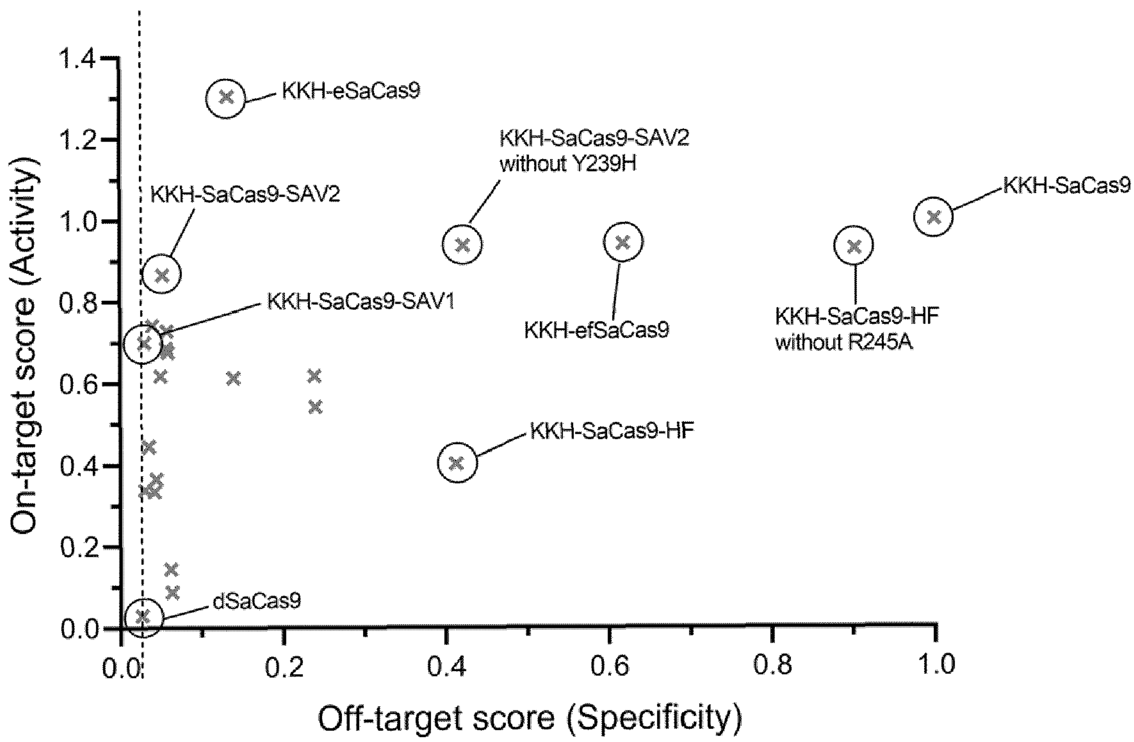


**FIG. 9**

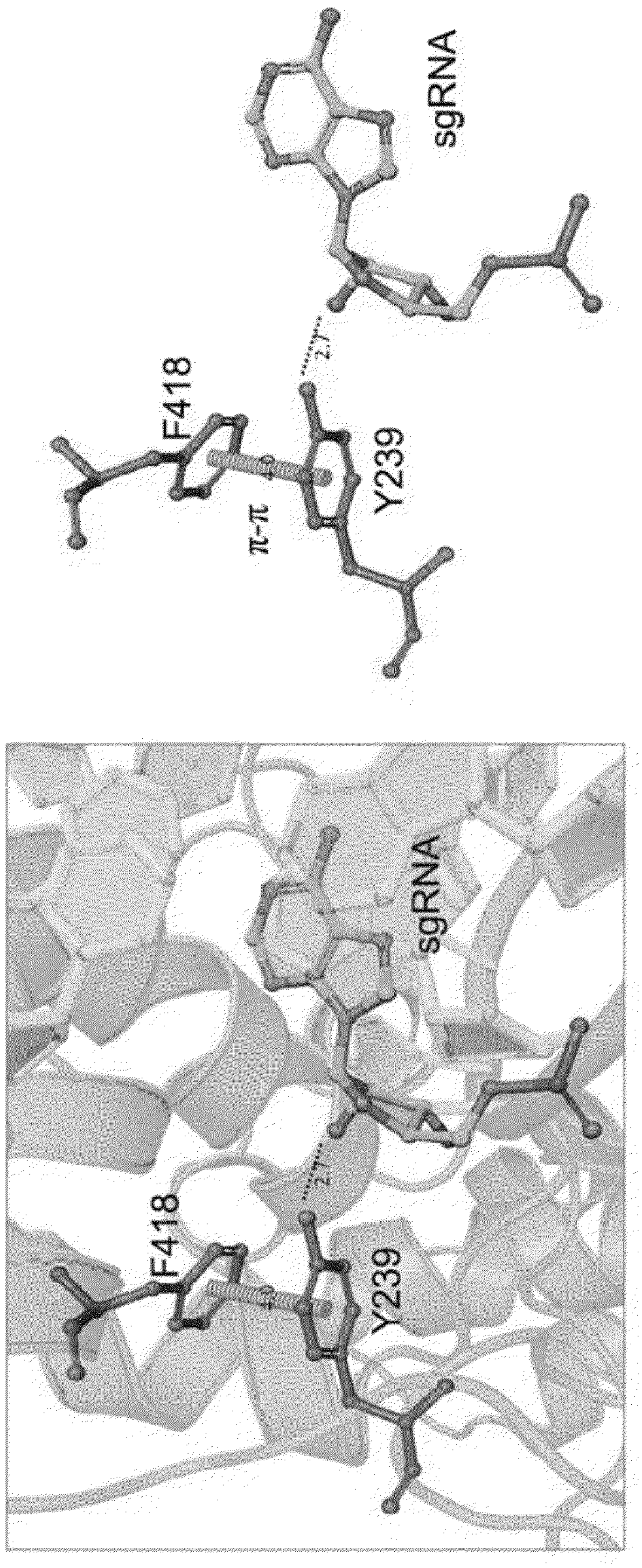


FIG. 10A

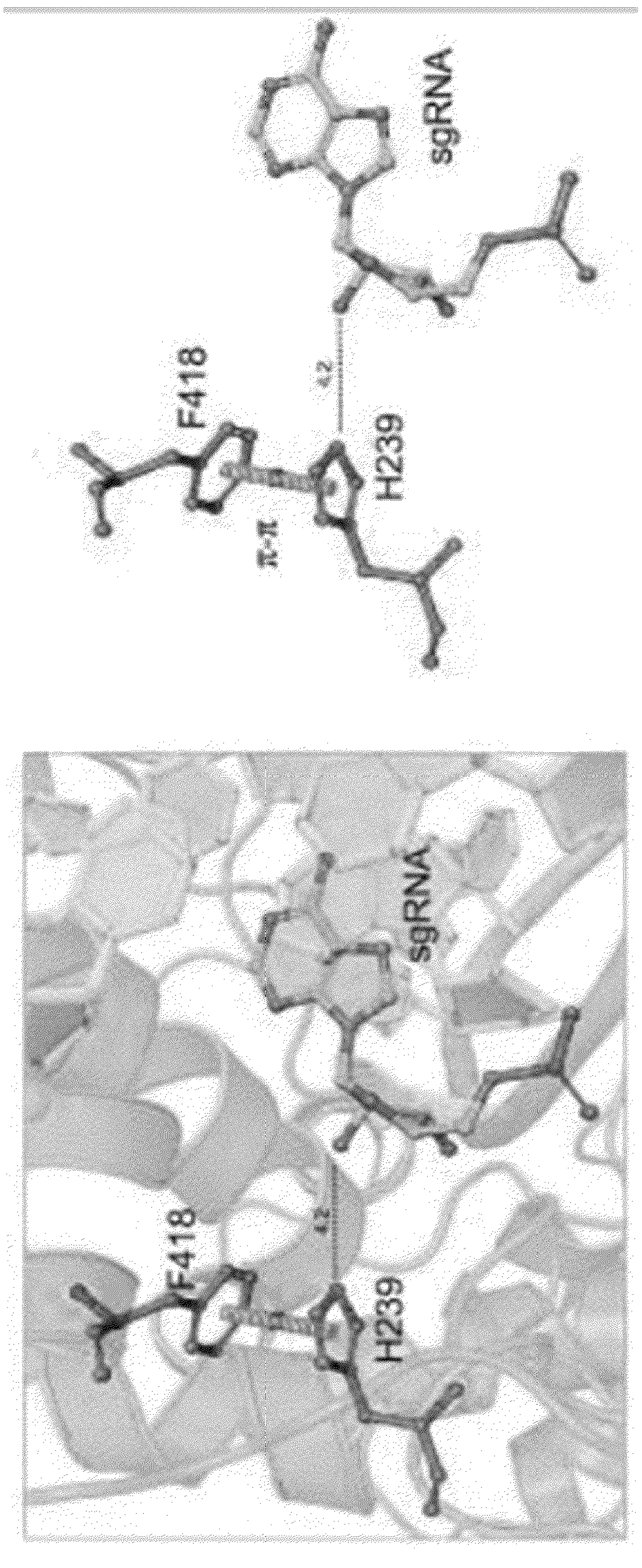


FIG. 10B

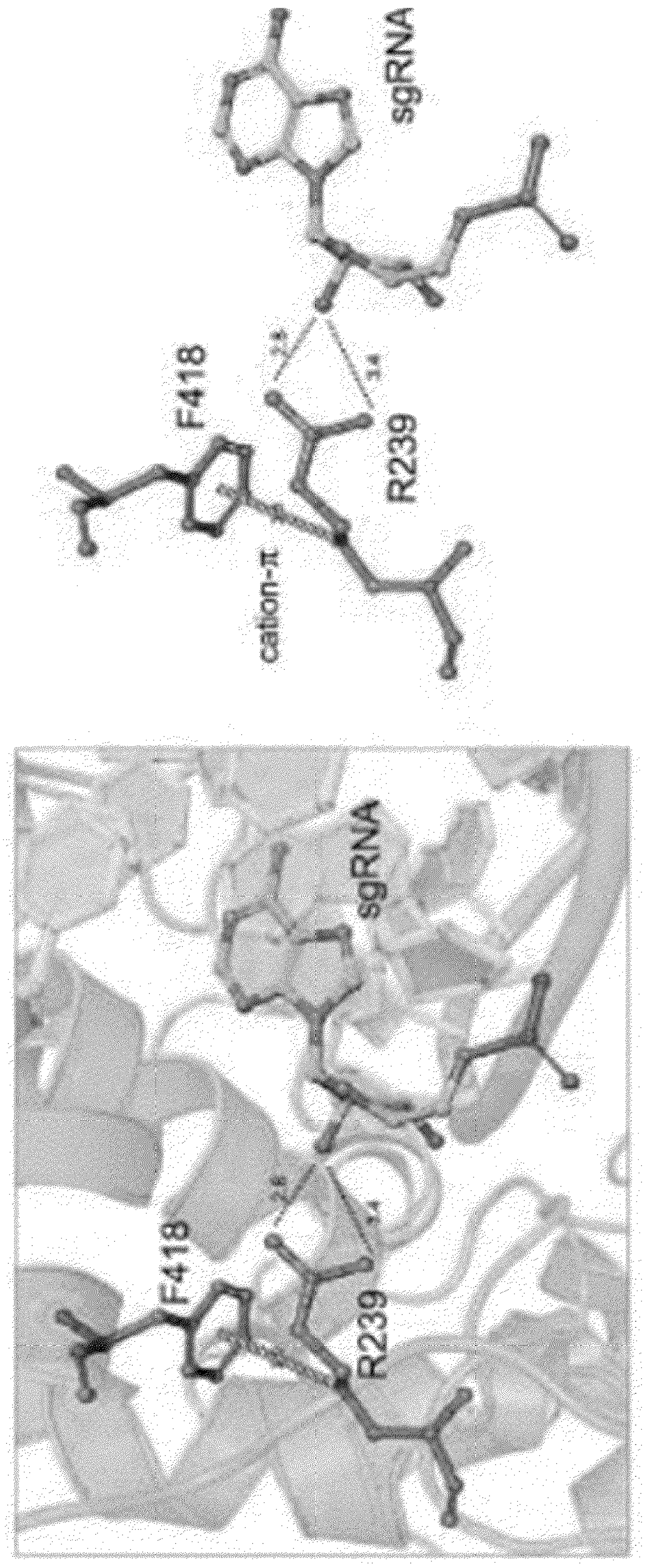


FIG. 10C

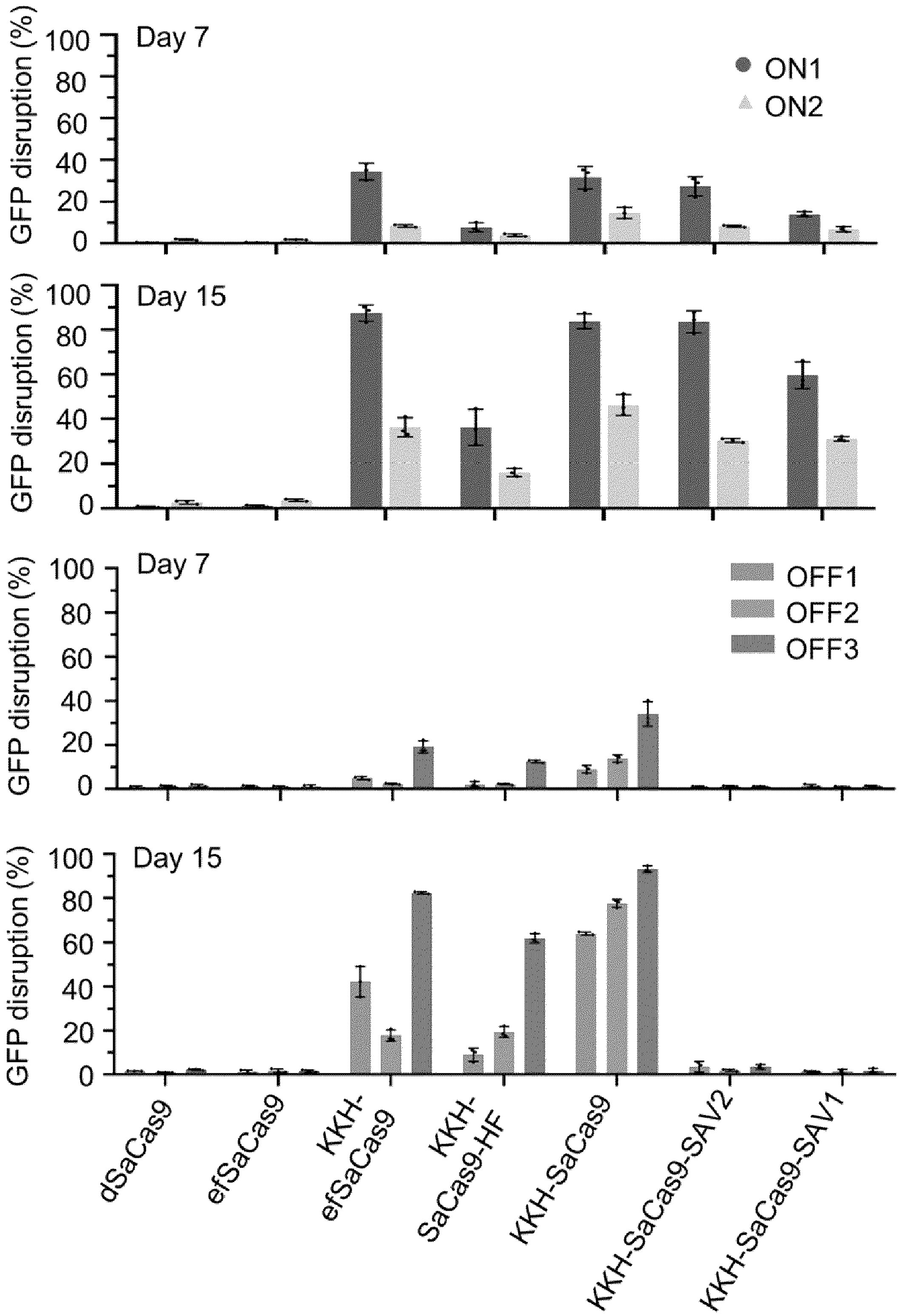

Day 7
ON1
ON2
GFP disruption (%)
100
80
60
40
20
0
Day 15
Day 7
OFF1
OFF2
OFF3
Day 15
dSaCas9
efSaCas9
KKH-efSaCas9
KKH-SaCas9-HF
KKH-SaCas9
KKH-SaCas9-SAV2
KKH-SaCas9-SAV1


FIG. 11

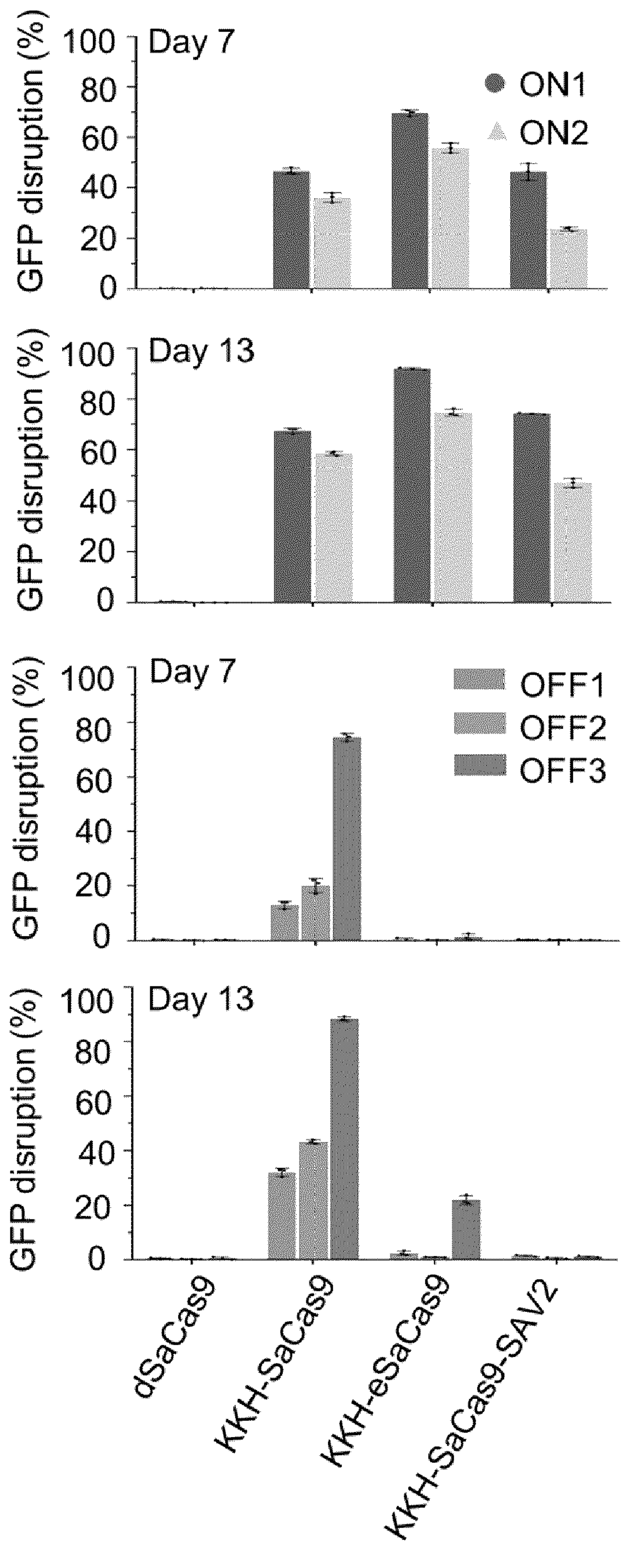
GFP disruption (%)
Day 7
ON1
ON2
Day 13
OFF1
OFF2
OFF3
0
20
40
60
80
100
dSaCas9
KKH-SaCas9
KKH-eSaCas9
KKH-SaCas9-SAV2

FIG. 12

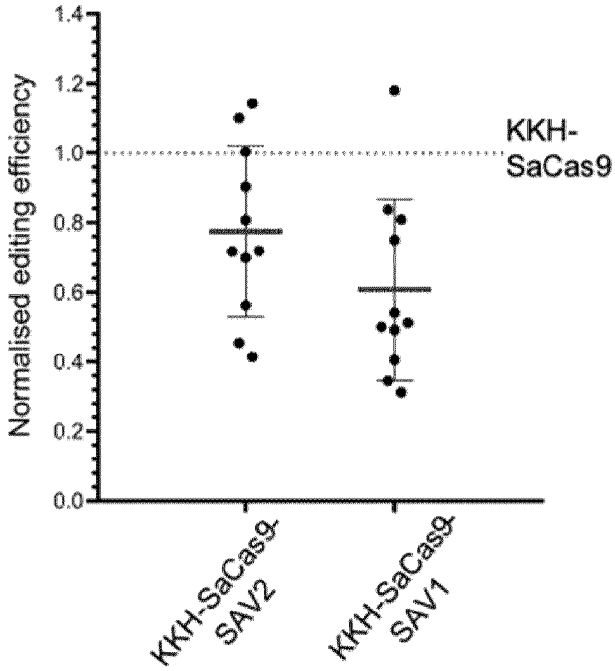


FIG. 13

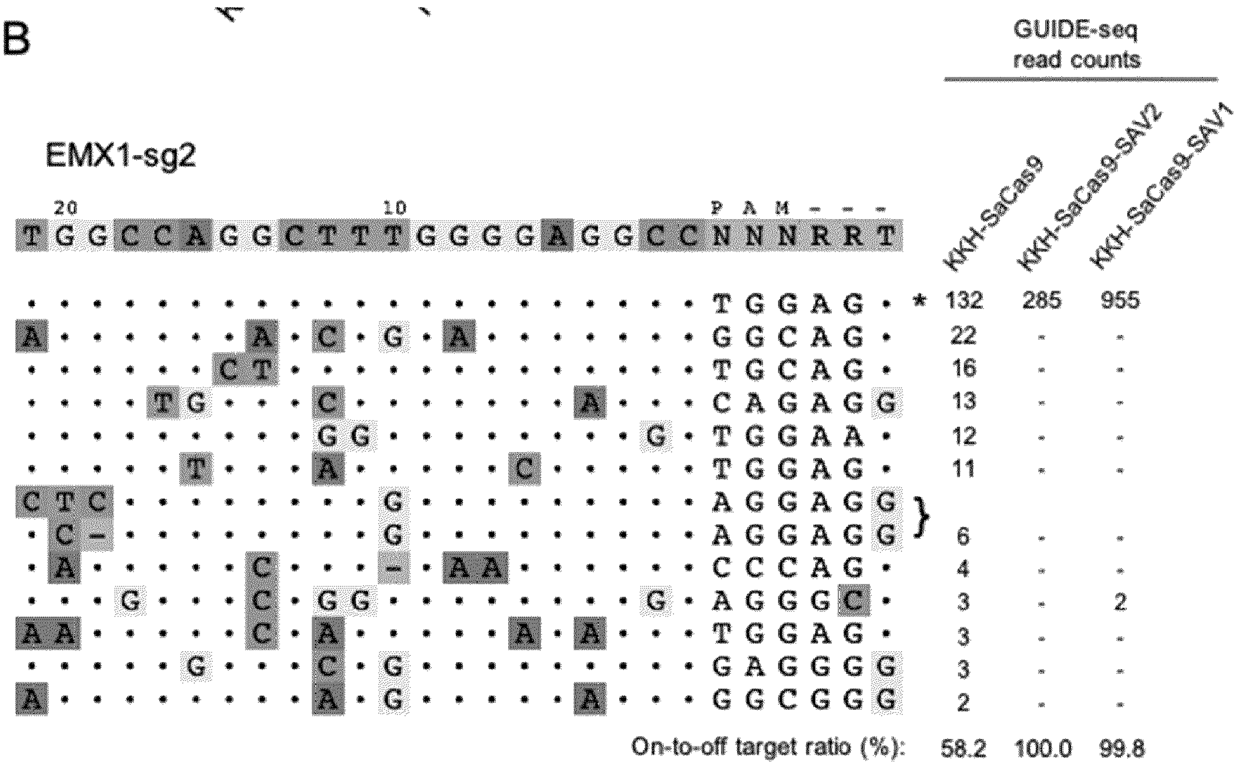


FIG. 14A

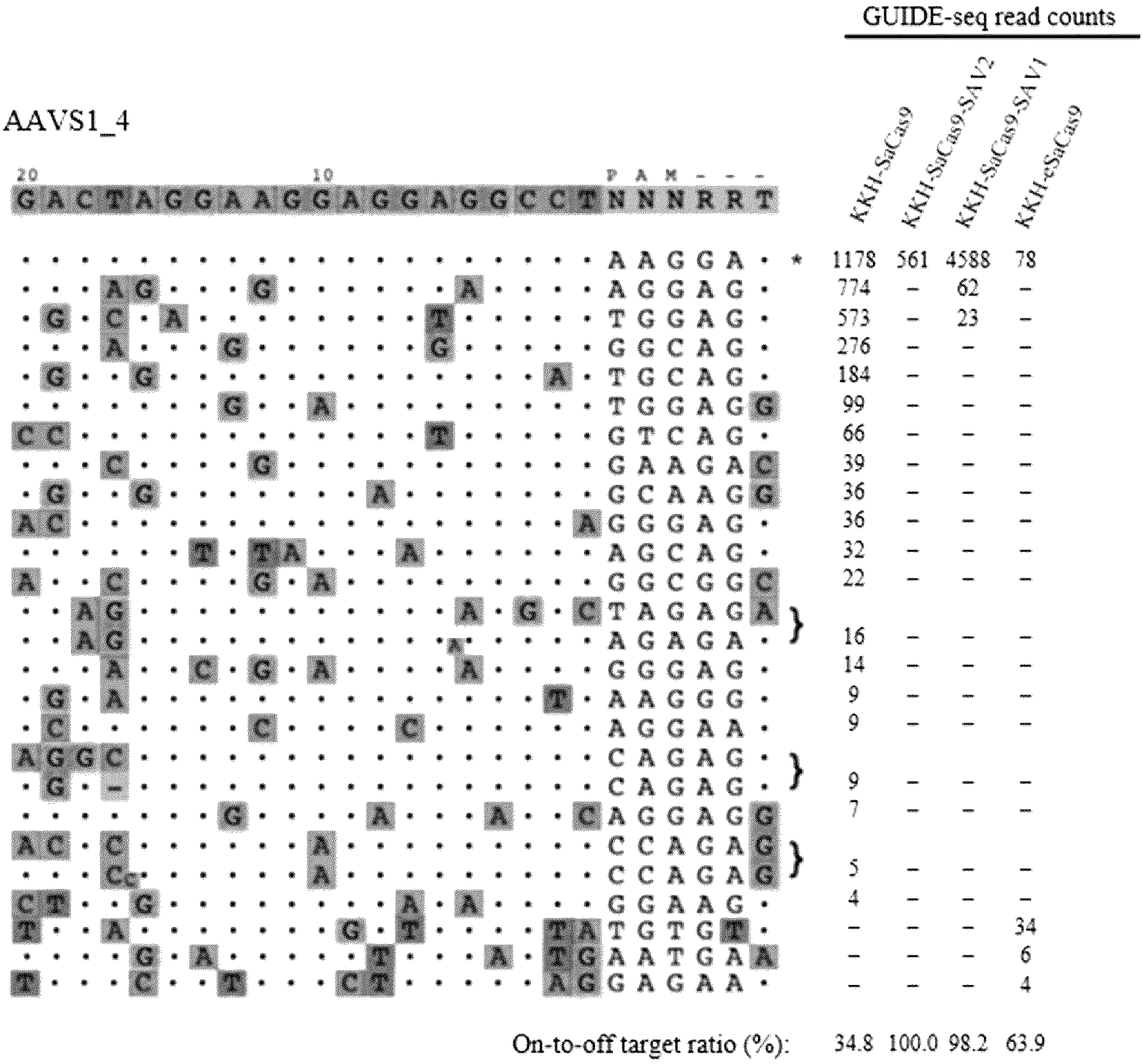
GUIDE-seq read counts
AAVS1_4
KKH-SaCas9
KKH-SaCas9-SAV2
KKH-SaCas9-SAV1
KKH-eSaCas9
20
10
PAM
GACTAGGAAGGAGGAGGCCTNNNRRT
1178 561 4588 78
774 – 62 –
573 – 23 –
276 – – –
184 – – –
99 – – –
66 – – –
39 – – –
36 – – –
36 – – –
32 – – –
22 – – –
16 – – –
14 – – –
9 – – –
9 – – –
9 – – –
7 – – –
5 – – –
4 – – –
– – – 34
– – – 6
– – – 4
On-to-off target ratio (%): 34.8 100.0 98.2 63.9

FIG. 14B

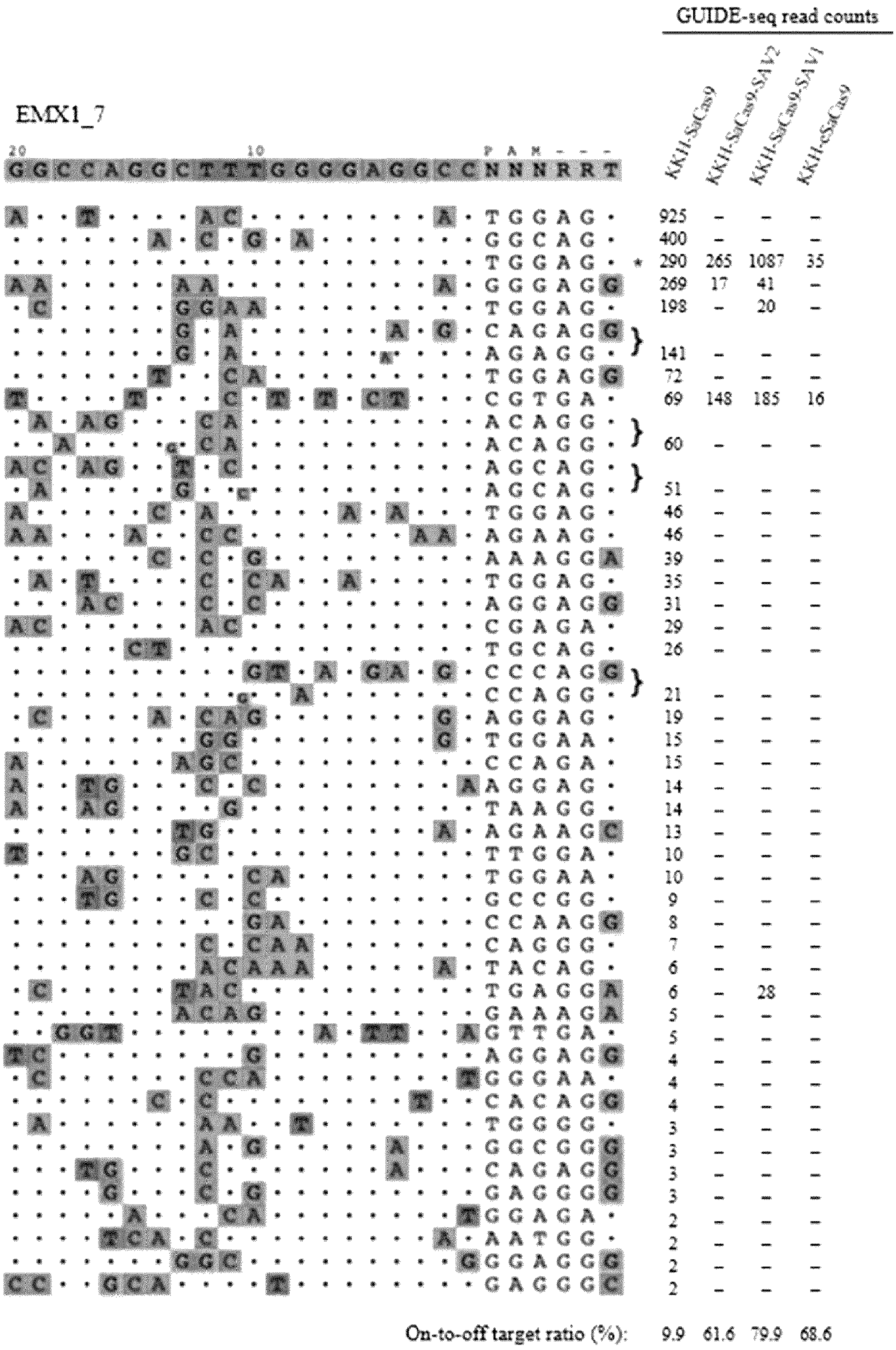
GUIDE-seq read counts
EMX1_7
KKH-SaCas9
KKH-SaCas9-SAV2
KKH-SaCas9-SAV1
KKH-eSaCas9
GGCCAGGCTTTGGGGAGGCCNNNRRT
On-to-off target ratio (%): 9.9 61.6 79.9 68.6

FIG. 14C

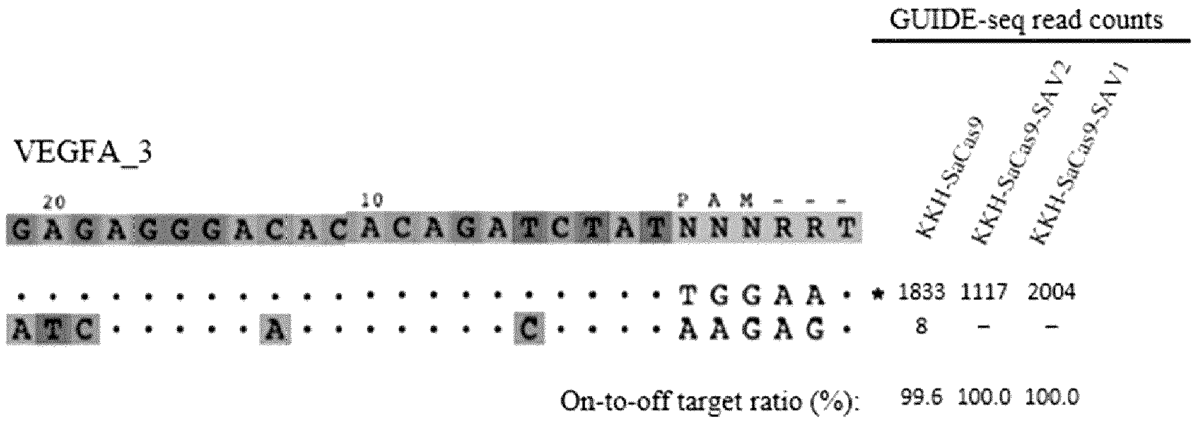


# FIG. 14D

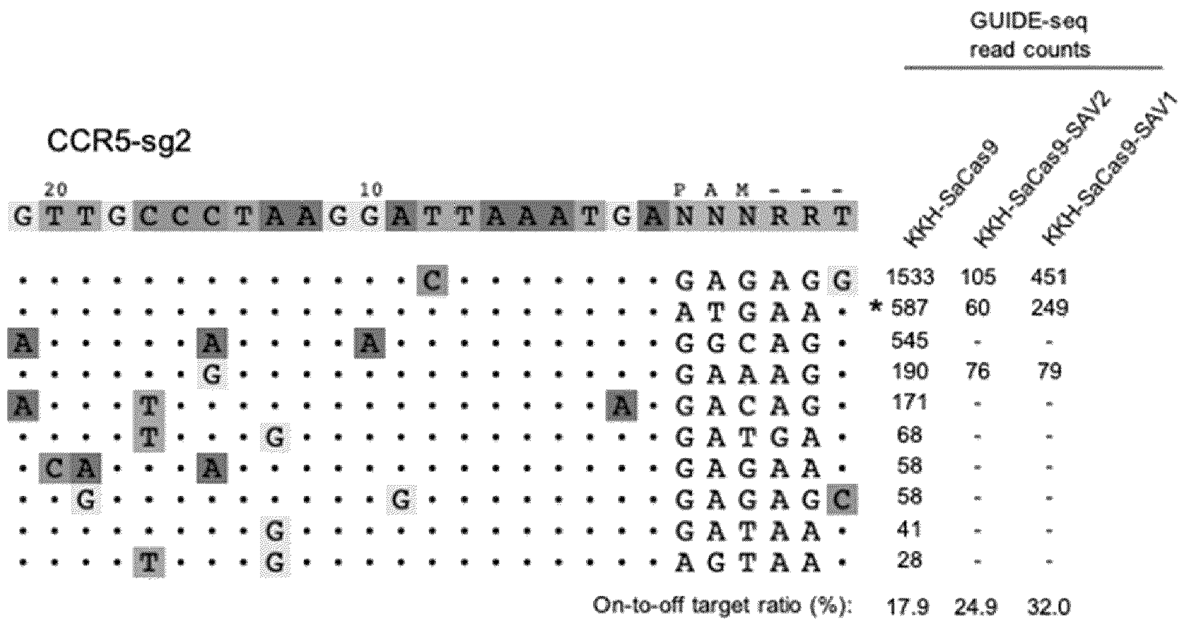


# FIG. 14E

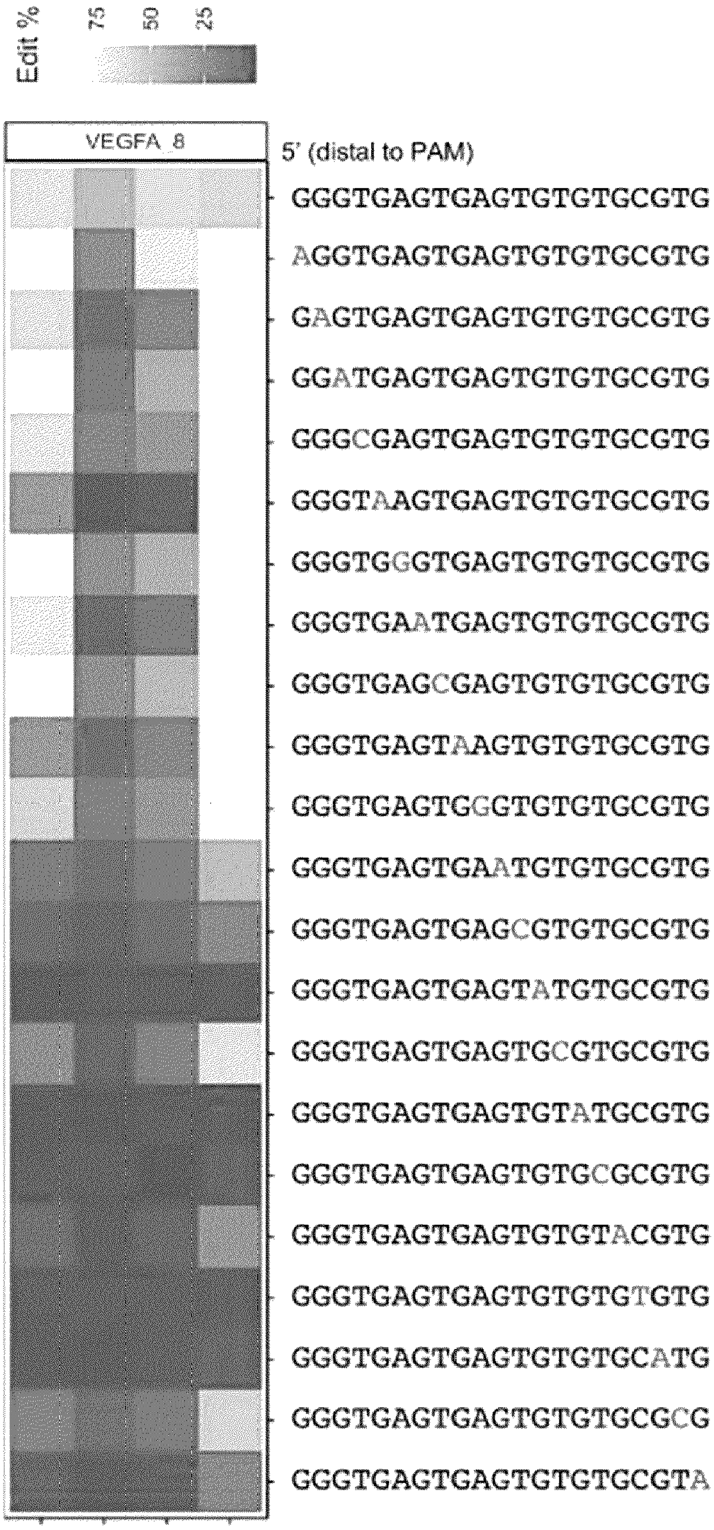
Edit %
75
50
25
VEGFA_8
5' (distal to PAM)
GGGTGAGTGAGTGTGTGCGTG
AGGTGAGTGAGTGTGTGCGTG
GAGTGAGTGAGTGTGTGCGTG
GGATGAGTGAGTGTGTGCGTG
GGGCGAGTGAGTGTGTGCGTG
GGGTAAGTGAGTGTGTGCGTG
GGGTGGGTGAGTGTGTGCGTG
GGGTGAATGAGTGTGTGCGTG
GGGTGAGCGAGTGTGTGCGTG
GGGTGAGTAAGTGTGTGCGTG
GGGTGAGTGGGTGTGTGCGTG
GGGTGAGTGAATGTGTGCGTG
GGGTGAGTGAGCGTGTGCGTG
GGGTGAGTGAGTATGTGCGTG
GGGTGAGTGAGTGCGTGCGTG
GGGTGAGTGAGTGTATGCGTG
GGGTGAGTGAGTGTGCGCGTG
GGGTGAGTGAGTGTGTACGTG
GGGTGAGTGAGTGTGTGTGTG
GGGTGAGTGAGTGTGTGCATG
GGGTGAGTGAGTGTGTGCGCG
GGGTGAGTGAGTGTGTGCGTA

FIG. 15A

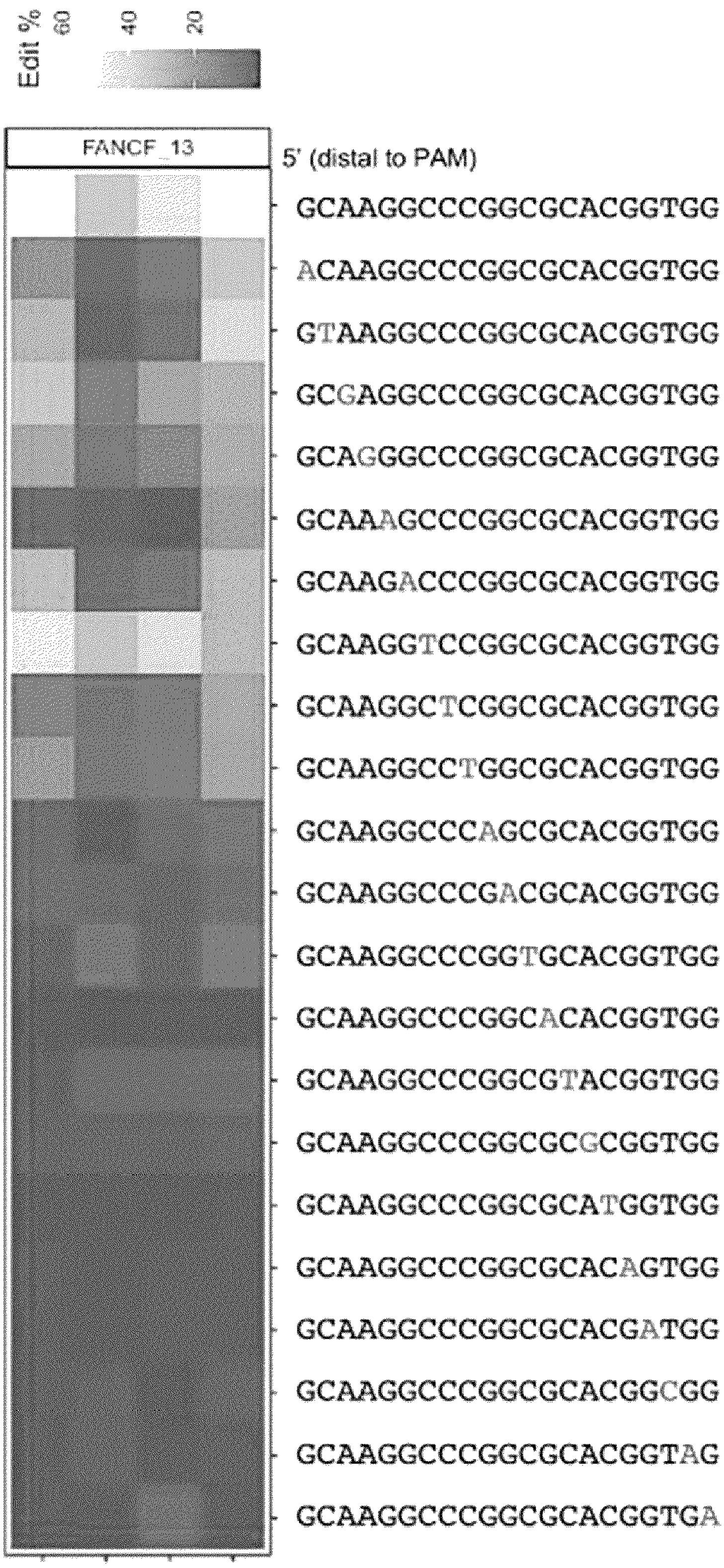

Edit %
60
40
20
FANCF_13
5' (distal to PAM)
GCAAGGCCCGGCGCACGGTGG
ACAAGGCCCGGCGCACGGTGG
GTAAGGCCCGGCGCACGGTGG
GCGAGGCCCGGCGCACGGTGG
GCAGGGCCCGGCGCACGGTGG
GCAAAGCCCGGCGCACGGTGG
GCAAGACCCGGCGCACGGTGG
GCAAGGTCCGGCGCACGGTGG
GCAAGGCTCGGCGCACGGTGG
GCAAGGCCTGGCGCACGGTGG
GCAAGGCCCAGCGCACGGTGG
GCAAGGCCCGACGCACGGTGG
GCAAGGCCCGGTGCACGGTGG
GCAAGGCCCGGCACACGGTGG
GCAAGGCCCGGCGTACGGTGG
GCAAGGCCCGGCGCGCGGTGG
GCAAGGCCCGGCGCATGGTGG
GCAAGGCCCGGCGCACAGTGG
GCAAGGCCCGGCGCACGATGG
GCAAGGCCCGGCGCACGGCGG
GCAAGGCCCGGCGCACGGTAG
GCAAGGCCCGGCGCACGGTGA


# FIG. 15B

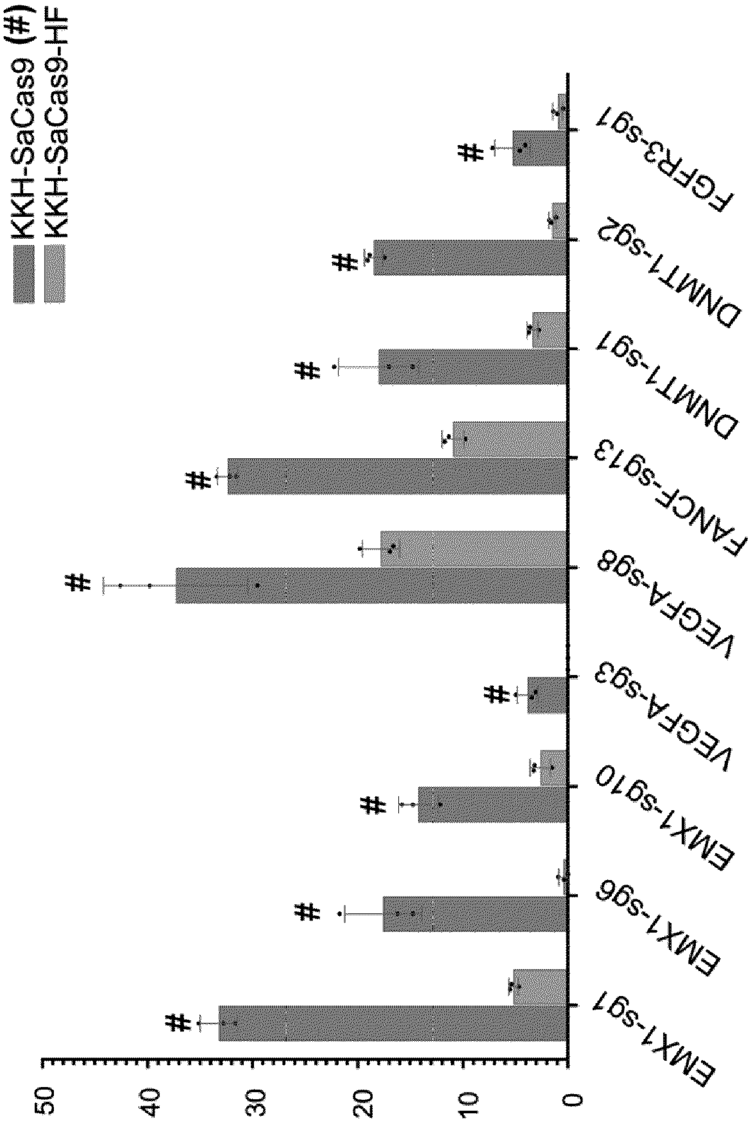
KKH-SaCas9 (#)
KKH-SaCas9-HF
Editing efficiency (%)
0
10
20
30
40
50
EMX1-sg1
EMX1-sg6
EMX1-sg10
VEGFA-sg3
VEGFA-sg8
FANCF-sg13
DNMT1-sg1
DNMT1-sg2
FGFR3-sg1
#

FIG. 16

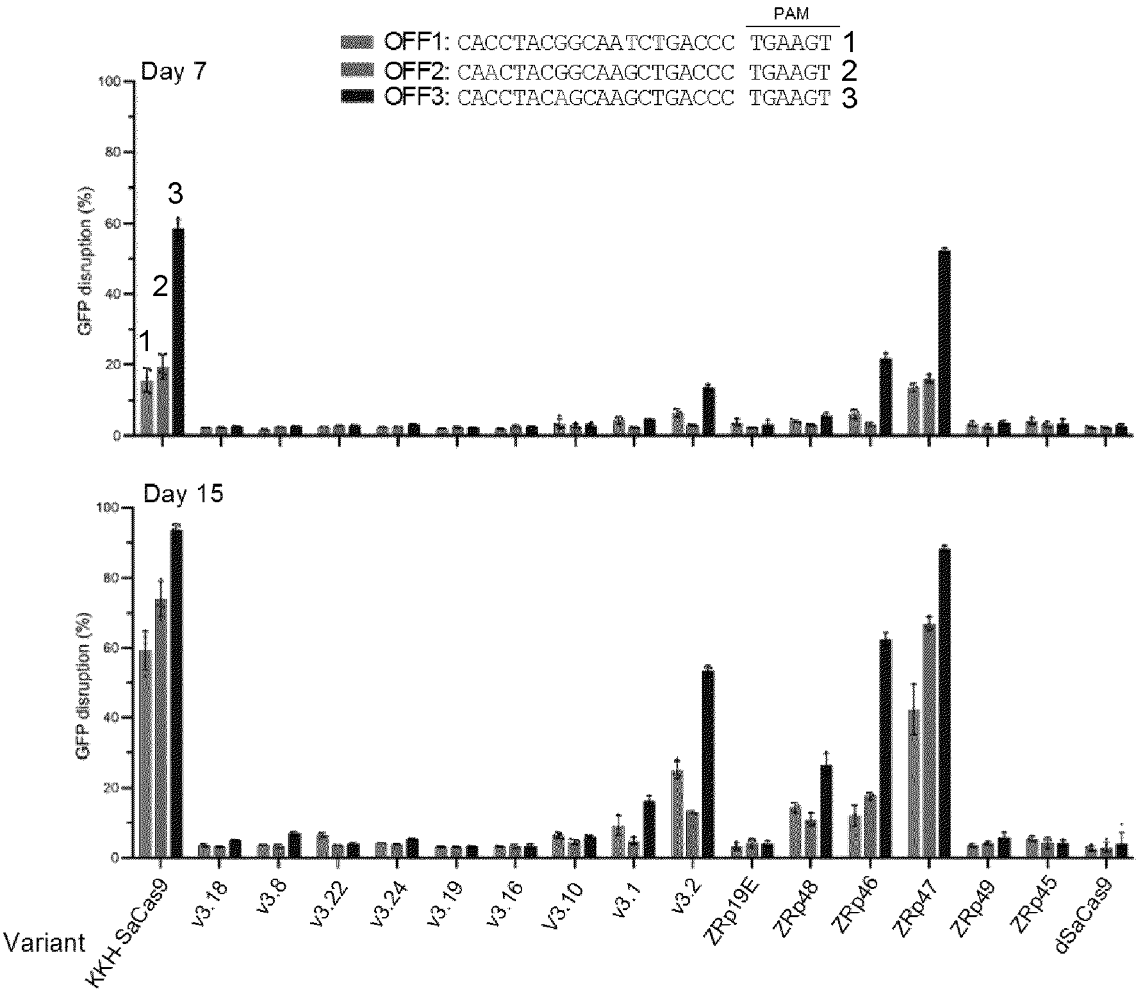


| Variant | KKH-SaCas9 | v3.18 | v3.8 | v3.22 | v3.24 | v3.19 | v3.16 | V3.10 | v3.1 | v3.2 | ZRp19E | ZRp48 | ZRp46 | ZRp47 | ZRp49 | ZRp45 | dSaCas9 |
|---|---|---|---|---|---|---|---|---|---|---|---|---|---|---|---|---|---|
| T238 | - | A | - | - | - | - | - | - | - | - | - | - | - | - | - | - | - |
| Y239 | - | H | H | H | H | H | H | H | - | - | H | H | - | - | H | - | - |
| R245 | - | - | - | - | - | - | - | - | A | - | A | - | A | - | A | A | - |
| N260 | - | - | - | - | - | - | - | - | - | - | - | - | - | - | - | - | - |
| T392 | - | A | - | - | - | - | - | - | - | - | - | - | - | - | - | - | - |
| N394 | - | T | T | - | T | A | - | - | - | - | - | - | - | - | - | - | - |
| N413 | - | - | - | - | - | - | - | - | - | - | - | A | A | A | A | A | - |
| Q414 | - | - | - | R | - | R | - | - | - | - | - | - | - | - | - | - | - |
| N419 | - | G | S | D | D | D | D | D | D | D | D | A | A | A | A | A | - |
| R499 | - | - | A | - | - | - | A | - | - | - | - | - | - | - | - | - | - |
| Q500 | - | - | A | - | - | - | A | - | - | - | - | - | - | - | - | - | - |
| G616 | - | - | - | - | - | - | - | - | - | - | - | - | - | - | - | - | - |
| Y651 | - | - | - | H | - | - | H | - | - | - | - | - | - | - | - | - | - |
| R654 | - | A | A | - | A | A | - | A | A | A | A | A | A | A | A | A | - |
| G655 | - | A | A | - | A | A | - | A | A | A | A | - | - | - | - | - | - |
| E782 | K | K | K | K | K | K | K | K | K | K | K | K | K | K | K | - | - |
| N968 | K | K | K | K | K | K | K | K | K | K | K | K | K | K | K | - | - |
| R1015 | H | H | H | H | H | H | H | H | H | H | H | H | H | H | H | - | - |

FIG. 17

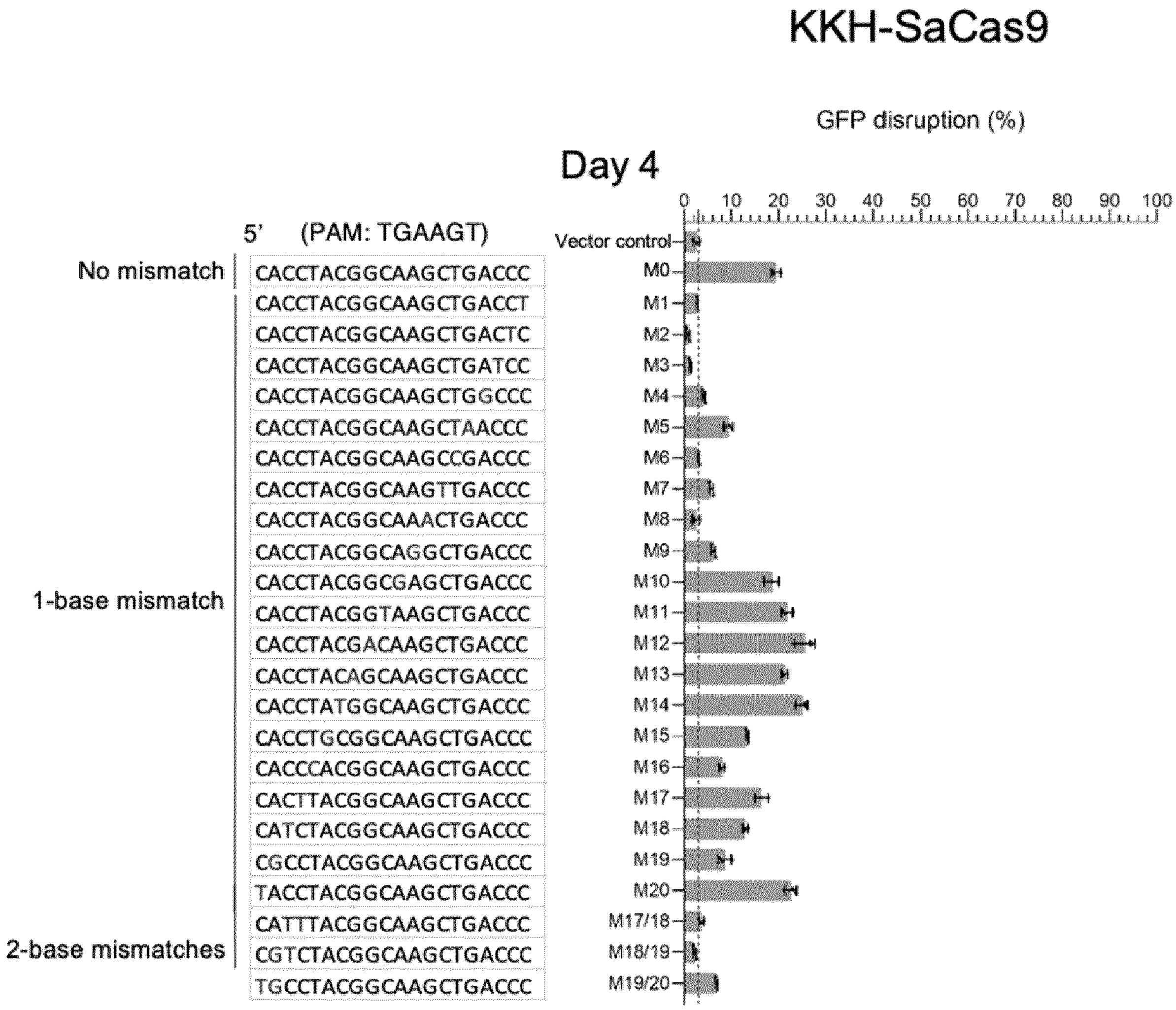
KKH-SaCas9
GFP disruption (%)
Day 4
0 10 20 30 40 50 60 70 80 90 100
5' (PAM: TGAAGT)
No mismatch
1-base mismatch
2-base mismatches
CACCTACGGCAAGCTGACCC
CACCTACGGCAAGCTGACCT
CACCTACGGCAAGCTGACTC
CACCTACGGCAAGCTGATCC
CACCTACGGCAAGCTGGCCC
CACCTACGGCAAGCTAACCC
CACCTACGGCAAGCCGACCC
CACCTACGGCAAGTTGACCC
CACCTACGGCAAACTGACCC
CACCTACGGCAGGCTGACCC
CACCTACGGCGAGCTGACCC
CACCTACGGTAAGCTGACCC
CACCTACGACAAGCTGACCC
CACCTACAGCAAGCTGACCC
CACCTATGGCAAGCTGACCC
CACCTGCGGCAAGCTGACCC
CACCCACGGCAAGCTGACCC
CACTTACGGCAAGCTGACCC
CATCTACGGCAAGCTGACCC
CGCCTACGGCAAGCTGACCC
TACCTACGGCAAGCTGACCC
CATTTACGGCAAGCTGACCC
CGTCTACGGCAAGCTGACCC
TGCCTACGGCAAGCTGACCC
Vector control
M0
M1
M2
M3
M4
M5
M6
M7
M8
M9
M10
M11
M12
M13
M14
M15
M16
M17
M18
M19
M20
M17/18
M18/19
M19/20

# FIG. 18A

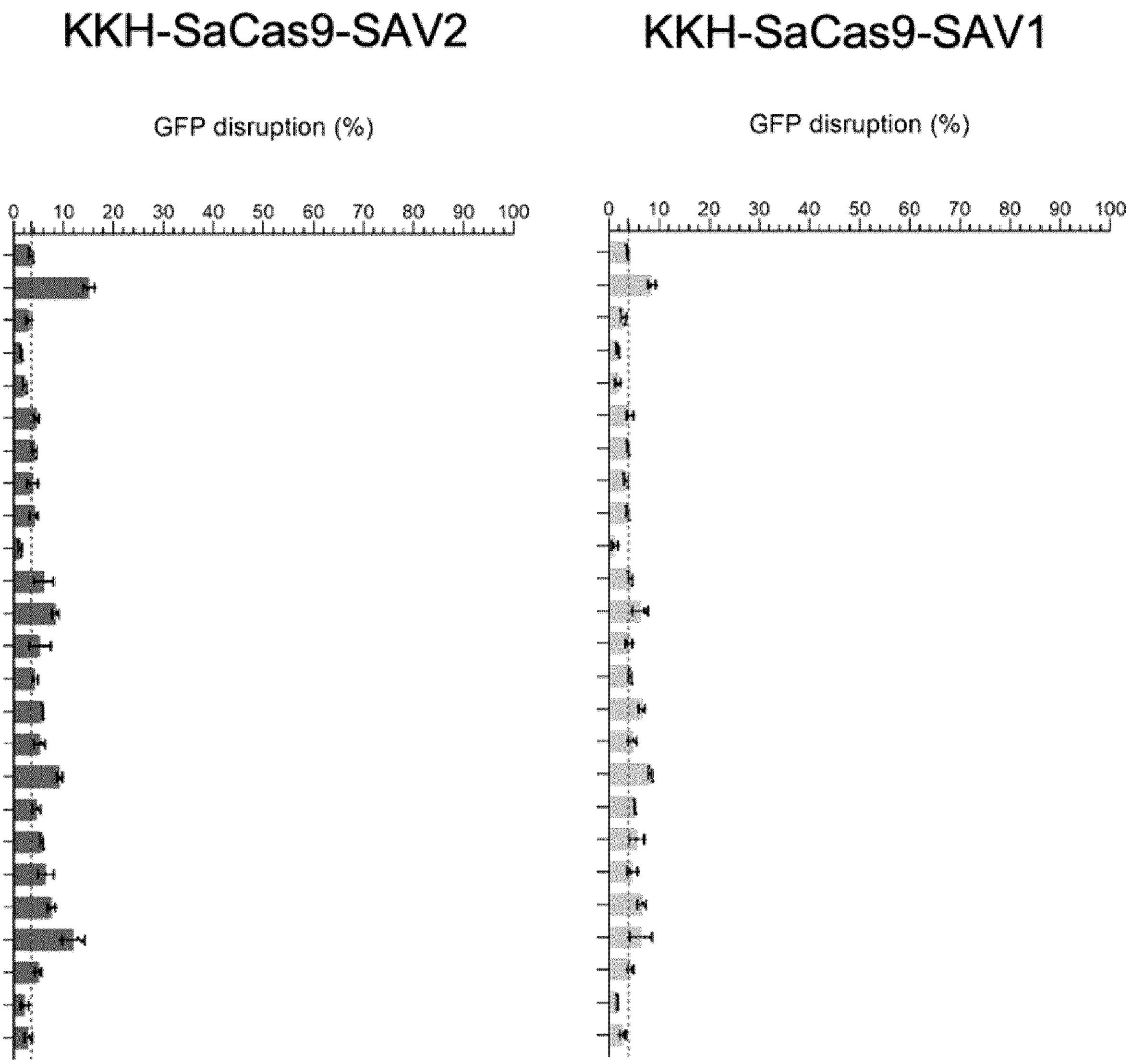
KKH-SaCas9-SAV2
GFP disruption (%)
0 10 20 30 40 50 60 70 80 90 100
KKH-SaCas9-SAV1
GFP disruption (%)
0 10 20 30 40 50 60 70 80 90 100

FIG. 18B

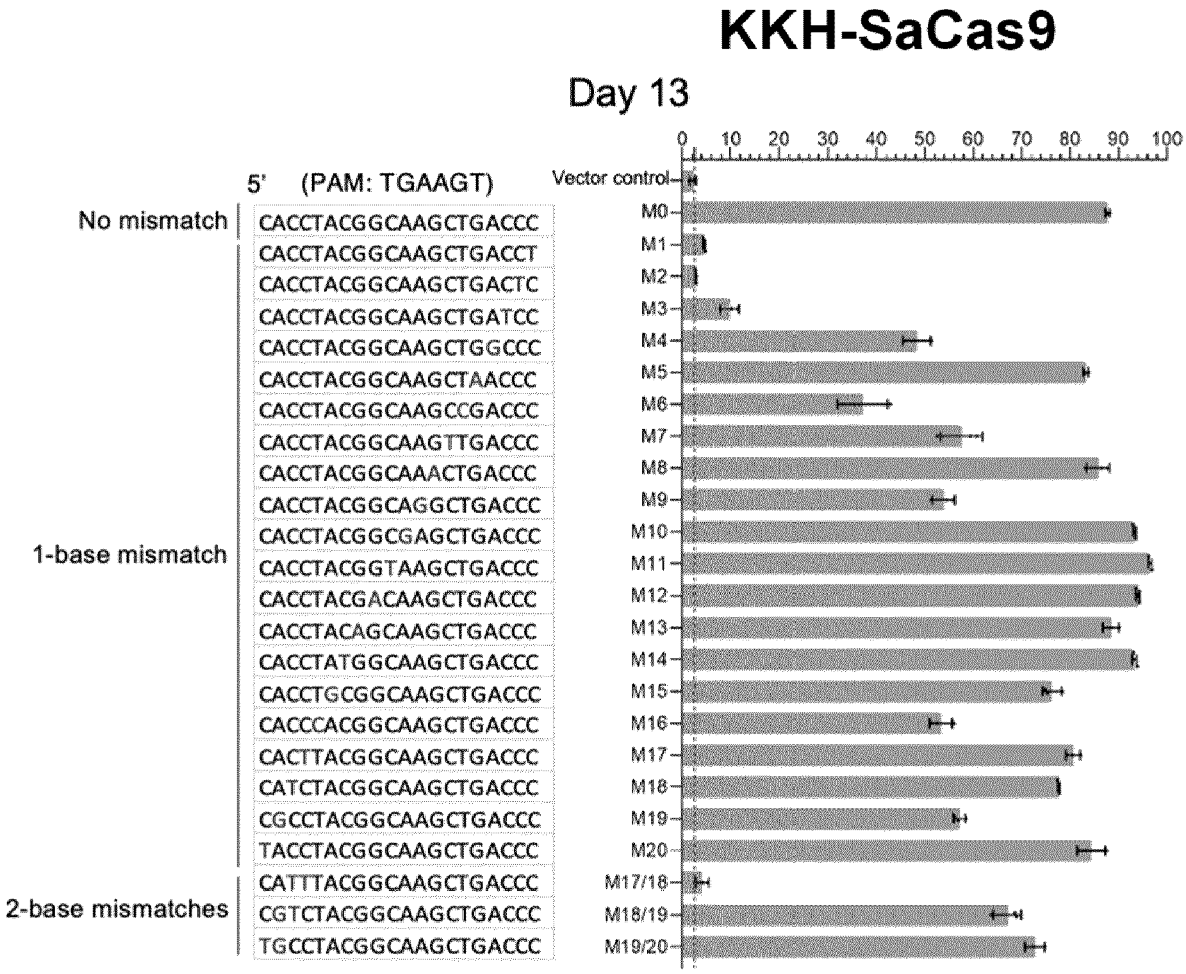
KKH-SaCas9
Day 13
5'
(PAM: TGAAGT)
No mismatch
1-base mismatch
2-base mismatches
CACCTACGGCAAGCTGACCC
CACCTACGGCAAGCTGACCT
CACCTACGGCAAGCTGACTC
CACCTACGGCAAGCTGATCC
CACCTACGGCAAGCTGGCCC
CACCTACGGCAAGCTAACCC
CACCTACGGCAAGCCGACCC
CACCTACGGCAAGTTGACCC
CACCTACGGCAAACTGACCC
CACCTACGGCAGGCTGACCC
CACCTACGGCGAGCTGACCC
CACCTACGGTAAGCTGACCC
CACCTACGACAAGCTGACCC
CACCTACAGCAAGCTGACCC
CACCTATGGCAAGCTGACCC
CACCTGCGGCAAGCTGACCC
CACCCACGGCAAGCTGACCC
CACTTACGGCAAGCTGACCC
CATCTACGGCAAGCTGACCC
CGCCTACGGCAAGCTGACCC
TACCTACGGCAAGCTGACCC
CATTTACGGCAAGCTGACCC
CGTCTACGGCAAGCTGACCC
TGCCTACGGCAAGCTGACCC
Vector control
M0
M1
M2
M3
M4
M5
M6
M7
M8
M9
M10
M11
M12
M13
M14
M15
M16
M17
M18
M19
M20
M17/18
M18/19
M19/20
0
10
20
30
40
50
60
70
80
90
100

FIG. 18C

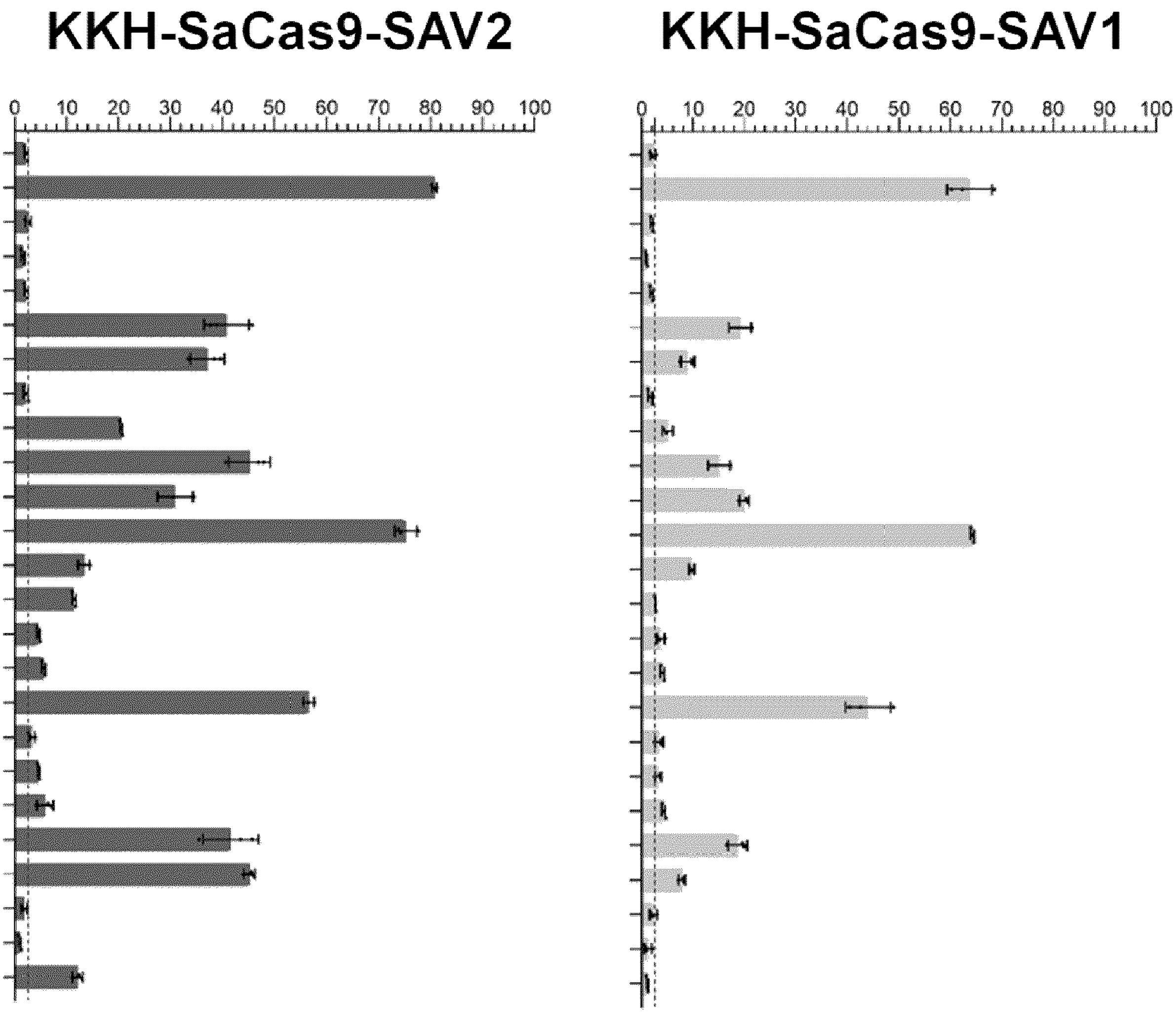
KKH-SaCas9-SAV2
0
10
20
30
40
50
60
70
80
90
100
KKH-SaCas9-SAV1
0
10
20
30
40
50
60
70
80
90
100

FIG. 18D

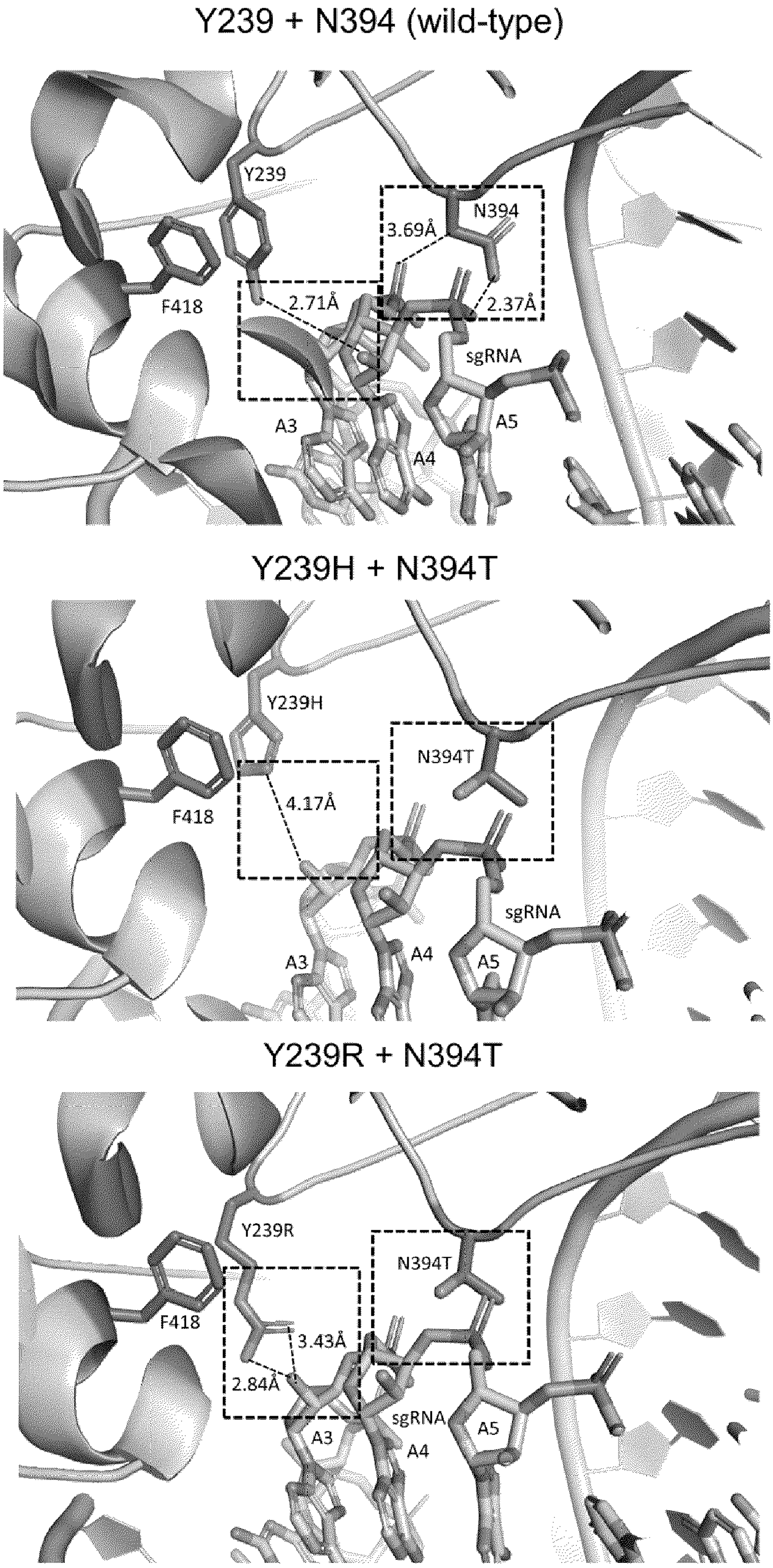
Y239 + N394 (wild-type)
Y239
N394
3.69Å
F418
2.71Å
2.37Å
sgRNA
A3
A5
A4
Y239H + N394T
Y239H
N394T
F418
4.17Å
sgRNA
A3
A4
A5
Y239R + N394T
Y239R
N394T
F418
3.43Å
2.84Å
sgRNA
A3
A5
A4

# FIG. 19

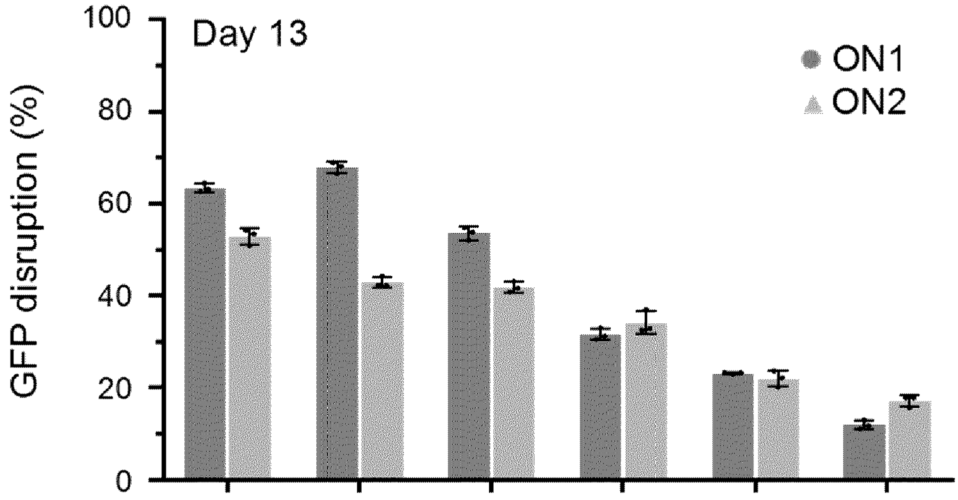


FIG. 20A

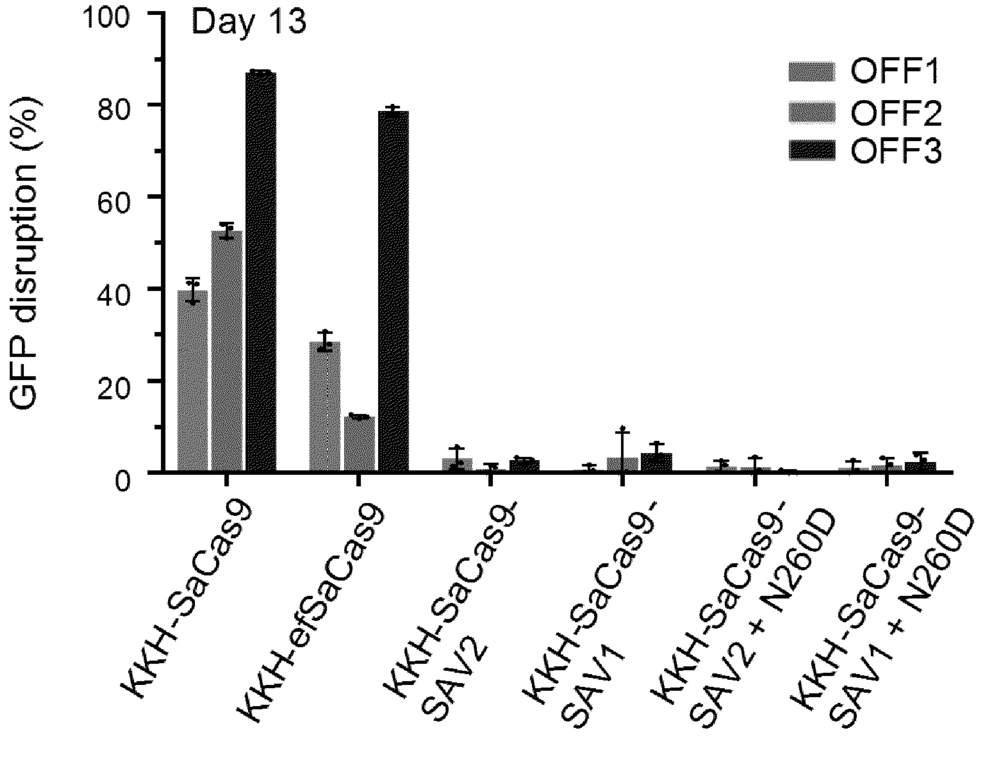


FIG. 20B

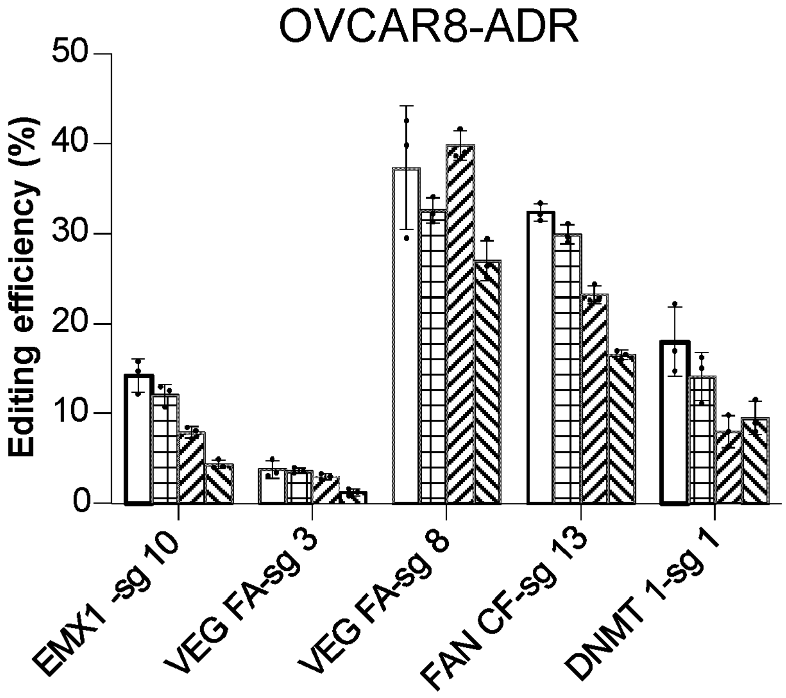


**FIG. 21A**

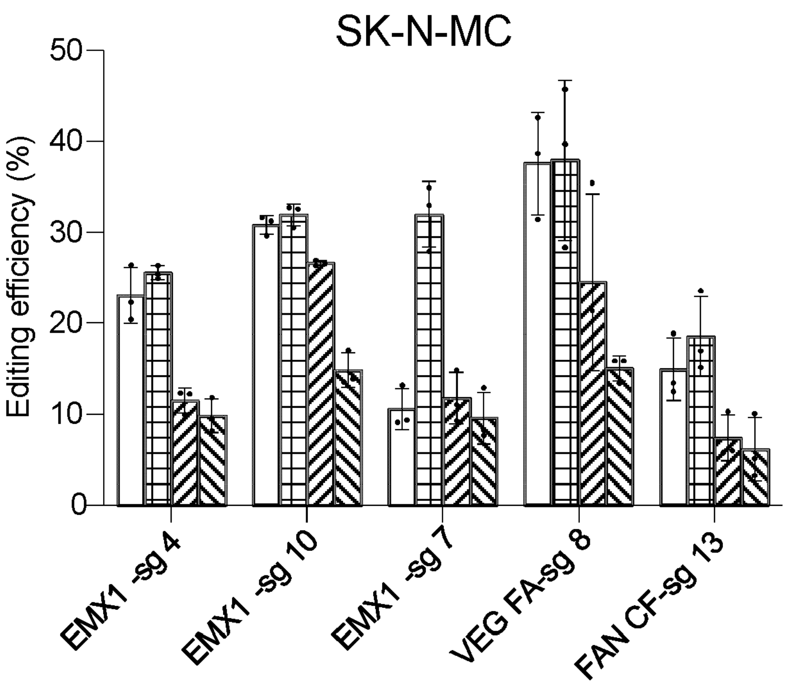


**FIG. 21B**

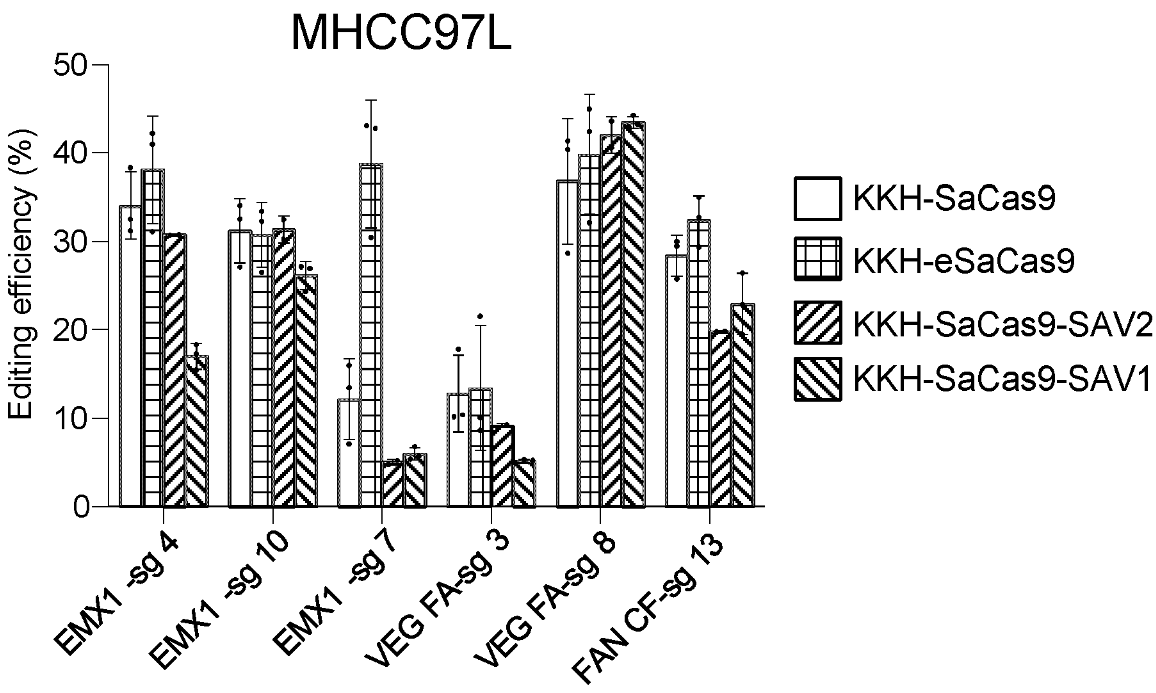


FIG. 21C

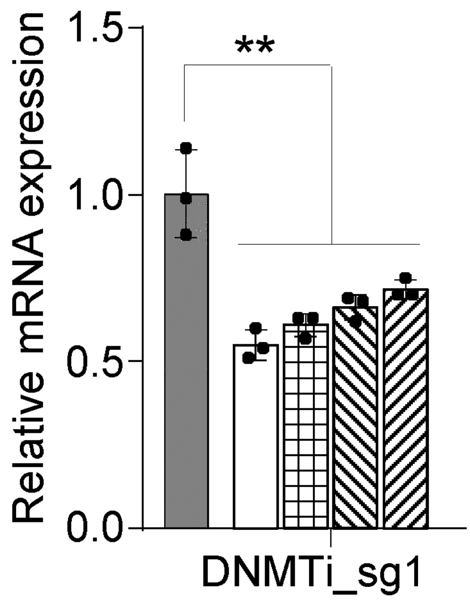


FIG. 22A

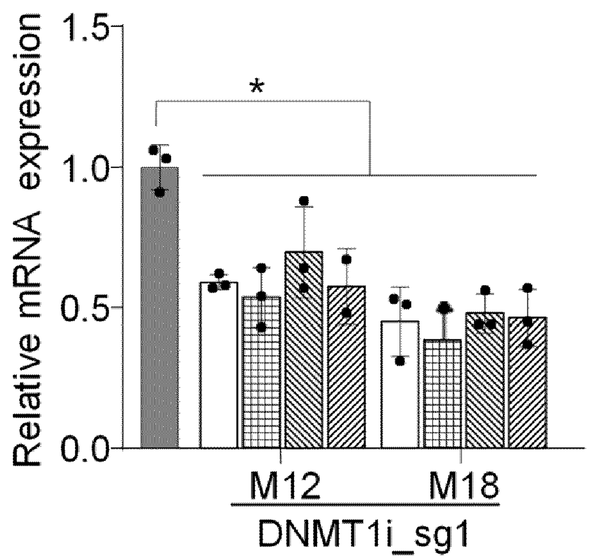


FIG. 22B

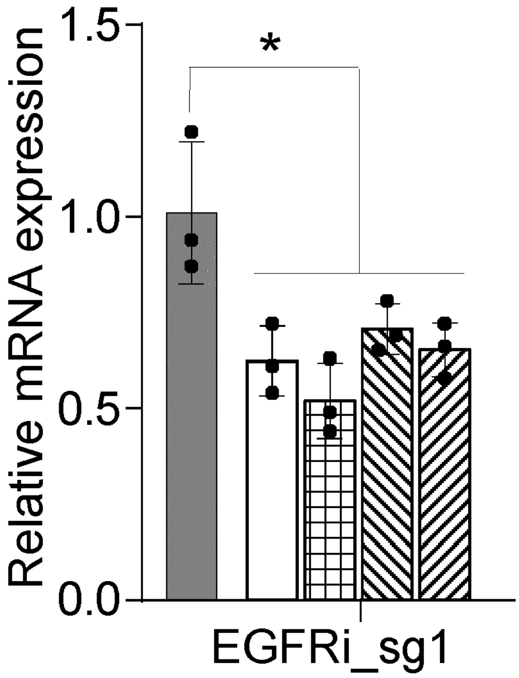


FIG. 22C

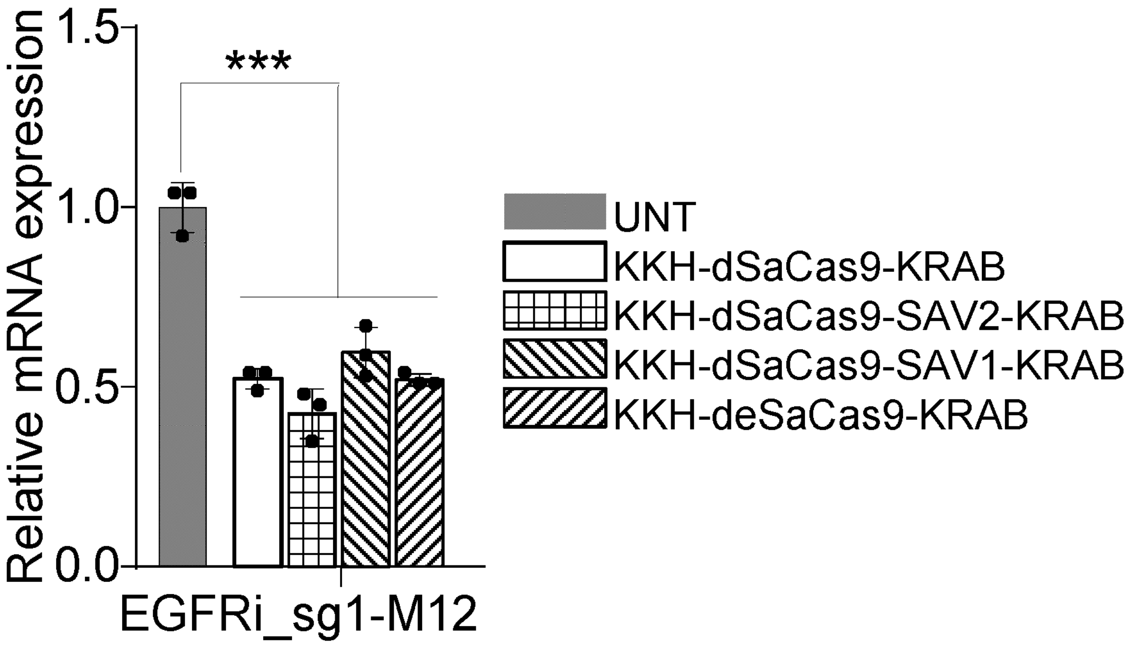


FIG. 22D

Europäisches Patentamt
European Patent Office
Office européen des brevets

# EUROPEAN SEARCH REPORT

Application Number
EP 22 21 4015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YU XIN ET AL: "Selection of novel affinity-matured human chondroitin sulfate proteoglycan 4 antibody fragments by yeast display", PROTEIN ENGINEERING, DESIGN AND SELECTION, vol. 30, no. 9, 28 July 2017 (2017-07-28), pages 639-647, XP093041442, GB ISSN: 1741-0126, DOI: 10.1093/protein/gzx038 * the whole document * ----- | 1,9 | INV. C12N9/22 |
| A,D | YUANYAN TAN ET AL: "Rationally engineered Staphylococcus aureus Cas9 nucleases with high genome-wide specificity", PROCEEDINGS OF THE NATIONAL ACADEMY OF SCIENCES, vol. 116, no. 42, 30 September 2019 (2019-09-30), pages 20969-20976, XP055755937, ISSN: 0027-8424, DOI: 10.1073/pnas.1906843116 * the whole document * ----- | 1-15 | TECHNICAL FIELDS SEARCHED (IPC) C12N |
| A,D | SLAYMAKER IAN M. ET AL: "Rationally engineered Cas9 nucleases with improved specificity", SCIENCE, vol. 351, no. 6268, 1 December 2015 (2015-12-01), pages 84-88, XP093017516, US ISSN: 0036-8075, DOI: 10.1126/science.aac8608 Retrieved from the Internet: URL:https://www.science.org/doi/10.1126/science.aad5227> * the whole document * -/-- | 1-15 | |

The present search report has been drawn up for all claims

1

| Place of search | Date of completion of the search | Examiner |
|---|---|---|
| Munich | 26 April 2023 | Westphal-Daniel, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 21 4015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & SLAYMAKER IAN M. ET AL: "Supplementary material - Rationally engineered Cas9 nucleases with improved specificity", SCIENCE, vol. 351, no. 6268, 1 January 2016 (2016-01-01), pages 84-88, XP093041934, US ISSN: 0036-8075, DOI: 10.1126/science.aad5227 ----- | | |
| A,D | HIROSHI NISHIMASU ET AL: "Crystal Structure of Staphylococcus aureus Cas9", CELL, vol. 162, no. 5, 1 August 2015 (2015-08-01), pages 1113-1126, XP055304450, Amsterdam NL ISSN: 0092-8674, DOI: 10.1016/j.cell.2015.08.007 * the whole document * ----- | 1-15 | |
| | | | TECHNICAL FIELDS SEARCHED (IPC) |
| A | US 2017/081650 A1 (JOUNG J KEITH [US] ET AL) 23 March 2017 (2017-03-23) * the whole document * ----- | 1-15 | |
| A | WO 2017/040348 A1 (MASSACHUSETTS GEN HOSPITAL [US]) 9 March 2017 (2017-03-09) * the whole document * ----- | 1-15 | |
| A | US 2021/355465 A1 (JOUNG J KEITH [US] ET AL) 18 November 2021 (2021-11-18) * the whole document * ----- | 1-15 | |

-/--

The present search report has been drawn up for all claims

1

| Place of search | Date of completion of the search | Examiner |
|---|---|---|
| Munich | 26 April 2023 | Westphal-Daniel, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 22 21 4015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BENJAMIN P. KLEINSTIVER ET AL: "Broadening the targeting range of Staphylococcus aureus CRISPR-Cas9 by modifying PAM recognition- Supplementary Text and Figures", NATURE, vol. 529, no. 7587, 1 January 2016 (2016-01-01), pages 490-495, XP055650074, London ISSN: 0028-0836, DOI: 10.1038/nature16526 * the whole document * | 1-15 | |
| A,D | & KLEINSTIVER BENJAMIN P ET AL: "Broadening the targeting range of Staphylococcus aureus CRISPR-Cas9 by modifying PAM recognition- Supplementary Text and Figures", NATURE BIOTECHNOLOGY, vol. 33, no. 12, 2 November 2015 (2015-11-02), pages 1293-1298, XP055832821, New York ISSN: 1087-0156, DOI: 10.1038/nbt.3404 Retrieved from the Internet: URL:http://www.nature.com/articles/nbt.3404> * the whole document * ----- | 1-15 | TECHNICAL FIELDS SEARCHED (IPC) |

-/--

The present search report has been drawn up for all claims

| Place of search | Date of completion of the search | Examiner |
|---|---|---|
| Munich | 26 April 2023 | Westphal-Daniel, K |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 21 4015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHOI GIGI C ET AL: "Combinatorial mutagenesis en masse optimizes the genome editing activities of SpCas9", NATURE METHODS, NATURE PUBLISHING GROUP US, NEW YORK, vol. 16, no. 8, 15 July 2019 (2019-07-15), pages 722-730, XP036847274, ISSN: 1548-7091, DOI: 10.1038/S41592-019-0473-0 [retrieved on 2019-07-15] * the whole document * ----- | 1-15 | |
| T | YUEN CHAYA T L ET AL: "High-fidelity KKH variant of Staphylococcus aureus Cas9 nucleases with improved base mismatch discrimination", NUCLEIC ACIDS RESEARCH, vol. 50, no. 3, 22 February 2022 (2022-02-22), pages 1650-1660, XP093041402, GB ISSN: 0305-1048, DOI: 10.1093/nar/gkab1291 Retrieved from the Internet: URL:https://www.ncbi.nlm.nih.gov/pmc/articles/PMC8860571/pdf/gkab1291.pdf> * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 26 April 2023 | Westphal-Daniel, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 21 4015

This annex lists the patent family members relating to the patent documents cited in the above-mentioned European search report. The members are as contained in the European Patent Office EDP file on The European Patent Office is in no way liable for these particulars which are merely given for the purpose of information.

26-04-2023

| Patent document cited in search report | Publication date | Patent family member(s) | Publication date |
|---|---|---|---|
| US 2017081650 A1 | 23-03-2017 | US 9512446 B1 | 06-12-2016 |
| | | US 2017081650 A1 | 23-03-2017 |
| | | US 2019071657 A1 | 07-03-2019 |
| WO 2017040348 A1 | 09-03-2017 | AU 2016316845 A1 | 22-03-2018 |
| | | AU 2022203146 A1 | 02-06-2022 |
| | | CA 2996888 A1 | 09-03-2017 |
| | | CN 108350449 A | 31-07-2018 |
| | | CN 114875012 A | 09-08-2022 |
| | | EP 3341477 A1 | 04-07-2018 |
| | | EP 4036236 A1 | 03-08-2022 |
| | | HK 1257676 A1 | 25-10-2019 |
| | | JP 6745391 B2 | 26-08-2020 |
| | | JP 6799586 B2 | 16-12-2020 |
| | | JP 6942848 B2 | 29-09-2021 |
| | | JP 7074827 B2 | 24-05-2022 |
| | | JP 2018525019 A | 06-09-2018 |
| | | JP 2020010695 A | 23-01-2020 |
| | | JP 2021003110 A | 14-01-2021 |
| | | JP 2021040644 A | 18-03-2021 |
| | | JP 2022023028 A | 07-02-2022 |
| | | KR 20180042394 A | 25-04-2018 |
| | | WO 2017040348 A1 | 09-03-2017 |
| US 2021355465 A1 | 18-11-2021 | US 2017058271 A1 | 02-03-2017 |
| | | US 2018216088 A1 | 02-08-2018 |
| | | US 2020149024 A1 | 14-05-2020 |
| | | US 2021355465 A1 | 18-11-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63289914
- US 8993233 B
- US 9023649 B
- US 8697359 B
- US 20140186958
- US 20160024529
- US 20160024524
- US 20160024523
- US 20160024510
- US 20160017366
- US 20160017301
- US 20150376652
- US 20150356239
- US 20150315576
- US 20150291965
- US 20150252358
- US 20150247150
- US 20150232883
- US 20150232882
- US 20150203872
- US 20150191744
- US 20150184139
- US 20150176064
- US 20150167000
- US 20150166969
- US 20150159175
- US 20150159174
- US 20150093473
- US 20150079681
- US 20150067922
- US 20150056629
- US 20150044772
- US 20150024500
- US 20150024499
- US 20150020223
- US 20140356867
- US 20140295557
- US 20140273235
- US 20140273226
- US 20140273037
- US 20140189896
- US 20140113376
- US 20140093941
- US 20130330778
- US 20130288251
- US 20120088676
- US 20110300538
- US 20110236530
- US 20110217739
- US 20110002889
- US 20100076057
- US 20110189776
- US 20110223638
- US 20130130248
- US 20150050699
- US 20150071899
- US 20150045546
- US 20150031134
- US 20140377868
- US 20140357530
- US 20140349400
- US 20140335620
- US 20140335063
- US 20140315985
- US 20140310830
- US 20140310828
- US 20140309487
- US 20140304853
- US 20140298547
- US 20140295556
- US 20140294773
- US 20140287938
- US 20140273234
- US 20140273232
- US 20140273231
- US 20140273230
- US 20140271987
- US 20140256046
- US 20140248702
- US 20140242702
- US 20140242700
- US 20140242699
- US 20140242664
- US 20140234972
- US 20140227787
- US 20140212869
- US 20140201857
- US 20140199767
- US 20140186919
- US 20140186843
- US 20140179770
- US 20140179006
- US 20140170753
- WO 2014099744 A
- WO 2014089290 A
- WO 2014144592 A
- WO 2014004288 A
- WO 2014204578 A

- WO 2014152432 A
- WO 2015099850 A
- WO 2008108989 A
- WO 2010054108 A
- WO 2012164565 A
- WO 2013098244 A
- WO 2013176772 A
- US 61652086
- US 3610795 A

## Non-patent literature cited in the description


- **CONG et al.** *Science,* 2013, vol. 339, 819-823
- **MALI et al.** *Science,* 2013, vol. 339, 823-826
- **SLAYMAKER et al.** *Science,* 2016, vol. 351, 84-88
- **KLEINSTIVER et al.** *Nature,* 2016, vol. 529, 490-495
- **CHEN et al.** *Nature,* 2017, vol. 550, 407-410
- **CASINI et al.** *NatBiotechnol,* 2018, vol. 6, 265-271
- **LEE et al.** *Nat Commun,* 2018, vol. 9, 3048
- **VAKULSKAS et al.** *Nat Med,* 2018, vol. 24, 1216-1224
- **CHOI et al.** *Nat Methods,* 2019, vol. 16, 722-730
- **RAN et al.** *Nature,* 2015, vol. 520, 186-191
- **KLEINSTIVER et al.** *Nat Biotechnol,* 2015, vol. 33
- **MA et al.** *Nat Commun,* 2019, vol. 10, 560
- **LUAN et al.** *JAm Chem Soc,* 2019, vol. 141, 6545-6552
- **KIM et al.** *Nat Commun,* 2017, vol. 8, 14500
- **EDRAKI et al.** *Mol Cell,* 2019, vol. 73, 714-726
- **ZETSCHE et al.** *Cell,* 2015, vol. 163, 759-771
- **PAUSCH et al.** *Science,* 2020, vol. 369, 333-337
- **TAN et al.** *Proc Natl Acad Sci U.S.A,* 2019, vol. 116, 20969-20976
- **NISHIMASU et al.** *Cell,* 2016, vol. 162, 1113-1126
- **MA et al.** *Nature Communications,* 2019, vol. 10, 560
- **JINEK et al.** *Science,* 2012, vol. 337, 816-821
- **FERRETTI et al.** *Proc Natl Acad Sci U.S.A,* 2001, vol. 98, 4658-4683
- **DELTCHEVA et al.** *Nature,* 2011, vol. 471, 602-607
- **CASINI et al.** *Nat Biotechnol,* 2018, vol. 6, 265-271
- **GREEN ; SAMBROOK.** Molecular Cloning: A Laboratory Manual. Cold Spring Harbor Laboratory Press, 2012
- **SMITH, T. F. ; M. S. WATERMAN.** *J Mol Biol,* 1981, vol. 147, 195-7
- **SMITH ; WATERMAN.** *Advances in Applied Mathematics,* 1981, 482-489
- **SCHWARZ ; DAYHOF.** Atlas of Protein Sequence and Structure. 1979, 353-358
- **ALTSCHUL, S. F. ; W. GISH et al.** *J Mol Biol,* 1990, vol. 215, 403-10
- **GOSSEN ; BUJARD.** *Proc. Natl. Acad. Sci. USA,* 1992, vol. 89, 5547
- **OLIGINO et al.** *Gene Ther.,* 1998, vol. 5, 491-496
- **WANG et al.** *Gene Ther.,* 1997, vol. 4, 432-441
- **NEERING et al.** *Blood,* 1996, vol. 88, 1147-55
- **RENDAHL et al.** *Nat. Biotechnol.,* 1998, vol. 16, 757-761
- **MAKAROVA et al.** Evolution and classification of the CRISPR-Cas systems. *Nature Reviews Microbiology,* June 2011, vol. 9 (6), 467-477
- **WIEDENHEFT et al.** RNA-guided genetic silencing systems in bacteria and archaea. *Nature,* 16 February 2012, vol. 482, 331-338
- **GASIUNAS et al.** Cas9-crRNA ribonucleoprotein complex mediates specific DNA cleavage for adaptive immunity in bacteria. *Proceedings of the National Academy of Sciences USA,* 04 September 2012, vol. 109 (39), E2579-E2586
- **JINEK et al.** A Programmable Dual-RNA-Guided DNA Endonuclease in Adaptive Bacterial Immunity. *Science,* 17 August 2012, vol. 337, 816-821
- **CARROLL.** A CRISPR Approach to Gene Targeting. *Molecular Therapy,* September 2012, vol. 20 (9), 1658-1660
- **AL-ATTAR et al.** Clustered Regularly Interspaced Short Palindromic Repeats (CRISPRs): The Hallmark of an Ingenious Antiviral Defense Mechanism in Prokaryotes. *Biol Chem,* 2011, vol. 392 (4), 277-289
- **HALE et al.** Essential Features and Rational Design of CRISPR RNAs That Function With the Cas RAMP Module Complex to Cleave RNAs. *Molecular Cell,* 2012, vol. 45 (3), 292-302
- **E.W. MARTIN.** Remington's Pharmaceutical Sciences. Mack Pub. Co,
- **TAN, Y. et al.** *Proc Natl Acad Sci USA,* 2019, vol. 116, 20969-20976
- **NISHIMASU et al.** *Cell,* 2015
- **TAN et al.** *PNAS,* 2019
- **SLAY MAKER et al.** *Science,* 2016
- **CHOI, G.C.G. et al.** *Nat Methods,* 2019, vol. 16, 722-730

- **GUSCHIN, D.Y. et al.** *Methods Mol Biol,* 2010, vol. 649, 247-256
- **TSAI, S.Q. et al.** *Nat Biotechnol,* 2016, vol. 34 (5
- **WONG, A.S. et al.** *Proc Natl Acad Sci USA,* 2016, vol. 113 (9), 2544-2549
- **CLEMENT, K. et al.** *NatBiotechnol,* 2019, vol. 37 (3), 224-226
- **RODRIGUES, C.H. et al.** *Nucleic Acids Res,* 2018, vol. 46 (W1), W350-W355
- **BISWAS, S. et al.** *Nat Methods,* 2021, vol. 18, 389-396
- **WU, Z. et al.** *Proc NatlAcad Sci USA,* 2019, vol. 116, 8852-8858
- **VAKULSKAS, C.A. et al.** *Nat Med,* 2018, vol. 24 (8), 1216-1224
- **XIE, H. et al.** *PLoS Biol,* 2020, vol. 18 (7), e3000747
- **KLEINSTIVER, B.P. et al.** *Nat Biotechnol,* 2015, vol. 33, 1293-1298
- **TAN et al.** *Proc NatlAcadSci U.S.A,* 2019, vol. 116, 20969-20976
- **CHEN, J.S. et al.** *Nature,* 2017, vol. 550, 407-410
- **KULCSAR, P.I. et al.** *Nat Commun,* 2020, vol. 11